# EUROPEAN PATENT APPLICATION

(11) **EP 4 806 894 A1**
(43) Date of publication of application: **16.09.2026**
(21) Application number: 24889156.6
(22) Date of filing: 07.11.2024
(51) Int. Cl.: C07D 471/04, A61K 31/437, A61K 31/4375, A61P 29/00, A61P 19/08, A61P 43/00

(54) **TNAP INHIBITORY COMPOUND AND USE THEREOF**

(30) Priority: 07.11.2023 KR 20230152806; 06.11.2024 KR 20240155794
(71) Applicant: Rednvia Co., Ltd., Seoul 05505 (KR)
(72) Inventor: KU, Il Whea, Seoul 05505 (KR); JO, Yong Hwa, Seoul 05505 (KR); KWON, Hye Shin, Seoul 05505 (KR); KWON, Min Jeong, Seoul 05505 (KR)
(74) Representative: Abel & Imray LLP
(86) International application number: PCT/KR2024/017551
(87) International publication number: WO 2025/100970

(57) **Abstract**

The present invention relates to a tissue-nonspecific alkaline phosphatase (TNAP) inhibitory compound and a use thereof in the prevention or treatment of diseases associated with TNAP overexpression or overactivation, wherein the compound is a compound of the following formula 1 or a pharmaceutically acceptable salt thereof.

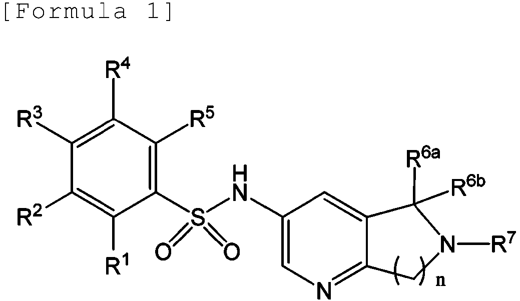

## Description

### BACKGROUND OF THE INVENTION

### 1. Field of the Invention

The present invention relates to a tissue-nonspecific alkaline phosphatase (TNAP) inhibitory compound and a use thereof.

The research on the present invention was conducted with the support of the Korea Drug Development Fund, funded by the Ministry of Science and ICT, Ministry of Trade, Industry and Energy, and Ministry of Health and Welfare (Project No.: RS-2023-00217370).

### 2. Description of the Related Art

Among alkaline phosphatases (ALPs), tissue-nonspecific alkaline phosphatase (TNAP) has been shown to hydrolyze extracellular pyrophosphate (ePPi) during bone mineralization. This reduces the amount of ePPi, an inhibitor of hydroxyapatite formation, and provides phosphate (Pi) for the formation of hydroxyapatite. The balance between ePPi and Pi is important in mineralization, so TNAP plays an important role in bone formation.

TNAP is expressed in bone, liver, and kidney, and is particularly highly expressed in matrix vesicles of chondrocytes and osteoblasts. This enzyme is known to play an important role in calcification in vivo through the degradation of pyrophosphate, an endogenous anti-calcification factor, and calcification in unwanted sites, known as ectopic calcification, leads to various diseases.

The increased expression level or elevated activity of TNAP was shown in the lesion site of ectopic calcification. Therefore, inhibition of TNAP is expected to increase pyrophosphate concentrations in blood and tissues and suppress ectopic calcification, leading to the development of TNAP inhibitors.

There are various diseases in which ectopic calcification is found, such as pseudoxanthoma elasticum (PXE), generalized arterial calcification of infancy (GACI), craniometaphyseal dysplasia (CMD), ossification of the yellow ligament (OYL), arterial calcification, calcification of joints, arthrosis deformans, osteoarthritis, ankylosis of the joint, idiopathic infantile arterial calcification (IIAC), ankylosing spondylitis (AS), tumoral calcinosis (TC), progressive osseous heteroplasia (POH), Keutel syndrome, vascular calcification associated with chronic renal failure (glomerulonephritis), IgA nephropathy (hypertensive nephropathy and diabetic nephropathy), secondary parathyroid hyperplasia, metastatic calcification, calciphylaxis, calcific tendinitis of the longus colli muscle, fibrodysplasia ossificans progressive (FOP), calcific aortic stenosis, pericarditis calculosa, atherosclerotic vascular calcification, calcific uremic arteriopathy (CUA), Kawasaki disease, calcification due to obesity and aging, tibial arterial calcification, bone metastasis, prosthetic calcification, Paget 's disease, idiopathic basal ganglia calcification (IBGC), heterotopic ossification (HO), calcific aortic valve disease (CAVD), aortic valve stenosis (AVS), calcific tendinitis, ossification of the posterior longitudinal ligament (OPLL), ossification of the anterior longitudinal ligament (OALL), diffuse idiopathic skeletal hyperostosis (DISH), meniscal calcification and peritoneal calcification.

As patents for inhibitors of TNAP, several technologies including benzene sulfonamide have been disclosed. For example, there are WO 2013/126608, WO 2017/007943, etc.

The present inventors developed a novel structure of TNAP inhibiting compounds based on benzene sulfonamide but containing fused pyridine compounds, which led to the present invention.

### SUMMARY OF THE INVENTION

It is an object of the present invention to provide a novel TNAP inhibitory compound.

It is another object of the present invention to provide a preventive or therapeutic agent for a disease caused by TNAP overexpression or overactivation.

It is another object of the present invention to provide a preventive or therapeutic agent for ectopic calcification and/or various diseases associated therewith.

To achieve the above objects, in an aspect of the present invention, the present invention provides a compound of formula 1 below or a pharmaceutically acceptable salt thereof.

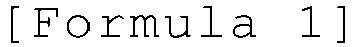

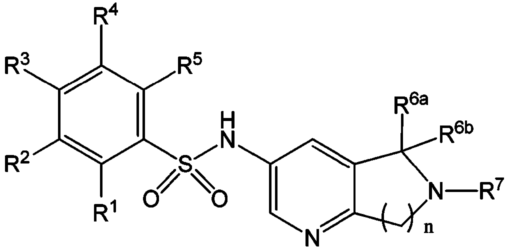

In formula 1,
R¹ is hydrogen, halogen, nonsubstituted or substituted phenyl, or C₁₋₁₀ alkoxy;
R² is hydrogen;
R³ is hydrogen or halogen;
R⁴ is hydrogen, halogen, nonsubstituted or substituted phenyl, nonsubstituted or substituted 5-9 membered heteroaryl, 6 membered cycloalkenyl nonsubstituted or substituted with one or more halogens, nonsubstituted or substituted 6 membered heterocycloalkenyl, or C₁₋₁₀ alkoxy;
R⁵ is hydrogen or halogen;
   or
two adjacent substituents of R¹ to R⁵ together form -(CH₂)m-O-, -O- (CH₂) m-0-, or -O-(CH₂)m-CO-NH-, while remaining three substituents are as described above;
the substituted phenyl is substituted with 1 to 3 substituents selected from the group consisting of C₁₋₁₀ alkoxy, carboxyl, nonsubstituted or substituted C₁₋₁₀ alkyl, 3-6 membered cycloalkyl substituted with carboxyl, halogen and hydroxyl, or is substituted at two adjacent positions to form -O-(CH₂)m-O-, wherein the substituted C₁₋₁₀ alkyl is substituted with carboxyl, C₁₋₁₀alkoxycarbonyl, amino or C₁₋₁₀alkylaminocarbonyl;
the substituted 5-9 membered heteroaryl is substituted with 1 to 3 substituents selected from the group consisting of halogen, amino, nonsubstituted C₁₋₁₀alkyl, substituted C₁₋₁₀ alkyl, NH₂CO-, nonsubstituted or substituted 4-6 membered heterocycloalkyl, C₁₋₁₀ alkylsulfonyl, 3-6 membered cycloalkyl, 5-6 membered heteroaryl and phenyl, wherein the substituted C₁₋₁₀ alkyl is substituted with carboxyl, C₁₋₁₀alkoxycarbonyl, C₁₋₁₀a lkylaminocarbonyl, 5-6 membered heteroaryl substituted with C₁₋₁₀ alkyl or phenyl substituted with one or more halogens or hydroxyl, one or more halogens, or hydroxyl, and the substituted 4-6 membered heterocycloalkyl is substituted with C₁₋₁₀alkyl or C₁₋₁₀ alkoxycarbonyl;
the substituted 6 membered heterocycloalkenyl is substituted with C₁₋₁₀a lkoxycarbonyl or C₁₋₁₀ alkylcarbonyl;
R^{6a} and R^{6b} are both hydrogen or together form oxo;
R⁷ is hydrogen, 5-6 membered heteroaryl, -COₓ-R^{8a}, -CONR^{8b}R^{8c}, or C₁₋₁₀alkyl, wherein x is 1 or 2,
R^{8a} and R^{8b} are independently nonsubstituted or substituted C₁₋₁₀alkyl, or C₆₋₁₀ aryl, wherein the substituted C₁₋₁₀ alkyl is substituted with one or more halogens, or 3-6 cycloalkyls,
R^{8c} is hydrogen or forms 5-6 membered heterocycloalkyl together with R^{8b} and the nitrogen atoms substituted therewith;
m is an integer of 1 to 4; and
n is an integer of 1 to 3.

In another aspect of the present invention, the present invention provides a use of a compound of formula 1, or a pharmaceutically acceptable salt thereof for the prevention or treatment of a TNAP-related disease.

In another aspect of the present invention, the present invention provides a pharmaceutical composition for the prevention or treatment of aTNAP-related disease, comprising a compound of formula 1 or a pharmaceutically acceptable salt thereof as an active ingredient.

In another aspect of the present invention, the present invention provides a use of a compound of formula 1 or a pharmaceutically acceptable salt thereof for the prevention or treatment of an ectopic calcification-related disease.

In another aspect of the present invention, the present invention provides a pharmaceutical composition for the prevention or treatment of an ectopic calcification-related disease, comprising a compound of formula 1 or a pharmaceutically acceptable salt thereof as an active ingredient.

In addition, the present invention provides a method of preventing or treating a TNAP-related disease or an ectopic calcification-related disease, comprising a step of administering the pharmaceutical composition to a subject in need thereof in a pharmaceutically effective amount.

### ADVANTAGEOUS EFFECT

The compound according to the present invention effectively inhibits the activity of TNAP and effectively suppresses calcification. Therefore, the composition of the present invention can effectively prevent or treat diseases caused by TNAP overexpression or overactivation or diseases related to ectopic calcification.

### BRIEF DESCRIPTION OF THE DRAWINGS

Figure 1 is a set of photographs showing that the example compounds of the present invention inhibit inflammation and calcification in a warfarin-induced calcification animal model.
Figure 2 is a set of photographs showing that the example compounds of the present invention inhibit inflammation and calcification in a vitamin D-induced calcification animal model.

### DESCRIPTION OF THE PREFERRED EMBODIMENTS

Hereinafter, the present invention is described in detail.

The embodiments of this invention can be modified in various other forms, and the scope of the present invention is not limited to the embodiments described below. It is well understood by those in the art who has the average knowledge on this field that the embodiments of the present invention are given to explain the present invention more precisely.

In addition, the "inclusion" of an element throughout the specification does not exclude other elements, but may include other elements, unless specifically stated otherwise.

In this specification, "alkoxy" means a radical in which alkyl is substituted through an oxygen atom. Herein, alkyl refers to a saturated hydrocarbon containing both straight and branched chains. For example, "C₁₋₆ alkyl" refers to alkyl having a skeleton of 1 to 6 carbons. Specifically, C₁₋₆ alkyl includes methyl, ethyl, n-propyl, i-propyl, n-butyl, i-butyl, t-butyl, n-pentyl, i-pentyl, t-pentyl, sec-pentyl, neopentyl, hexyl, etc., and C₆ alkyl is a fully saturated hydrocarbon having 6 carbons, and includes all structural isomers that can be formed as a saturated hydrocarbon having 6 carbons. C₁₋₁₀ alkoxy includes C₁₋₆ alkoxy, C₁₋₃alkoxy, or methoxy.

In this specification, "alkyl" includes saturated hydrocarbon residues in straight or branched chains, unless otherwise specified. For example, "C₁₋₆ alkyl" refers to alkyl having a skeleton of 1 to 6 carbons. Specifically, C₁₋₆ alkyl can include methyl, ethyl, n-propyl, i-propyl, n-butyl, i-butyl, t-butyl, n-pentyl, i-pentyl, t-pentyl, sec-pentyl, neopentyl, hexyl, and the like.

In this specification, "cycloalkyl" means a cyclic hydrocarbon residue that is a cyclic hydrocarbon residue forming a ring, unless otherwise specified. For example, "3-6 membered cycloalkyl" refers to a cyclic hydrocarbon residue containing 3 to 6 carbons as ring atoms.

In this specification, "heterocycloalkyl" means a monovalent saturated residue consisting of 1 to 3 rings containing one or more (e.g., 1-5, 1-4, 1-3, 1-2, or 1) heteroatoms selected from N, O or S, unless otherwise specified. It may be bicyclic or tricyclic containing 2 or 3 rings, wherein these 2 or 3 rings may be bridged, fused or spiro heterocycloalkyl. When it consists of multiple rings, heteroatoms can be present in all or only some of the rings.

In this specification, "aryl" means an aromatic radical having a single ring or two or three hydrocarbon aromatic rings fused together, and C₆₋₁₀ aryl means an aromatic ring compound containing 6 to 10 carbons, including phenyl or naphthyl.

In this specification, "heteroaryl" means an aromatic radical with a single ring or two or three fused rings having at least one aromatic ring containing at least one (e.g., 1-5, 1-4, 1-3, 1-2, or 1) heteroatom selected from the group consisting of N, O, or S (the remaining ring atom being C) as a ring atom, unless otherwise specified. When it consists of multiple rings, heteroatoms can be present in all or only some of the rings.

In this specification, "halogen" means a halogen atom such as F, Cl, Br, or I, and substituted with halogen means substituted with one or more halogen atoms. The number of halogen atoms to be substituted at this time can be selected within the range of 1 to 5.

In this specification, "cycloalkenyl" means a monovalent monocyclic group having at least one unsaturated double bond in the ring and having no aromaticity.

In this specification, "heterocycloalkenyl" means a monocyclic group containing at least one (e.g., 1-5, 1-4, 1-3, 1-2, or 1) heteroatom selected from the group consisting of N, O, or S as a ring atom, and having at least one unsaturated double bond in the ring.

In this specification, "substituted with a plurality of substituents selected from ~" may include substitution with a plurality of different substituents, and may also include substitution with a plurality of the same substituent.

In the first embodiment of the present invention, the compound according to the present invention may be a compound of the following formula 1 or a pharmaceutically acceptable salt thereof.

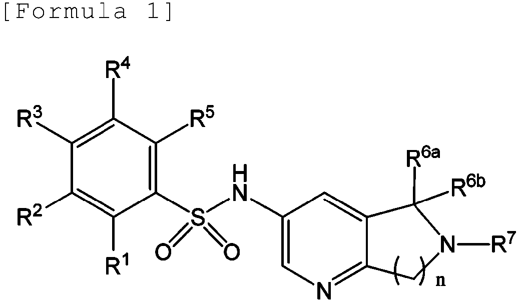

In formula 1,
R¹ is hydrogen, halogen, nonsubstituted or substituted phenyl, or C₁₋₁₀ alkoxy;
R² is hydrogen;
R³ is hydrogen or halogen;
R⁴ is hydrogen, halogen, nonsubstituted or substituted phenyl, nonsubstituted or substituted 5-9 membered heteroaryl, 6 membered cycloalkenyl nonsubstituted or substituted with one or more halogens, nonsubstituted or substituted 6 membered heterocycloalkenyl, or C₁₋₁₀ alkoxy;
R⁵ is hydrogen or halogen;
   or
two adjacent substituents of R¹ to R⁵ together form -(CH₂)m-O-, -O-(CH₂)m-O-, or -O-(CH₂)m-CO-NH-, while remaining three substituents are as described above;
the substituted phenyl is substituted with 1 to 3 substituents selected from the group consisting of C₁₋₁₀ alkoxy, carboxyl, nonsubstituted or substituted C₁₋₁₀ alkyl, 3-6 membered cycloalkyl substituted with carboxyl, halogen and hydroxyl, or is substituted at two adjacent positions to form -O-(CH₂)m-O-, wherein the substituted C₁₋₁₀ alkyl is substituted with carboxyl, C₁₋₁₀ alkoxycarbonyl, amino or C₁₋₁₀ alkylaminocarbonyl;
the substituted 5-9 membered heteroaryl is substituted with 1 to 3 substituents selected from the group consisting of halogen, amino, nonsubstituted C₁₋₁₀ alkyl, substituted C₁₋₁₀ alkyl, NH₂CO-, nonsubstituted or substituted 4-6 membered heterocycloalkyl, C₁₋₁₀ alkylsulfonyl, 3-6 membered cycloalkyl, 5-6 membered heteroaryl and phenyl, where the substituted C₁₋₁₀ alkyl is substituted with carboxyl, C₁₋₁₀ alkoxycarbonyl, C₁₋₁₀ alkylaminocarbonyl, 5-6 membered heteroaryl substituted with C₁₋₁₀ alkyl or phenyl substituted with one or more halogens, one or more halogens, or hydroxyl, and the substituted 4-6 membered heterocycloalkyl is substituted with C₁₋₁₀ alkyl or C₁₋₁₀ alkoxycarbonyl;
the substituted 6 membered heterocycloalkenyl is substituted with C₁₋₁₀ alkoxycarbonyl or C₁₋₁₀ alkylcarbonyl;
R^{6a} and R^{6b} are both hydrogen or together form oxo;
R⁷ is hydrogen, 5-6 membered heteroaryl, -COₓ-R^{8a}, -CONR^{8b}R^{8c}, or C₁₋₁₀alkyl, wherein x is 1 or 2,
R^{8a} and R^{8b} are independently nonsubstituted or substituted C₁₋₁₀ alkyl, or C₆₋₁₀ aryl, wherein the substituted C₁₋₁₀ alkyl is substituted with one or more halogens, or 3-6 cycloalkyls,
R^{8c} is hydrogen or forms 5-6 membered heterocycloalkyl together with R^{8b} and the nitrogen atoms substituted therewith;
m is an integer of 1 to 4; and
n is an integer of 1 to 3.

In the second embodiment of the present invention, R¹ is hydrogen, halogen, nonsubstituted or substituted phenyl, or C₁₋₅ alkoxy, and the substituted phenyl is cyclopropyl substituted with carboxyl, or nonsubstituted or substituted C₁₋₅ alkyl, wherein the substituted C₁₋₅ alkyl may be substituted with carboxyl or amino.

In the third embodiment, R⁴ is hydrogen, halogen, nonsubstituted or substituted phenyl, 9 membered bicyclic heteroaryl, nonsubstituted or substituted 5-6 membered heteroaryl, 6 membered cycloalkenyl nonsubstituted or substituted with one or more halogens, nonsubstituted or substituted 6 membered heterocycloalkenyl containing one heteroatom among O and N, or C₁₋₅ alkoxy;
the substituted phenyl is substituted with 1 to 3 substituents selected from the group consisting of C₁₋₅ alkoxy, carboxyl, nonsubstituted or substituted C₁₋₅ alkyl, cyclopropyl substituted with carboxyl, halogen and hydroxyl, or is substituted at two adjacent positions to form -O-(CH₂)m-O-, wherein the substituted C₁₋₅ alkyl is substituted with carboxyl, C₁₋₅ alkoxycarbonyl, amino or C₁₋₅ alkylaminocarbonyl;
the substituted 5-6 membered heteroaryl is substituted with 1 to 3 substituents selected from the group consisting of halogen, amino, nonsubstituted C₁₋₅ alkyl, substituted C₁₋₅ alkyl, NH₂CO-, nonsubstituted or substituted 4-6 membered heterocycloalkyl, methylsulfonyl, cyclopropyl, 6 membered heteroaryl, and phenyl, wherein the substituted C₁₋₅ alkyl is substituted with carboxyl, C₁₋₅ alkoxycarbonyl, C₁₋₅ alkylaminocarbonyl, 5 membered heteroaryl substituted with C₁₋₅ alkyl or phenyl substituted with one or more halogens, one or more halogens, or hydroxyl, and the substituted 4-6 membered heterocycloalkyl is substituted with C₁₋₅alkyl or C₁₋₅alkoxycarbonyl;
the substituted 6 membered heterocycloalkenyl is substituted with C₁₋₅ alkoxycarbonyl or C₁₋₅ alkylcarbonyl; and
m can be an integer of 1 to 3.

In the fourth embodiment, the two adjacent substituents of R¹ to R⁵ may be R¹ and R² or R² and R³.

In the fifth embodiment, when the two adjacent substituents of R¹ to R⁵ together form -(CH₂)m-O-, -O-(CH₂)m-O-, or -O-(CH₂)ₘ-CO-NH-, each of remaining three substituents is independently hydrogen, halogen, or substituted phenyl,
wherein the substituted phenyl is substituted with 3-6 membered cycloalkyl substituted with carboxyl, or C₁₋₁₀ alkyl substituted with amino, and
m can be an integer of 1 to 3.

In the sixth embodiment, when the two adjacent substituents of R¹ tto R⁵ is R¹ and R², and R¹ and R² together form -(CH₂)m-O-, -O-(CH₂)m-O-, or -O-(CH₂)ₘ-CO-NH-,
each of remaining three substituents is independently hydrogen, halogen, or substituted phenyl,
wherein the substituted phenyl is substituted with cyclopropyl substituted with carboxyl, or C₁₋₅ alkyl substituted with amino;
   or
when the two adjacent substituents of R¹ to R⁵ is R² and R³, and R² and R³ together form -(CH₂)m-O- or -O-(CH₂)ₘ-CO-NH-,
remaining three substituents are all hydrogen; and
m can be an integer of 1 to 2.

In the seventh embodiment, R⁷ is hydrogen, 6 membered heteroaryl, -COₓ-R^{8a}, -CONR^{8b}R^{8c}, or C₁₋₅ alkyl, wherein x is 1 or 2,
R^{8a} is C₁₋₆ alkyl nonsubstituted or substituted with 1 halogen,
R^{8b} is nonsubstituted or substituted C₁₋₆alkyl, or phenyl,
wherein the substituted C₁₋₆ alkyl is substituted with 1 or 2 halogen, or cyclopropyl,
R^{8c} is hydrogen or may form 6 membered heterocycloalkyl together with R^{8b} and the nitrogen atoms substituted therewith.

In the eighth embodiment, R¹ is C₁₋₅alkoxy, halogen, hydrogen, or nonsubstituted or substituted phenyl;
R² is hydrogen;
R³ is hydrogen or halogen;
R⁴ is hydrogen, halogen, nonsubstituted or substituted phenyl, pyrazolopyridine, nonsubstituted or substituted 5-6 membered heteroaryl, 6 membered cycloalkenyl nonsubstituted or substituted with one or more halogens, nonsubstituted or substituted heterocycloalkenyl containing one heteroatom among O and N, or C₁₋₅ alkoxy;
R⁵ is hydrogen or halogen;
   or
two adjacent substituents of R¹ to R⁵ together form -(CH₂)m-O-, -O-(CH₂)m-O-, or -O-(CH₂)m-CO-NH-, and each of remaining three substituents is independently hydrogen, halogen, or substituted phenyl, wherein the substituted phenyl is substituted with cyclopropyl substituted with carboxyl or C₁₋₃ alkyl substituted with amino;
the substituted phenyl is substituted with C₁₋₅ alkoxy, carboxyl, nonsubstituted or substituted C₁₋₅ alkyl, or cyclopropyl substituted with carboxyl, or is substituted with 1 to 3 substituents selected from the group consisting of halogen and hydroxyl, or is substituted at two adjacent positions to form -O-(CH₂)m-O-, wherein the substituted C₁₋₅ alkyl is substituted with carboxyl, C₁₋₅ alkoxycarbonyl, amino or C₁₋₅ alkylaminocarbonyl;
the substituted 5-6 membered heteroaryl is substituted with 1 to 3 substituents selected from the group consisting of halogen, amino, nonsubstituted C₁₋₅ alkyl, substituted C₁₋₅ alkyl, NH₂CO-, nonsubstituted or substituted 4-6 membered heterocycloalkyl, methylsulfonyl, cyclopropyl, 6 membered heteroaryl and phenyl, where the substituted C₁₋₅alkyl is substituted with carboxyl, C₁₋₅ alkoxycarbonyl, C₁₋₅ alkylaminocarbonyl, 5 membered heteroaryl substituted with C₁₋₅ alkyl or phenyl substituted with one halogen, one or more halogens, or hydroxyl, and the substituted 4-6 membered heterocycloalkyl is substituted with C₁₋₅ alkyl or C₁₋₅alkoxycarbonyl;
the substituted 6 membered heterocycloalkenyl is substituted with C₁₋₅ alkoxycarbonyl or C₁₋₅ alkylcarbonyl;
R^{6a} and R^{6b} are both hydrogen or together form oxo;
R⁷ is hydrogen, pyrimidinyl, -COₓ-R^{8a}, -CONR^{8b}R^{8c}, or C₁₋₅ alkyl, wherein x is 1 or 2,
R^{8a} is C₁₋₆ alkyl nonsubstituted or substituted with 1 halogen,
R^{8b} is nonsubstituted or substituted C₁₋₆alkyl, or phenyl,
wherein the substituted C₁₋₆ alkyl is substituted with 1 or 2 halogens, or cyclopropyl,
R^{8c} is hydrogen or forms morpholinyl together with R^{8b} and the nitrogen atoms substituted therewith;
m is an integer of 1 or 2; and
n is an integer of 1 to 3.

In the ninth embodiment, the two adjacent substituents of R¹ to R⁵ may be R¹ and R² or R² and R³.

In the tenth embodiment, when the two adjacent substituents of R¹ to R⁵ is R¹ and R², and R¹ and R² together form -(CH₂)m-O-, -O-(CH₂)ₘ-O-, or -O-(CH₂)ₘ-CO-NH-,
each of remaining three substituents is independently hydrogen, halogen, or substituted phenyl,
wherein the substituted phenyl is substituted with cyclopropyl substituted with carboxyl, or C₁₋₅ alkyl substituted with amino;
   or
when the two adjacent substituents of R¹ to R⁵ is R² and R³, and R² and R³ together form -(CH₂)ₘ-O- or -O-(CH₂)ₘ-CO-NH-,
remaining three substituents are all hydrogen; and
m can be an integer of 1 to 2.

In the eleventh embodiment, R¹ is hydrogen, halogen, 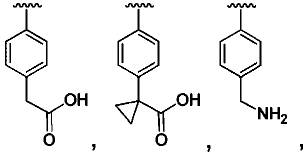 or methoxy;
R² is hydrogen;
R³ is hydrogen or halogen;
R⁴ is hydrogen, halogen, 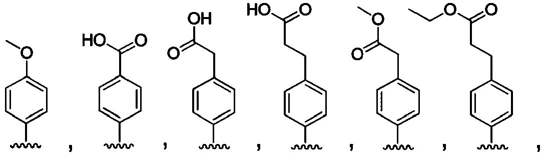 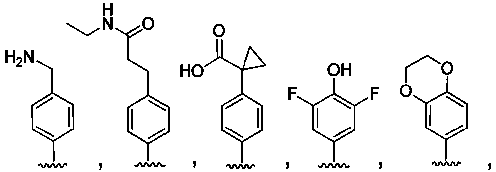 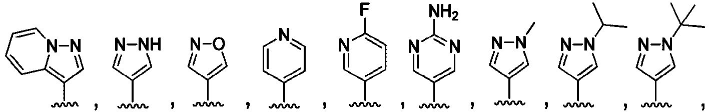 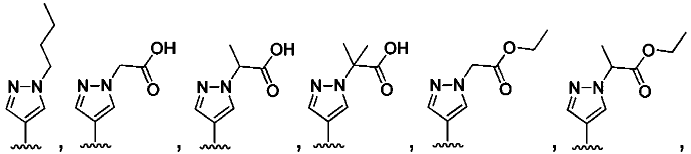 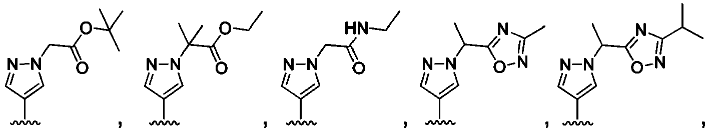 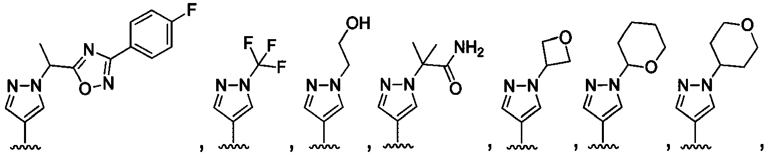 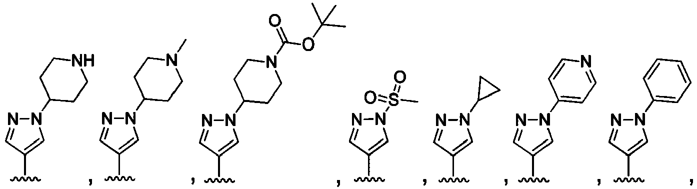 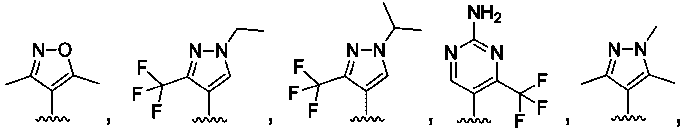 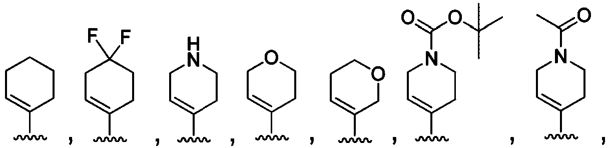 or methoxy;
R⁵ is hydrogen or halogen;
   Or
two adjacent substituents of R¹ to R⁵ together form 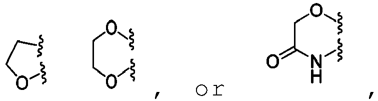 and each of remaining three substituents is independently hydrogen, halogen, or substituted phenyl, wherein the substituted phenyl is substituted with cyclopropyl substituted with carboxyl or C₁₋₃ alkyl substituted with amino;
R^{6a} and R^{6b} are both hydrogen or together form oxo;
R⁷ is hydrogen, 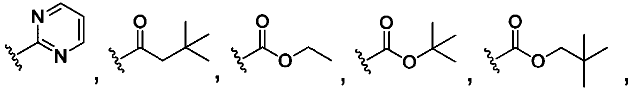 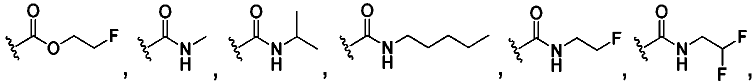 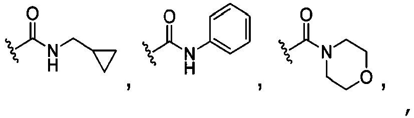 or C₁₋₃ alkyl; and
n can be an integer of 1 to 3.

In the twelfth embodiment, the compound or the pharmaceutically acceptable salt thereof may be any one selected from the following compounds.
<1> 5-chloro-2-methoxy-N-(5-oxo-6,7-dihydro-5H-pyrrolo[3,4-b]pyridine-3-yl)benzenesulfonamide;
<2> 5-bromo-2-methoxy-N-(5-oxo-6,7-dihydro-5H-pyrrolo[3,4-b]pyridine-3-yl)benzenesulfonamide;
<3> 2,5-dimethoxy-N-(5-oxo-6,7-dihydro-5H-pyrrolo[3,4-b]pyridine-3-yl)benzenesulfonamide;
<4> 5-fluoro-2-methoxy-N-(5-oxo-6,7-dihydro-5H-pyrrolo[3,4-b]pyridine-3-yl)benzenesulfonamide;
<5> 5-bromo-N-(5-oxo-6,7-dihydro-5H-pyrrolo[3,4-b]pyridine-3-yl)-2,3-dihydrobenzofuran-7-sulfonamide;
<6> 5-chloro-2-methoxy-N-(5-oxo-5,6,7,8-tetrahydro-1,6-naphthyridine-3-yl)benzenesulfonamide;
<7> 2,5-dimethoxy-N-(5-oxo-5,6,7,8-tetrahydro-1,6-naphthyridine-3-yl)benzenesulfonamide;
<8> 5-fluoro-2-methoxy-N-(5-oxo-5,6,7,8-tetrahydro-1,6-naphthyridine-3-yl)benzenesulfonamide;
<9> 5-bromo-2-methoxy-N-(5-oxo-5,6,7,8-tetrahydro-1,6-naphthyridine-3-yl)benzenesulfonamide;
<10> 5-bromo-N-(5-oxo-5,6,7,8-tetrahydro-1,6-naphthyridine-3-yl)-2,3-dihydrobenzofuran-7-sulfonamide;
<11> 2-bromo-5-fluoro-N-(5-oxo-6,7-dihydro-5H-pyrrolo[3,4-b]pyridine-3-yl)benzenesulfonamide;
<12> 2-bromo-4,6-difluoro-N-(5-oxo-6,7-dihydro-5H-pyrrolo[3,4-b]pyridine-3-yl)benzenesulfonamide;
<13> 5-chloro-2-methoxy-N-(5-oxo-6,7,8,9-tetrahydro-5H-pyrido[3,2-c]azepine-3-yl)benzenesulfonamide;
<14> 5-bromo-2-methoxy-N-(5-oxo-6,7,8,9-tetrahydro-5H-pyrido[3,2-c]azepine-3-yl)benzenesulfonamide;
<15> 5-fluoro-2-methoxy-N-(5-oxo-6,7,8,9-tetrahydro-5H-pyrido[3,2-c]azepine-3-yl)benzenesulfonamide;
<16> 2,5-dimethoxy-N-(5-oxo-6,7,8,9-tetrahydro-5H-pyrido[3,2-c]azepine-3-yl)benzenesulfonamide;
<17> 5-bromo-N-(5-oxo-6,7,8,9-tetrahydro-5H-pyrido[3,2-c]azepine-3-yl)-2,3-dihydrobenzofuran-7-sulfonamide;
<18> N-(5-oxo-6,7-dihydro-5H-pyrrolo[3,4-b]pyridine-3-yl)-2,3-dihydrobenzofuran-7-sulfonamide;
<19> N-(5-oxo-5,6,7,8-tetrahydro-1,6-naphthyridine-3-yl)-2,3-dihydrobenzofuran-7-sulfonamide;
<20> 5-chloro-N-(5-oxo-6,7-dihydro-5H-pyrrolo[3,4-b]pyridine-3-yl)-2,3-dihydrobenzofuran-7-sulfonamide;
<21> 5-fluoro-N-(5-oxo-6,7-dihydro-5H-pyrrolo[3,4-b]pyridine-3-yl)-2,3-dihydrobenzofuran-7-sulfonamide;
<22> 5-chloro-N-(5-oxo-5,6,7,8-tetrahydro-1,6-naphthyridine-3-yl)-2,3-dihydrobenzofuran-7-sulfonamide;
<23> 5-fluoro-N-(5-oxo-5,6,7,8-tetrahydro-1,6-naphthyridine-3-yl)-2,3-dihydrobenzofuran-7-sulfonamide;
<24> N-(5-oxo-5,6,7,8-tetrahydro-1,6-naphthyridine-3-yl)-2,3-dihydrobenzo[b][1,4]dioxine-6-sulfonamide;
<25> 3-oxo-N-(5-oxo-5,6,7,8-tetrahydro-1,6-naphthyridine-3-yl)-3,4-dihydro-2H-benzo[b][1,4]oxazine-6-sulfonamide;
<26> 2-methoxy-N-(5-oxo-5,6,7,8-tetrahydro-1,6-naphthyridine-3-yl)benzenesulfonamide;
<27> methyl 2-(4'-methoxy-3'-(N-(5-oxo-6,7-dihydro-5H-pyrrolo[3,4-b]pyridine-3-yl)sulfamoyl)-[1,1'-biphenyl]-4-yl)acetate;
<28> 2-(4'-methoxy-3'-(N-(5-oxo-6,7-dihydro-5H-pyrrolo[3,4-b]pyridine-3-yl)sulfamoyl)-[1,1'-biphenyl]-4-yl)acetic acid;
<29> 5-(2,3-dihydrobenzo[b][1,4]dioxine-6-yl)-2-methoxy-N-(5-oxo-5,6,7,8-tetrahydro-1,6-naphthyridine-3-yl)benzenesulfonamide;
<30> 4,4'-dimethoxy-N-(5-oxo-5,6,7,8-tetrahydro-1,6-naphthyridine-3-yl)-[1,1'-biphenyl]-3-sulfonamide;
<31> 2-methoxy-N-(5-oxo-5,6,7,8-tetrahydro-1,6-naphthyridine-3-yl)-5-(pyridine-4-yl)benzenesulfonamide;
<32> 1-(4'-methoxy-3'-(N-(5-oxo-5,6,7,8-tetrahydro-1,6-naphthyridine-3-yl)sulfamoyl)-[1,1'-biphenyl]-4-yl)cyclopropane-1-carboxylic acid;
<33> 2-(4'-methoxy-3'-(N-(5-oxo-5,6,7,8-tetrahydro-1,6-naphthyridine-3-yl)sulfamoyl)-[1,1'-biphenyl]-4-yl)acetic acid;
<34> 4'-methoxy-3'-(N-(5-oxo-5,6,7,8-tetrahydro-1,6-naphthyridine-3-yl)sulfamoyl)-[1,1'-biphenyl]-4-carboxylic acid;
<35> 2-(4'-fluoro-2'-(N-(5-oxo-5,6,7,8-tetrahydro-1,6-naphthyridine-3-yl)sulfamoyl)-[1,1'-biphenyl]-4-yl)acetic acid;
<36> 1-(4'-fluoro-2'-(N-(5-oxo-5,6,7,8-tetrahydro-1,6-naphthyridine-3-yl)sulfamoyl)-[1,1'-biphenyl]-4-yl)cyclopropane-1-carboxylic acid;
<37> 1-(3',5'-difluoro-2'-(N-(5-oxo-5,6,7,8-tetrahydro-1,6-naphthyridine-3-yl)sulfamoyl)-[1,1'-biphenyl]-4-yl)cyclopropane-1-carboxylic acid;
<38> 1-(4-(7-(N-(5-oxo-5,6,7,8-tetrahydro-1,6-naphthyridine-3-yl)sulfamoyl)-2,3-dihydrobenzofuran-5-yl)phenyl)cyclopropane-1-carboxylic acid;
<39> 4'-(aminomethyl)-4-fluoro-N-(5-oxo-5,6,7,8-tetrahydro-1,6-naphthyridine-3-yl)-[1,1'-biphenyl]-2-sulfonamide hydrochloride;
<40> 4'-(aminomethyl)-3,5-difluoro-N-(5-oxo-5,6,7,8-tetrahydro-1,6-naphthyridine-3-yl)-[1,1'-biphenyl]-2-sulfonamide hydrochloride;
<41> 5-(4-(aminomethyl)phenyl)-N-(5-oxo-5,6,7,8-tetrahydro-1,6-naphthyridine-3-yl)-2,3-dihydrobenzofuran-7-sulfonamide;
<42> ethyl 2-(4-(4-methoxy-3-(N-(5-oxo-5,6,7,8-tetrahydro-1,6-naphthyridine-3-yl)sulfamoyl)phenyl)-1H-pyrazole-1-yl)propanoate;
<43> 2-(4-(4-methoxy-3-(N-(5-oxo-5,6,7,8-tetrahydro-1,6-naphthyridine-3-yl)sulfamoyl)phenyl)-1H-pyrazole-1-yl)propanoic acid;
<44> 5-(2-aminopyrimidine-5-yl)-2-methoxy-N-(5-oxo-5,6,7,8-tetrahydro-1,6-naphthyridine-3-yl)benzenesulfonamide;
<45> 2-methoxy-N-(5-oxo-5,6,7,8-tetrahydro-1,6-naphthyridine-3-yl)-5-(pyrazolo[1,5-a]pyridine-3-yl)benzenesulfonamide;
<46> 3',5'-difluoro-4'-hydroxy-4-methoxy-N-(5-oxo-5,6,7,8-tetrahydro-1,6-naphthyridine-3-yl)-[1,1'-biphenyl]-3-sulfonamide;
<47> tert-butyl 2-(4-(4-methoxy-3-(N-(5-oxo-5,6,7,8-tetrahydro-1,6-naphthyridine-3-yl)sulfamoyl)phenyl)-1H-pyrazole-1-yl)acetate;
<48> 2-(4-(4-methoxy-3-(N-(5-oxo-5,6,7,8-tetrahydro-1,6-naphthyridine-3-yl)sulfamoyl)phenyl)-1H-pyrazole-1-yl)acetic acid;
<49> ethyl 3-(4'-methoxy-3'-(N-(5-oxo-5,6,7,8-tetrahydro-1,6-naphthyridine-3-yl)sulfamoyl)-[1,1'-biphenyl]-4-yl)propanoate;
<50> 3-(4'-methoxy-3'-(N-(5-oxo-5,6,7,8-tetrahydro-1,6-naphthyridine-3-yl)sulfamoyl)-[1,1'-biphenyl]-4-yl)propanoic acid;
<51> 5-(3,6-dihydro-2H-pyran-4-yl)-2-methoxy-N-(5-oxo-5,6,7,8-tetrahydro-1,6-naphthyridine-3-yl)benzenesulfonamide;
<52> tert-butyl 4-(4-methoxy-3-(N-(5-oxo-5,6,7,8-tetrahydro-1,6-naphthyridine-3-yl)sulfamoyl)phenyl)-3,6-dihydropyridine-1(2H)-carboxylate;
<53> 2-methoxy-N-(5-oxo-5,6,7,8-tetrahydro-1,6-naphthyridine-3-yl)-5-(1,2,3,6-tetrahydropyridine-4-yl)benzenesulfonamide hydrochloride;
<54> 2-methoxy-N-(5-oxo-5,6,7,8-tetrahydro-1,6-naphthyridine-3-yl)-5-(1-(tetrahydro-2H-pyran-2-yl)-1H-pyrazole-4-yl)benzenesulfonamide;
<55> 2-methoxy-N-(5-oxo-5,6,7,8-tetrahydro-1,6-naphthyridine-3-yl)-5-(1H-pyrazole-4-yl)benzenesulfonamide;
<56> 2-bromo-5-fluoro-N-(5-oxo-5,6,7,8-tetrahydro-1,6-naphthyridine-3-yl)benzenesulfonamide;
<57> 2-methoxy-5-(1-methyl-1H-pyrazole-4-yl)-N-(5-oxo-5,6,7,8-tetrahydro-1,6-naphthyridine-3-yl)benzenesulfonamide;
<58> 2-(4-(4-methoxy-3-(N-(5-oxo-5,6,7,8-tetrahydro-1,6-naphthyridine-3-yl)sulfamoyl)phenyl)-1H-pyrazole-1-yl)-2-methylpropanamide;
<59> ethyl 2-(4-(4-methoxy-3-(N-(5-oxo-5,6,7,8-tetrahydro-1,6-naphthyridine-3-yl)sulfamoyl)phenyl)-1H-pyrazole-1-yl)-2-methylpropanoate;
<60> 2-(4-(4-methoxy-3-(N-(5-oxo-5,6,7,8-tetrahydro-1,6-naphthyridine-3-yl)sulfamoyl)phenyl)-1H-pyrazole-1-yl)-2-methylpropanoic acid;
<61> 5-(1-acetyl-1,2,3,6-tetrahydropyridine-4-yl)-2-methoxy-N-(5-oxo-5,6,7,8-tetrahydro-1,6-naphthyridine-3-yl)benzenesulfonamide;
<62> 5-(5,6-dihydro-2H-pyran-3-yl)-2-methoxy-N-(5-oxo-5,6,7,8-tetrahydro-1,6-naphthyridine-3-yl)benzenesulfonamide;
<63> 4-methoxy-N-(5-oxo-5,6,7,8-tetrahydro-1,6-naphthyridine-3-yl)-2',3',4',5'-tetrahydro-[1,1'-biphenyl]-3-sulfonamide;
<64> 4',4'-difluoro-4-methoxy-N-(5-oxo-5,6,7,8-tetrahydro-1,6-naphthyridine-3-yl)-2',3',4',5'-tetrahydro-[1,1'-biphenyl]-3-sulfonamide;
<65> 2-methoxy-5-(1-(1-methylpiperidine-4-yl)-1H-pyrazole-4-yl)-N-(5-oxo-5,6,7,8-tetrahydro-1,6-naphthyridine-3-yl)benzenesulfonamide;
<66> N-ethyl-2-(4-(4-methoxy-3-(N-(5-oxo-5,6,7,8-tetrahydro-1,6-naphthyridine-3-yl)sulfamoyl)phenyl)-1H-pyrazole-1-yl)acetamide;
<67> N-ethyl-3-(4'-methoxy-3'-(N-(5-oxo-5,6,7,8-tetrahydro-1,6-naphthyridine-3-yl)sulfamoyl)-[1,1'-biphenyl]-4-yl)propanamide;
<68> ethyl 2-(4-(4-methoxy-3-(N-(5-oxo-5,6,7,8-tetrahydro-1,6-naphthyridine-3-yl)sulfamoyl)phenyl)-1H-pyrazole-1-yl)acetate;
<69> 5-(1-(1-(3-isopropyl-1,2,4-oxadiazole-5-yl)ethyl)-1H-pyrazole-4-yl)-2-methoxy-N-(5-oxo-5,6,7,8-tetrahydro-1,6-naphthyridine-3-yl)benzenesulfonamide;
<70> 2-methoxy-5-(1-(1-(3-methyl-1,2,4-oxadiazole-5-yl)ethyl)-1H-pyrazole-4-yl)-N-(5-oxo-5,6,7,8-tetrahydro-1,6-naphthyridine-3-yl)benzenesulfonamide;
<71> 5-(1-(1-(3-(4-fluorophenyl)-1,2,4-oxadiazole-5-yl)ethyl)-1H-pyrazole-4-yl)-2-methoxy-N-(5-oxo-5,6,7,8-tetrahydro-1,6-naphthyridine-3-yl)benzenesulfonamide;
<72> 5-(1-isopropyl-1H-pyrazole-4-yl)-2-methoxy-N-(5-oxo-5,6,7,8-tetrahydro-1,6-naphthyridine-3-yl)benzenesulfonamide;
<73> 2-methoxy-N-(5-oxo-5,6,7,8-tetrahydro-1,6-naphthyridine-3-yl)-5-(1-(trifluoromethyl)-1H-pyrazole-4-yl)benzenesulfonamide;
<74> 5-(1-cyclopropyl-1H-pyrazole-4-yl)-2-methoxy-N-(5-oxo-5,6,7,8-tetrahydro-1,6-naphthyridine-3-yl)benzenesulfonamide;
<75> 2-methoxy-5-(1-(methylsulfonyl)-1H-pyrazole-4-yl)-N-(5-oxo-5,6,7,8-tetrahydro-1,6-naphthyridine-3-yl)benzenesulfonamide;
<76> 2-methoxy-N-(5-oxo-5,6,7,8-tetrahydro-1,6-naphthyridine-3-yl)-5-(1-(tetrahydro-2H-pyran-4-yl)-1H-pyrazole-4-yl)benzenesulfonamide;
<77> 5-(6-fluoropyridine-3-yl)-2-methoxy-N-(5-oxo-5,6,7,8-tetrahydro-1,6-naphthyridine-3-yl)benzenesulfonamide;
<78> 5-(2-amino-4-(trifluoromethyl)pyrimidine-5-yl)-2-methoxy-N-(5-oxo-5,6,7,8-tetrahydro-1,6-naphthyridine-3-yl)benzenesulfonamide;
<79> 2-methoxy-5-(1-(oxetane-3-yl)-1H-pyrazole-4-yl)-N-(5-oxo-5,6,7,8-tetrahydro-1,6-naphthyridine-3-yl)benzenesulfonamide;
<80> 5-(1-(2-hydroxyethyl)-1H-pyrazole-4-yl)-2-methoxy-N-(5-oxo-5,6,7,8-tetrahydro-1,6-naphthyridine-3-yl)benzenesulfonamide;
<81> 5-(1-isopropyl-3-(trifluoromethyl)-1H-pyrazole-4-yl)-2-methoxy-N-(5-oxo-5,6,7,8-tetrahydro-1,6-naphthyridine-3-yl)benzenesulfonamide;
<82> 5-(1-ethyl-3-(trifluoromethyl)-1H-pyrazole-4-yl)-2-methoxy-N-(5-oxo-5,6,7,8-tetrahydro-1,6-naphthyridine-3-yl)benzenesulfonamide;
<83> 5-(isoxazole-4-yl)-2-methoxy-N-(5-oxo-5,6,7,8-tetrahydro-1,6-naphthyridine-3-yl)benzenesulfonamide;
<84> 5-(3,5-dimethylisoxazole-4-yl)-2-methoxy-N-(5-oxo-5,6,7,8-tetrahydro-1,6-naphthyridine-3-yl)benzenesulfonamide;
<85> tert-butyl 4-(4-(4-methoxy-3-(N-(5-oxo-5,6,7,8-tetrahydro-1,6-naphthyridine-3-yl)sulfamoyl)phenyl)-1H-pyrazole-1-yl)piperidine-1-carboxylate;
<86> 2-methoxy-N-(5-oxo-5,6,7,8-tetrahydro-1,6-naphthyridine-3-yl)-5-(1-(piperidine-4-yl)-1H-pyrazole-4-yl)benzenesulfonamide hydrochloride;
<87> 5-bromo-2-methoxy-N-(6-methyl-5-oxo-5,6,7,8-tetrahydro-1,6-naphthyridine-3-yl)benzenesulfonamide;
<88> tert-butyl 3-((5-bromo-2-methoxyphenyl)sulfonamido)-7,8-dihydro-1,6-naphthyridine-6(5H)-carboxylate;
<89> tert-butyl 3-((2,5-dimethoxyphenyl)sulfonamido)-7,8-dihydro-1,6-naphthyridine-6(5H)-carboxylate;
<90> 5-fluoro-2-methoxy-N-(5,6,7,8-tetrahydro-1,6-naphthyridine-3-yl)benzenesulfonamide hydrochloride;
<91> 5-fluoro-2-bromo-N-(5,6,7,8-tetrahydro-1,6-naphthyridine-3-yl)benzenesulfonamide hydrochloride;
<92> 5-chloro-2-methoxy-N-(5,6,7,8-tetrahydro-1,6-naphthyridine-3-yl)benzenesulfonamide hydrochloride;
<93> 5-bromo-2-methoxy-N-(5,6,7,8-tetrahydro-1,6-naphthyridine-3-yl)benzenesulfonamide hydrochloride;
<94> 2,5-dimethoxy-N-(5,6,7,8-tetrahydro-1,6-naphthyridine-3-yl)benzenesulfonamide hydrochloride;
<95> ethyl 3-((5-fluoro-2-methoxyphenyl)sulfonamido)-7,8-dihydro-1,6-naphthyridine-6(5H)-carboxylate;
<96> ethyl 3-((5-chloro-2-methoxyphenyl)sulfonamido)-7,8-dihydro-1,6-naphthyridine-6(5H)-carboxylate;
<97> ethyl 3-((5-bromo-2-methoxyphenyl)sulfonamido)-7,8-dihydro-1,6-naphthyridine-6(5H)-carboxylate;
<98> neopentyl 3-((5-chloro-2-methoxyphenyl)sulfonamido)-7,8-dihydro-1,6-naphthyridine-6(5H)-carboxylate;
<99> neopentyl 3-((5-bromo-2-methoxyphenyl)sulfonamido)-7,8-dihydro-1,6-naphthyridine-6(5H)-carboxylate;
<100> neopentyl 3-((5-methoxy-2-methoxyphenyl)sulfonamido)-7,8-dihydro-1,6-naphthyridine-6(5H)-carboxylate;
<101> 2-fluoroethyl-3-((5-chloro-2-methoxyphenyl)sulfonamido)-7,8-dihydro-1,6-naphthyridine-6(5H)-carboxylate;
<102> 2-fluoroethyl3-((5-bromo-2-methoxyphenyl)sulfonamido)-7,8-dihydro-1,6-naphthyridine-6(5H)-carboxylate;
<103> 2-fluoroethyl-3-((5-methoxy-2-methoxyphenyl)sulfonamido)-7,8-dihydro-1,6-naphthyridine-6(5H)-carboxylate;
<104> 3-((5-chloro-2-methoxyphenyl)sulfonamido)-N-isopropyl-7,8-dihydro-1,6-naphthyridine-6(5H)-carboxamide;
<105> 3-((5-fluoro-2-methoxyphenyl)sulfonamido)-N-isopropyl-7,8-dihydro-1,6-naphthyridine-6(5H)-carboxamide;
<106> 3-((5-chloro-2-methoxyphenyl)sulfonamido)-N-pentyl-7,8-dihydro-1,6-naphthyridine-6(5H)-carboxamide;
<107> 3-((5-chloro-2-methoxyphenyl)sulfonamido)-N-(2,2-difluoroethyl)-7,8-dihydro-1,6-naphthyridine-6(5H)-carboxamide;
<108> 3-((5-bromo-2-methoxyphenyl)sulfonamido)-N-(2,2-difluoroethyl)-7,8-dihydro-1,6-naphthyridine-6(5H)-carboxamide;
<109> 5-chloro-2-methoxy-N-(6-(morpholine-4-carbonyl)-5,6,7,8-tetrahydro-1,6-naphthyridine-3-yl)benzenesulfonamide;
<110> 5-bromo-2-methoxy-N-(6-(morpholine-4-carbonyl)-5,6,7,8-tetrahydro-1,6-naphthyridine-3-yl)benzenesulfonamide;
<111> 3-((5-bromo-2-methoxyphenyl)sulfonamido)-N-methyl-7,8-dihydro-1,6-naphthyridine-6(5H)-carboxamide;
<112> 5-chloro-N-(6-isopropyl-5,6,7,8-tetrahydro-1,6-naphthyridine-3-yl)-2-methoxybenzenesulfonamide;
<113> 5-bromo-N-(6-isopropyl-5,6,7,8-tetrahydro-1,6-naphthyridine-3-yl)-2-methoxybenzenesulfonamide;
<114> 3-5-chloro-2-methoxy-N-(6-(pyrimidine-2-yl)-5,6,7,8-tetrahydro-1,6-naphthyridine-3-yl)benzenesulfonamide;
<115> 3-((5-chloro-2-methoxyphenyl)sulfonamido)-N-phenyl-7,8-dihydro-1,6-naphthyridine-6(5H)-carboxamide;
<116> 3-((5-chloro-2-methoxyphenyl)sulfonamido)-N-(cyclopropylmethyl)-7,8-dihydro-1,6-naphthyridine-6(5H)-carboxamide;
<117> 3-((5-chloro-2-methoxyphenyl)sulfonamido)-N-(2-fluoroethyl)-7,8-dihydro-1,6-naphthyridine-6(5H)-carboxamide;
<118> 5-chloro-N-(6-(3,3-dimethylbutanoyl)-5,6,7,8-tetrahydro-1,6-naphthyridine-3-yl)-2-methoxybenzenesulfonamide;
<119> 5-bromo-N-(6-(3,3-dimethylbutanoyl)-5,6,7,8-tetrahydro-1,6-naphthyridine-3-yl)-2-methoxybenzenesulfonamide;
<120> 2-methoxy-N-(5-oxo-5,6,7,8-tetrahydro-1,6-naphthyridine-3-yl)-5-(1-(pyridine-4-yl)-1H-pyrazole-4-yl)benzenesulfonamide;
<121> 2-methoxy-N-(5-oxo-5,6,7,8-tetrahydro-1,6-naphthyridine-3-yl)-5-(1,3,5-trimethyl-1H-pyrazole-4-yl)benzenesulfonamide;
<122> 5-(1-(tert-butyl)-1H-pyrazole-4-yl)-2-methoxy-N-(5-oxo-5,6,7,8-tetrahydro-1,6-naphthyridine-3-yl)benzenesulfonamide;
<123> 5-(1-butyl-1H-pyrazole-4-yl)-2-methoxy-N-(5-oxo-5,6,7,8-tetrahydro-1,6-naphthyridine-3-yl)benzenesulfonamide; and
<124> 2-methoxy-N-(5-oxo-5,6,7,8-tetrahydro-1,6-naphthyridine-3-yl)-5-(1-phenyl-1H-pyrazole-4-yl)benzenesulfonamide.

The compound of formula 1 of the present invention can be provided in the form of a pharmaceutically acceptable salt, and the term "pharmaceutically acceptable salt" means a salt formed by ionic bonding when the compound contains a carboxylic acid or amine functional group, which is pharmaceutically acceptable.

The compound represented by formula 1 of the present invention can be used as a form of a pharmaceutically acceptable salt, in which the salt is preferably acid addition salt formed by pharmaceutically acceptable free acids. The acid addition salt herein can be obtained from inorganic acids such as hydrochloric acid, nitric acid, phosphoric acid, sulfuric acid, hydrobromic acid, hydroiodic acid, nitrous acid, and phosphorous acid; non-toxic organic acids such as aliphatic mono/dicarboxylate, phenyl-substituted alkanoate, hydroxy alkanoate, alkandioate, aromatic acids, and aliphatic/aromatic sulfonic acids; or organic acids such as acetic acid, benzoic acid, citric acid, lactic acid, maleic acid, gluconic acid, methanesulfonic acid, 4-toluenesulfonic acid, tartaric acid, and fumaric acid. The pharmaceutically non-toxic salts are exemplified by sulfate, pyrosulfate, bisulfate, sulphite, bisulphite, nitrate, phosphate, monohydrogen phosphate, dihydrogen phosphate, metaphosphate, pyrophosphate, chloride, bromide, iodide, fluoride, acetate, propionate, decanoate, caprylate, acrylate, formate, isobutylate, caprate, heptanoate, propiolate, oxalate, malonate, succinate, suberate, cabacate, fumarate, maliate, butyne-1,4-dioate, hexane-1,6-dioate, benzoate, chlorobenzoate, methylbenzoate, dinitrobenzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutylate, citrate, lactate, hydroxybutylate, glycolate, malate, tartrate, methanesulfonate, propanesulfonate, naphthalene-1-sulfonate, naphthalene-2-sulfonate, and mandelate.

The acid addition salt according to the present invention can be prepared by the conventional method known to those in the art. For example, the derivative represented by formula 1 is dissolved in an organic solvent such as methanol, ethanol, acetone, methylenechloride, and acetonitrile, to which organic acid or inorganic acid is added to induce precipitation. Then, the precipitate is filtered and dried to give the salt. Or the solvent and the excessive acid are distillated under reduced pressure, and dried to give the salt. Or the precipitate is crystallized in an organic solvent to give the same.

A pharmaceutically acceptable metal salt can be prepared by using a base. Alkali metal or alkali earth metal salt is obtained by the following processes: dissolving the compound in excessive alkali metal hydroxide or alkali earth metal hydroxide solution; filtering non-soluble compound salt; evaporating the remaining solution and drying thereof. At this time, the metal salt is preferably prepared in the pharmaceutically suitable form of sodium, potassium, or calcium salt. And the corresponding silver salt is prepared by the reaction of alkali metal or alkali earth metal salt with proper silver salt (ex; silver nitrate).

The use of the compound of formula 1 or the pharmaceutically acceptable salt thereof is described hereinafter.

In one aspect of the present invention, the compound of formula 1 or the pharmaceutically acceptable salt thereof inhibits TNAP.

In another aspect of the present invention, the compound of formula 1 or the pharmaceutically acceptable salt thereof can improve or treat a disease associated with overexpression or overactivation of TNAP by inhibiting TNAP.

In another aspect of the present invention, the compound of formula 1 or the pharmaceutically acceptable salt thereof can improve or treat a disease associated with ectopic calcification by inhibiting TNAP.

An embodiment of the present invention relates to a compound of formula 1 or a pharmaceutically acceptable salt thereof for use in the prevention or treatment of a disease associated with TNAP overexpression or overactivation.

Another embodiment of the present invention relates to a pharmaceutical composition for the prevention or treatment of a disease associated with TNAP overexpression or overactivation, comprising a compound of formula 1 or a pharmaceutically acceptable salt thereof as an effective ingredient or an active ingredient.

Another embodiment of the present invention relates to a pharmaceutical composition for use in the prevention or treatment of a disease associated with TNAP overexpression or overactivation, comprising a compound of formula 1 or a pharmaceutically acceptable salt thereof as an effective ingredient or an active ingredient.

Another embodiment of the present invention relates to a method for treating a disease associated with TNAP overexpression or overactivation comprising administering a compound of formula 1 or a pharmaceutically acceptable salt thereof in a pharmaceutically effective amount to a subject in need thereof.

Another embodiment of the present invention relates to a use of the compound of formula 1 or the pharmaceutically acceptable salt thereof for the preparation of a medicament for preventing or treating a disease associated with TNAP overexpression or overactivation.

In the present invention, the disease associated with TNAP overexpression or overactivation includes a disease involving ectopic calcification. The disease associated with TNAP overexpression or overactivation includes the following diseases, but not always limited thereto: pseudoxanthoma elasticum (PXE), generalized arterial calcification of infancy (GACI), craniometaphyseal dysplasia (CMD), ossification of the yellow ligament (OYL), arterial calcification, calcification of joints, arthrosis deformans, osteoarthritis, ankylosis of the joint, idiopathic infantile arterial calcification (IIAC), ankylosing spondylitis (AS), tumoral calcinosis (TC), progressive osseous heteroplasia (POH), Keutel syndrome, vascular calcification associated with chronic renal failure (Chronic renal failure includes glomerulonephritis, IgA nephropathy, hypertensive nephropathy, and diabetic nephropathy), secondary parathyroid hyperplasia, metastatic calcification, calciphylaxis, calcific tendinitis of the longus colli muscle, fibrodysplasia ossificans progressive (FOP), calcific aortic stenosis, pericarditis calculosa, atherosclerotic vascular calcification, calcific uremic arteriopathy (CUA), Kawasaki disease, calcification due to obesity and aging, tibial arterial calcification, bone metastasis, prosthetic calcification, Paget 's disease, idiopathic basal ganglia calcification (IBGC), heterotopic ossification (HO), calcific aortic valve disease (CAVD), aortic valve stenosis (AVS), calcific tendinitis, ossification of the posterior longitudinal ligament (OPLL), ossification of the anterior longitudinal ligament (OALL), diffuse idiopathic skeletal hyperostosis (DISH), meniscal calcification, and peritoneal calcification.

The pharmaceutical composition may be formulated further comprising pharmaceutically acceptable additives.

The compound of formula 1 or the pharmaceutically acceptable salt thereof can be administered orally or parenterally and be used in general forms of pharmaceutical formulation. When formulated, it is usually prepared using generally used diluents or excipients such as fillers, extenders, binders, wetting agents, disintegrating agents and surfactants. Solid formulations for oral administration are tablets, pills, powders, granules and capsules. These solid formulations are prepared by mixing one or more the compounds with one or more suitable excipients such as starch, calcium carbonate, sucrose or lactose, gelatin, etc. Except for simple excipients, lubricants, for example magnesium stearate, talc, etc, can be used. Liquid formulations for oral administrations are suspensions, solutions, emulsions and syrups, and the above-mentioned formulations can contain various excipients such as wetting agents, sweeteners, aromatics and preservatives in addition to generally used simple diluents such as water and liquid paraffin. Formulations for parenteral administration are sterilized aqueous solutions, water-insoluble excipients, suspensions, and emulsions. Water-insoluble excipients and suspensions can contain propylene glycol, polyethylene glycol, vegetable oil like olive oil, injectable ester like ethylolate, etc.

The pharmaceutical composition comprising the compound of formula 1 or the pharmaceutically acceptable salt thereof as an active ingredient can be administered by parenterally and the parenteral administration includes subcutaneous injection, intravenous injection, intramuscular injection, or intrathoracic injection.

To prepare the compound of formula 1 or the pharmaceutically acceptable salt thereof as a formulation for parenteral administration, the compound represented of formula 1 or the pharmaceutically acceptable salt thereof is mixed with a stabilizer or a buffering agent in water to produce a solution or suspension, which is then formulated as ampoules or vials. The composition herein can be sterilized and additionally contains preservatives, stabilizers, wettable powders or emulsifiers, salts and/or buffers for the regulation of osmotic pressure, and other therapeutically useful materials, and the composition can be formulated by the conventional mixing, granulating or coating method.

The formulations for oral administration are exemplified by tablets, pills, hard/soft capsules, solutions, suspensions, emulsions, syrups, granules, elixirs, troches, etc. These formulations can include diluents (for example, lactose, dextrose, sucrose, mannitol, sorbitol, cellulose, and/or glycine) and lubricants (for example, silica, talc, stearate and its magnesium or calcium salt, and/or polyethylene glycol) in addition to the active ingredient. Tablets can include binding agents such as magnesium aluminum silicate, starch paste, gelatin, methylcellulose, sodium carboxymethylcellulose and/or polyvinylpyrolidone, and if necessary disintegrating agents such as starch, agarose, alginic acid or its sodium salt or azeotropic mixtures and/or absorbents, coloring agents, flavors, and sweeteners can be additionally included thereto.

In the present invention, the term "pharmaceutically effective amount" means containing in a dosage range that brings about a preventive, ameliorative, or therapeutic effect, and the dosage range may vary depending on the severity and formulation, and the number of applications may also vary depending on the age, weight, and constitution of a subject. In one specific embodiment of the present invention, the compound of formula 1 or the pharmaceutically acceptable salt thereof may be included in the pharmaceutical composition of the present invention in an amount of, for example, 0.001 mg/kg or more, preferably 0.1 mg/kg or more, 1 mg/kg or more, 10 mg/kg or more, 100 mg/kg or more, or 250 mg/kg or more, and may be included within an amount that does not cause fatal toxicity. For example, it may be selected from a range of 10 g/kg or less, or 1 g/kg or less. The quantitative upper limit of the compound of formula 1 or the pharmaceutically acceptable salt thereof included in the pharmaceutical composition of the present invention may be selected by those skilled in the art within an appropriate range.

In the present invention, the term "prevention" means preventing the occurrence of symptoms of a disease associated with TNAP overexpression or overactivation in advance by administering, ingesting or applying the pharmaceutical composition of the present invention to a subject not suffering from a disease associated with TNAP overexpression or overactivation, thereby suppressing or blocking the symptoms of a disease associated with TNAP overexpression or overactivation.

In the present invention, the term "treatment" includes complete cure of symptoms of a disease associated with TNAP overexpression or overactivation as well as partial cure, amelioration, and alleviation of symptoms of a disease associated with TNAP overexpression or overactivation as a result of administering the pharmaceutical composition of the present invention to a subject suffering from renal disease.

The compound of formula 1 of the present invention or the pharmaceutically acceptable salt thereof can be prepared according to the following reaction formula.

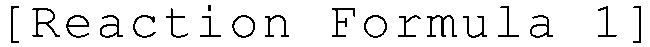

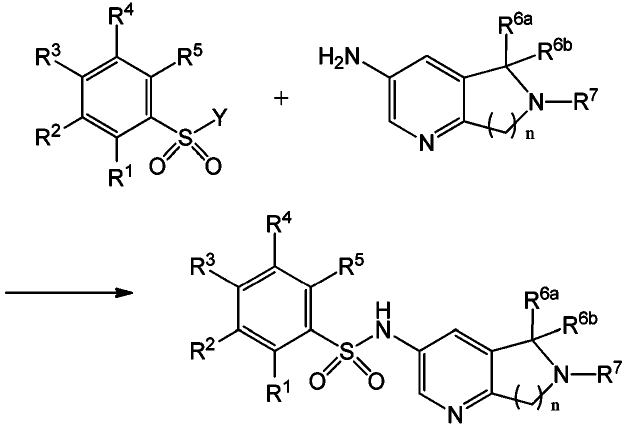

In reaction formula 1 above, R¹ to R⁷ and n are as defined in formula 1 of this specification. Y can be halogen, specifically Cl.

The above reaction can be carried out in the presence of a base, preferably an amine base, wherein the base preferably includes tertiary amine such as triethylamine, pyridine, etc. The reaction temperature is not particularly limited, but the reaction may be performed at, for example, 5 to 40°C or at room temperature. However, the initial mixing of the reactants may be performed at a low temperature, for example, below 0°C, or at -30 to 0°C, and then the temperature may be increased after mixing. The above reaction can be carried out in an organic solvent, for example, dichloromethane, THF, diethyl ether, or chloroform.

Hereinafter, specific examples for the compound according to the present invention and the preparation method therefor are presented. However, the following examples are provided only for understanding the invention and should not be construed as limiting the present invention.

### <Manufacturing examples of intermediates>

The synthesis method of the intermediates used in the present invention is as follows.

### Intermediate 1. Preparation of 3-amino-6,7-dihydro-5H-pyrrolo[3,4-b]pyridine-5-one

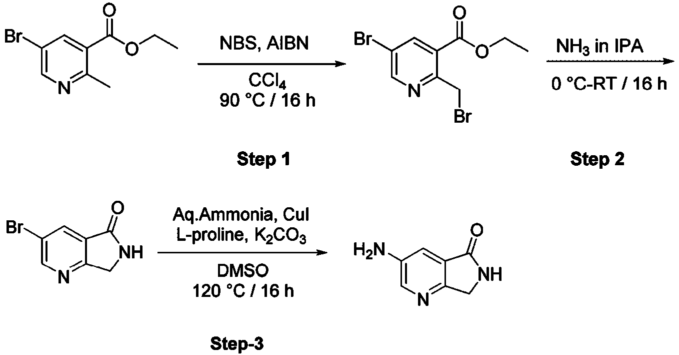

### Step 1. Preparation of ethyl 5-bromo-2-(bromomethyl)nicotinate

5-Bromo-2-methylnicotinate (9.70 g, 39.75 mmol, 1.0 eq) was dissolved in 100 mL of carbon tetrachloride, and then N-bromosuccinimide (7.07 g, 39.75 mmol, 1.0 eq) and azobisisobutyronitrile (1.95 g, 11.92 mmol, 0.3 eq) were slowly added while stirring at room temperature. The reaction temperature was raised to 90°C and stirred for 16 hours. The progress of the reaction was confirmed using TLC. Upon completion of the reaction, the temperature was lowered to room temperature. The mixture at room temperature was filtered and washed with ethyl acetate and petroleum ether. The filtrate was extracted with water and ethyl acetate (3 x 30 ml). The organic layer was washed with brine, treated with sodium sulfate, and the filtrate was concentrated. The concentrated mixture was purified by silica gel column chromatography using ethyl acetate/pet ether 5% as a developing solvent to give a target compound as a red solid (11.10 g, yield: mixture).

LCMS (m/z) : 322.06 (M+H).

### Step 2. Preparation of 3-bromo-6,7-dihydro-5H-pyrrolo[3,4-b]pyridine-5-one

To a sealed reaction vessel, 110 mL of ammonia-isopropyl alcohol solution (2 M in IPA) was added, and 5-bromo-2-(bromomethyl)nicotinate (5.60 g, 17.44 mmol, 1.0 eq) was added thereto at 0°C. The mixture was warmed to room temperature and stirred for 16 hours. The progress of the reaction was confirmed using TLC. Upon completion of the reaction, the mixture was filtered and washed with 80% ethyl acetate/pet ether. The washed solid was dried to give a target compound (2.70 g, yield: 64% (result of the second step reaction)).

¹H-NMR (400MHz, DMSO-d₆) : δ4.41 (s,2H), 8.30 (d,*J* = 2.20 Hz, 1H), 8.89 (d, *J* = 2.20 Hz, 1H), 8.94 (brs, 1H).

LCMS (m/z) : 215.06 (M+H).

### Step 3. Preparation of 3-amino-6,7-dihydro-5H-pyrrolo[3,4-b]pyridine-5-one

3-Bromo-6,7-dihydro-5H-pyrrolo[3,4-b]pyridine-5-one (3.80 g, 17.92 mmol, 1.0 eq) was dissolved in 45 mL of deoxygenated dimethylsulfoxide solution. Ammonia aqueous solution (25%) (6.09 ml, 89.62 mmol, 5.0 eq), copper iodide(I) (1.36 g, 7.17 mmol, 0.4 eq), potassium carbonate (12.36 g, 89.62 mmol, 5.0 eq), and L-proline (1.64 g, 14.34 mmol, 0.8 eq) were added to the mixed solution at room temperature. The mixture was stirred at 120°C for 16 hours. The progress of the reaction was confirmed using TLC. Upon completion of the reaction, the temperature of the reaction mixture was lowered to room temperature. The mixture at room temperature was filtered through a celite pad and washed with 10% methanol/dichloromethane. The filtrate was concentrated under reduced pressure. The concentrated mixture was purified by silica gel column chromatography using 2% methanol/ethyl acetate as a developing solvent to give a target compound as a yellow solid (1.50 g, yield: 56%) .

¹H-NMR (400MHz, DMSO-d₆) δ 4.20 (s, 2H), 5.53 (s, 2H), 7.12 (d, *J* = 2.75 Hz, 1H), 8.10 (d, *J* = 2.50 Hz, 1H), 8.52 (brs, 1H).

LCMS (m/z) : 150.15 (M+H).

### Intermediate 2. Preparation of 3-amino-7,8-dihydro-1,6-naphthyridine-5(6H)-one

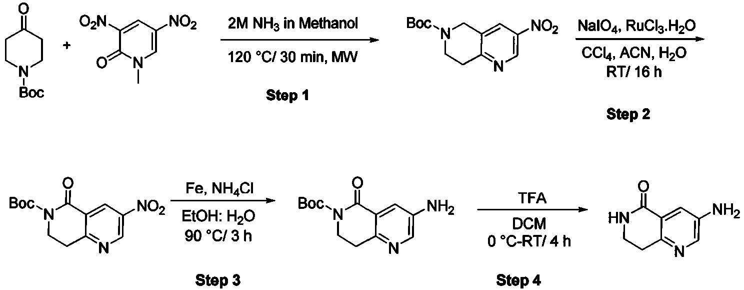

### Step 1. Preparation of tert-butyl 3-nitro-7,8-dihydro-1,6-naphthyridine-6(5H)-carboxylate

Tert-butyl 4-oxopiperidine-1-carboxylate (0.70 g, 3.51 mmol, 1.0 eq) and 1-methyl-3,5-dinitropyridine-2(1H)-one (0.70 g, 3.51 mmol, 1.0 eq) were added to 10 mL of ammonia solution (2 M in MeOH) at room temperature. The mixture was stirred using a microwave reactor at 120°C for 30 minutes. The progress of the reaction was confirmed using TLC. Upon completion of the reaction, the temperature of the reaction mixture was lowered to room temperature. The concentrated mixture was extracted using water and ethyl acetate. The organic layer was washed with brine, treated with sodium sulfate, and the filtrate was concentrated. The concentrated mixture was purified by silica gel column chromatography using 28% ethyl acetate/pet ether as a developing solvent to give a target compound as a solid (0.8 g, yield: 82%).

¹H-NMR (400 MHz, CDCl₃) δ 1.51 (s, 9H), 3.12 (t,*J* = 5.88 Hz, 2H), 3.80 (t, *J* = 6.00 Hz, 2H), 4.72 (s, 2H), 8.23 (d, *J* = 2.40 Hz, 1H), 9.25 (d, *J* = 2.25 Hz, 1H).

LCMS (m/z) : 280.20 (M+H).

### Step 2. Preparation of tert-butyl 3-nitro-5-oxo-7,8-dihydro-1,6-naphthyridine-6(5H)-carboxylate

While stirring a solution containing tert-butyl 3-nitro-7,8-dihydro-1,6-naphthyridine-6(5H)-carboxylate (6.50 g, 23.29 mmol, 1.0 eq) in a mixed solution of 95 mL of carbon tetrachloride and 12 mL of acetonitrile, 40 mL of sodium periodate aqueous solution (14.88 g, 69.89 mmol, 3.0 eq) and 20 mL of ruthenium chloride aqueous solution (1.44 g, 6.98 mmol, 0.3 eq) were added at room temperature. The mixture was stirred at room temperature for 16 hours. The progress of the reaction was confirmed using TLC. Upon completion of the reaction, the mixture was filtered through a celite pad. The filtrate was extracted using water and dichloromethane. The organic layer was washed with brine, treated with sodium sulfate, and the filtrate was concentrated. The concentrated mixture was purified by silica gel column chromatography using 25% ethyl acetate/pet ether as a developing solvent to give a target compound as a solid (3.60 g, yield: 53%).

¹H-NMR (400 MHz, CDCl₃) δ 1.60 (s, 9H), 3.35 (t,*J* = 6.48 Hz, 2H), 4.14 (t, *J* = 6.36 Hz, 2H), 9.17 (d, *J* = 2.69 Hz, 1H), 9.47 (d, *J* = 2.45 Hz, 1H).

LCMS (m/z) : 294.25 (M+H).

### Step 3. Preparation of tert-butyl 3-amino-5-oxo-7,8-dihydro-1,6-naphthyridine-6(5H)-carboxylate

While stirring a solution containing tert-butyl 3-nitro-5-oxo-7,8-dihydro-1,6-naphthyridine-6(5H)-carboxylate (3.60 g, 12.28 mmol, 1.0 eq) in a mixed solution of 100 mL of ethanol and 30 mL of water, ammonium chloride (3.25 g, 61.43 mmol, 5.0 eq) and iron (2.75 g, 49.14 mmol, 4.0 eq) were added at room temperature. The mixture was stirred at 80°C for 3 hours. The progress of the reaction was confirmed using TLC. Upon completion of the reaction, the temperature of the mixture was lowered to room temperature, the mixture was filtered through a celite pad, and concentrated under reduced pressure. The concentrated mixture was extracted using water and ethyl acetate. The organic layer was washed with brine, treated with sodium sulfate, and the filtrate was concentrated. The concentrated mixture was purified by silica gel column chromatography using 80% ethyl acetate/pet ether as a developing solvent to give a target compound (1.70 g, yield: 53%).

LCMS (m/z) : 264.22 (M+H).

### Step 4. Preparation of 3-amino-7,8-dihydro-1,6-naphthyridine-5(6H)-one

Trifluoroacetic acid (4.97 ml, 64.63 mmol, 10.0 eq) was slowly added to a solution containing tert-butyl 3-amino-5-oxo-7,8-dihydro-1,6-naphthyridine-6(5H)-carboxylate (1.70 g, 6.46 mmol, 1.0 eq) in 20 mL of dichloromethane solution at 0°C while stirring. The mixture was stirred at room temperature for 4 hours. The progress of the reaction was confirmed using TLC. Upon completion of the reaction, the mixture was concentrated and alkalized using 2 M ammonia solution (2 M in MeOH). The alkalized mixture was concentrated under reduced pressure. The concentrated mixture was purified by silica gel column chromatography using 95% ethyl acetate/pet ether as a developing solvent to give a target compound as a solid (1.04 g, yield: 99%).

¹H-NMR (400 MHz, DMSO-d₆) δ 2.82 (t,*J* = 6.75 Hz, 2H), 3.36 (td, *J* = 6.75, 2.75 Hz, 2H), 5.36 (s, 2H), 7.34 (d, *J* = 2.75 Hz, 1H), 7.90 (brs, 1H), 7.95 (d, *J* = 2.75 Hz, 1H).

LCMS (m/z) : 164.08 (M+H).

### Intermediate 3. Preparation of 3-amino-6,7,8,9-tetrahydro-5H-pyrido[3,2-c]azepine-5-one

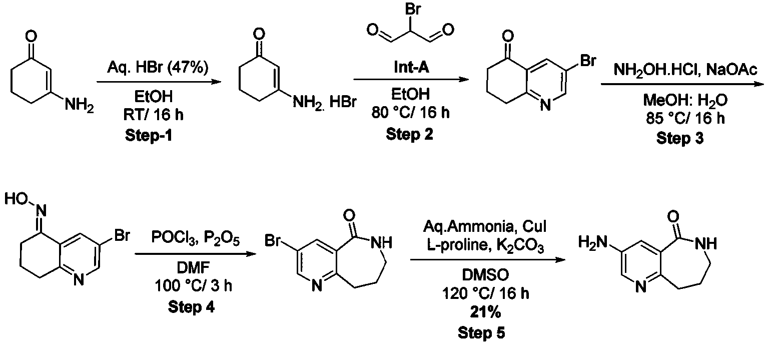

### Step 1. 3-aminocyclohex-2-en-1-one hydrobromide

47% Aq. HBr solution (38.77 mL, 225.22 mmol, 1.0 eq.) was added to 3-aminocyclohex-2-en-1-one (25.0 g, 225.22 mmol, 1.0 eq.) solution dissolved in 500 ml of ethanol at room temperature, and the reaction mixture was stirred at the same temperature for 16 hours. The progress of the reaction was confirmed using TLC. Upon completion of the reaction, the reaction mixture was evaporated under reduced pressure to obtain a residue. The residue was co-distilled with toluene to give 3-aminocyclohex-2-en-1-one hydrobromide (40.0 g).

### Step 2. Preparation of 3-bromo-7,8-dihydroquinoline-5(6H)-one

2-Bromomalonaldehyde (34.60 g, 229.16 mmol, 1.1 eq.) was added to a stirred solution of 3-aminocyclohex-2-en-1-one hydrobromide (40.0 g, 208.33 mmol, 1.0 eq.) dissolved in 600 ml of ethanol at room temperature. The reaction mixture was heated to 80°C and stirred at the same temperature for 16 hours. The progress of the reaction was confirmed using TLC and LC-MS. Upon completion of the reaction, the reaction mixture was cooled to room temperature and evaporated under reduced pressure to obtain a residue. The residue was treated with water and extracted with ethyl acetate. The organic layer was dried over anhydrous Na₂SO₄, filtered, and the filtrate was evaporated under reduced pressure to obtain a mixture. The mixture was purified by silica gel column chromatography and eluted with 30% ethyl acetate/pet ether to give 3-bromo-7,8-dihydroquinoline-5(6H)-one (28.0 g).

LC-MS(M+H): 225.99.

### Step 3. Preparation of (E)-3-bromo-7,8-dihydroquinoline-5(6H)-one oxime

Hydroxylamine hydrochloride (24.26 g, 346.66 mmol, 3.0 eq.) and sodium acetic acid (28.42 g, 346.66 mmol, 3.0 eq.) were added to a stirred solution of 3-bromo-7,8-dihydroquinoline-5(6H)-one (26.0 g, 115.55 mmol, 1.0 eq.) in a mixed solution of methanol and water (325.0 mL, 4:1) at room temperature. The reaction mixture was heated to 85°C and stirred at the same temperature for 16 hours. The progress of the reaction was confirmed using TLC and LC-MS. Upon completion of the reaction, the reaction mixture was cooled to room temperature and evaporated under reduced pressure to obtain a residue. The residue was diluted with water to precipitate the solid. The precipitated solid was filtered, washed with n-pentane, and dried under vacuum to give (E)-3-bromo-7,8-dihydroquinoline-5(6H)-one oxime (16.0 g).

¹H-NMR(500 MHz, DMSO-d6) δ 1.80-1.86(m, 2H), 2.63-2.68(m, 2H), 2.81-2.84(m, 2H), 8.20-8.24(m, 1H), 8.55-8.61(m, 1H), 11.57(s, 1H).

LC-MS(M+H): 241.17.

### Step 4. Preparation of 3-bromo-6,7,8,9-tetrahydro-5H-pyrido[3,2-c]azepine-5-one

Phosphorus pentoxide (13.43 g, 93.33 mmol, 1.4 eq.) and N,N-dimethylformamide (2.60 mL, 33.33 mmol, 0.5 eq.) were added to a stirred solution of (E)-3-bromo-7,8-dihydroquinoline-5(6H)-one oxime (16.0 g, 66.66 mmol, 1.0 eq.) dissolved in phosphate chloride (128 mL) at 0°C. The reaction mixture was heated to 100°C and stirred at the same temperature for 3 hours. The progress of the reaction was confirmed using TLC and LC-MS. Upon completion of the reaction, the reaction mixture was cooled to room temperature and evaporated under reduced pressure to obtain a residue. The residue was alkalized with 3 N NaOH solution and extracted with ethyl acetate. The organic layer was dried over anhydrous Na₂SO₄, filtered, and the filtrate was evaporated under reduced pressure to obtain a mixture. The mixture was purified by silica gel column chromatography and eluted with 80% ethyl acetate/pet ether to give 3-bromo-6,7,8,9-tetrahydro-5H-pyrido[3,2-c]azepine-5-one (2.20 g).

¹H NMR(400MHz, DMSO-d6) δ 1.96-2.00(m, 2H), 2.88-2.91(m, 2H), 2.96-3.02(m, 2H), 8.03(d, J = 2.45Hz, 1H), 8.37(brs, 1H), 8.70(d, J = 2.20Hz, 1H).

LC-MS((M+2) +H, isotope mass): 242.92.

### Step 5. Preparation of 3-amino-6,7,8,9-tetrahydro-5H-pyrido[3,2-c]azepine-5-one

L-proline (0.843 g, 7.33 mmol, 0.8 eq.), copper iodide(I) (0.696 g, 3.66 mmol, 0.4 eq.), and potassium carbonate (6.32 g, 45.83 mmol, 5.0 eq.) were added to a stirred solution of 3-bromo-6,7,8,9-tetrahydro-5H-pyrido[3,2-c]azepine-5-one (2.20 g, 9.16 mmol, 1.0 eq.) dissolved in dimethyl sulfoxide (33.0 mL), and then 25% ammonia aqueous solution (3.11 mL, 45.83 mmol, 5.0 eq.) was added to the sealed tube at room temperature. The reaction mixture was heated to 120°C and stirred at the same temperature for 16 hours. The progress of the reaction was confirmed using TLC and LC-MS. Upon completion of the reaction, the reaction mixture was cooled to room temperature and evaporated under reduced pressure to obtain a residue. The residue was diluted with 10% methanol in dichloromethane, filtered through a celite pad of, and the filtrate was evaporated under reduced pressure to obtain a mixture. The mixture was purified by -NH silica gel column chromatography, eluted with 2% methanol/dichloromethane, and recrystallized with acetonitrile to give 3-amino-6,7,8,9-tetrahydro-5H-pyrido[3,2-c]azepine-5-one (0.34 g, 21%).

¹H-NMR(400MHz, DMSO-d6) δ 1.87(quin, J = 6.82Hz, 2H), 2.73(t, J = 7.25Hz, 2H), 2.93(q, J = 6.42Hz, 2H), 5.31(s, 2H), 7.05(d, J = 2.75Hz, 1H), 7.89(d, J = 2.75Hz, 1H), 8.02(t, J = 5.50Hz, 1H).

LC-MS(M+H): 178.12.

### Intermediate 4. Preparation of neopentyl-3-amino-7,8-dihydro-1,6-naphthylpyridine-6(5H)-carboxylate

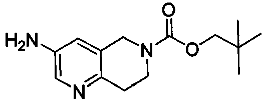

### Step 1. Preparation of neopentyl-3-nitro-7,8-dihydro-1,6-naphthylpyridine-6(5H)-carboxylate

Neopentyl carbonochloroformate (0.24 mL, 1.611 mmol, 1.5 eq) was slowly added to a solution of 3-nitro-5,6,7,8-tetrahydro-1,6-naphthyridine (192 mg, 1.074 mmol, 1.0 eq) and triethylamine (0.6 mL, 4.296 mmol, 4 eq) in 6 mL of dichloromethane solution at 0°C while stirring. The mixture was stirred at room temperature for 1 hour. The progress of the reaction was confirmed using TLC. The mixture was extracted using water and ethyl acetate. The organic layer was washed with brine, treated with sodium sulfate, and the filtrate was concentrated. The concentrated mixture was purified by silica gel column chromatography using acetate/hexane as a developing solvent to give a target compound as a solid (0.304 g, yield: 96%).

LCMS (m/z) : 294.1 (M+H).

### Step 2. Preparation of neopentyl-3-amino-7,8-dihydro-1,6-naphthylpyridine-6(5H)-carboxylate

4,4'-Bipyridine (32 mg, 0.208 mmol, 0.2 eq) 4,4'-bipyridine was slowly added to a solution of neopentyl-3-nitro-7,8-dihydro-1,6-naphthylpyridine-6(5H)-carboxylate (304 mg, 1.038 mmol, 1.0 eq) and tetrahydroxydiboron (279 mg, 3.113 mmol, 3.0 eq) in 2 mL of dimethylformamide solution at 0°C while stirring. The mixture was stirred for an additional 30 minutes, then warmed to room temperature and stirred for more than 1 hour. The progress of the reaction was confirmed using TLC. The mixture was extracted using water and ethyl acetate. The organic layer was washed with brine, treated with sodium sulfate, and the filtrate was concentrated. The concentrated mixture was purified by silica gel column chromatography using methanol/dichloromethane as a developing solvent to give a target compound as a solid (158 mg, yield: 58%).

LCMS (m/z) : 264.1 (M+H).

### Intermediate 5. Preparation of 2-fluoroethyl-3-amino-7,8-dihydro-1,6-naphthylpyridine-6(5H)-carboxylate

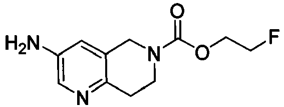

### Step 1. Preparation of 2-fluoroethyl-3-nitro-7,8-dihydro-1,6-naphthylpyridine-6(5H)-carboxylate

2-Fluoroethyl carbonochloroformate (0.15 mL, 1.611 mmol, 1.5 eq) was slowly added to a solution of 3-nitro-5,6,7,8-tetrahydro-1,6-naphthyridine (192 mg, 1.074 mmol, 1.0 eq) and triethylamine (0.6 mL, 4.296 mmol, 4 eq) in 6 mL of dichloromethane solution at 0°C while stirring. The mixture was stirred at room temperature for 1 hour. The progress of the reaction was confirmed using TLC. The mixture was extracted using water and ethyl acetate. The organic layer was washed with brine, treated with sodium sulfate, and the filtrate was concentrated. The concentrated mixture was purified by silica gel column chromatography using ethyl acetate/hexane as a developing solvent to give a target compound as a solid (0.280 g, yield: 97%).

LCMS (m/z) : 270.2 (M+H).

### Step 2. Preparation of 2-fluoroethyl-3-amino-7,8-dihydro-1,6-naphthylpyridine-6(5H)-carboxylate

2-Fluoroethyl-3-nitro-7,8-dihydro-1,6-naphthylpyridine-6(5H)-carboxylate was reacted according to the manufacturing method of step 2 of Intermediate 4 to give a target compound as a solid (179 mg, yield: 72%).

LCMS (m/z) : 240.2 (M+H).

### Intermediate 6. Preparation of 3-amino-N-pentyl-7,8-dihydro-1,6-naphthyridine-6(5H)-carboxamide

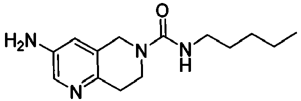

### Step 1. Preparation of 3-nitro-N-pentyl-7,8-dihydro-1,6-naphthyridine-6(5H)-carboxamide

A target compound (181 mg, yield: 86%) was obtained by reacting according to the manufacturing method of step 1 of Example 104 using 1-pentylamine as an amine substituent.

LCMS (m/z) : 293.1 (M+H).

### Step 2. Preparation of 3-amino-N-pentyl-7,8-dihydro-1,6-naphthyridine-6(5H)-carboxamide

3-Nitro-N-pentyl-7,8-dihydro-1,6-naphthyridine-6(5H)-carboxamide was reacted according to the manufacturing method of step 2 of Intermediate 4 to give a target compound (153 mg, yield: 94%).

LCMS (m/z) : 263.1 (M+H).

### Intermediate 7. Preparation of 3-amino-N-(2,2-difluoroethyl)-7,8-dihydro-1,6-naphthyridine-6(5H)-carboxamide

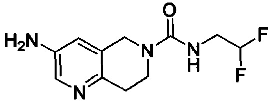

### Step 1. Preparation of N-(2,2-difluoroethyl)-3-nitro-7,8-dihydro-1,6-naphthyridine-6(5H)-carboxamide

A target compound (69 mg, yield: 21%) was obtained by reacting according to the manufacturing method of step 1 of Example 104 using 2,2-difluoroethane-1-amine hydrochloride as an amine substituent.

LCMS (m/z) : 287.1 (M+H).

### Step 2. Preparation of 3-amino-N-(2,2-difluoroethyl)-7,8-dihydro-1,6-naphthyridine-6(5H)-carboxamide

N-(2,2-difluoroethyl)-3-nitro-7,8-dihydro-1,6-naphthyridine-6(5H)-carboxamide was reacted according to the manufacturing method of step 2 of Intermediate 4 to give a target compound (59 mg, yield: 96%).

LCMS (m/z) : 257.1 (M+H).

### Intermediate 8. Preparation of (3-amino-7,8-dihydro-1,6-naphthyridine-6(5H)-yl)(morpholino)methanone

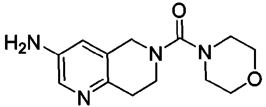

### Step 1. Preparation of N-morpholino(3-nitro-7,8-dihydro-1,6-naphthyridine-6(5H)-yl)methanone

A target compound (72 mg, yield: 44%) was obtained by reacting according to the manufacturing method of step 1 of Example 104 using morpholine as an amine substituent.

LCMS (m/z) : 293.1 (M+H).

### Step 2. Preparation of (3-amino-7,8-dihydro-1,6-naphthyridine-6(5H)-yl)(morpholino)methanone

N-morpholino(3-nitro-7,8-dihydro-1,6-naphthyridine-6(5H)-yl)methanone was reacted according to the manufacturing method of step 2 of Intermediate 4 to give a target compound (63 mg, yield: 97%).

LCMS (m/z) : 263.1 (M+H).

### <Examples: Preparation of TNAP inhibitor compounds >

Hereinafter, the present invention will be described more specifically by the following examples. However, the content of the present invention is not limited by the following examples.

### Example 1. Preparation of 5-chloro-2-methoxy-N-(5-oxo-6,7-dihydro-5H-pyrrolo[3,4-b]pyridine-3-yl)benzenesulfonamide

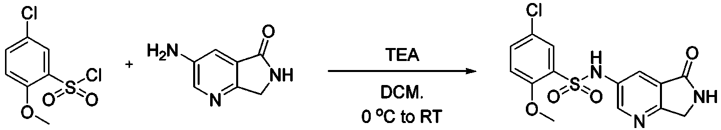

Triethylamine (0.03 mL, 0.249 mmol, 2.0 eq) was added to a solution of 5-chloro-2-methoxybenzenesulfonyl chloride (30 mg, 0.124 mmol, 1.0 eq) and Intermediate 1 (3-amino-6,7-dihydro-5H-pyrrolo[3,4-b]pyridine-5-one) (20.42 mg, 0.137 mmol, 1.1 eq) in 20 mL of dichloromethane at 0°C. The mixture was stirred at room temperature for 12 hours. The progress of the reaction was confirmed using TLC. Upon completion of the reaction, the mixture was concentrated. The concentrated mixture was purified by silica gel column chromatography using methanol/dichloromethane as a developing solvent to give a target compound as a solid (22 mg, yield: 50%).

¹H-NMR (400 MHz, DMSO-*d*₆) δ10.67 (s, 1H), 8.80 (s, 1H), 8.44 (d,*J* = 2.8 Hz, 1H), 7.69 (dd, *J* = 2.9, 1.2 Hz, 1H), 7.66 - 7.59 (m, 2H), 7.20 (dd, *J* = 8.2, 1.7 Hz, 1H), 4.28 (s, 2H), 3.82 (s, 3H).

LCMS (m/z) : 345.1 (M+H).

### Example 2. Preparation of 5-bromo-2-methoxy-N-(5-oxo-6,7-dihydro-5H-pyrrolo[3,4-b]pyridine-3-yl)benzenesulfonamide

A target compound (17 mg, yield: 41%) was obtained by reacting 5-bromo-2-methoxybenzenesulfonyl chloride with the compound of Intermediate 1 according to the manufacturing method of Example 1.

¹H-NMR (400 MHz, DMSO-*d*₆) δ 10.66 (s, 1H), 8.80 (s, 1H), 8.47-8.40 (m,1H), 7.82-7.75 (m,1H), 7.74 (dq, *J* = 8.9, 1.9 Hz, 1H), 7.64 (q, *J* = 1.9 Hz, 1H), 7.14 (dt, *J* = 8.9, 1.7 Hz, 1H), 4.28 (s, 2H), 3.81 (s, 3H).

LCMS (m/z) : 398.1 (M+H).

### Example 3. Preparation of 2,5-dimethoxy-N-(5-oxo-6,7-dihydro-5H-pyrrolo[3,4-b]pyridine-3-yl)benzenesulfonamide

A target compound (20 mg, yield: 45%) was obtained by reacting 2,5-dimethoxybenzenesulfonyl chloride with the compound of Intermediate 1 according to the manufacturing method of Example 1.

¹H-NMR (400 MHz, DMSO-*d*₆) δ 10.53 (s, 1H), 8.77 (s, 1H), 8.44 (t,*J* = 2.2 Hz, 1H), 7.65 (t, *J* = 2.1 Hz, 1H), 7.27 - 7.20 (m, 1H), 7.17 - 7.05 (m, 2H), 4.27 (s, 2H), 3.75 (s, 3H), 3.68 (s, 3H).

LCMS (m/z) : 350.2 (M+H).

### Example 4. Preparation of 5-fluoro-2-methoxy-N-(5-oxo-6,7-dihydro-5H-pyrrolo[3,4-b]pyridine-3-yl)benzenesulfonamide

A target compound (24 mg, yield: 32%) was obtained by reacting 5-fluoro-2-methoxybenzenesulfonyl chloride with the compound of Intermediate 1 according to the manufacturing method of Example 1.

¹H-NMR (400 MHz, DMSO-*d*₆) δ 10.67 (s, 1H), 8.80 (s, 1H), 8.48-8.39 (m, 1H), 7.70-7.61 (m, 1H), 7.59-7.51 (m, 1H), 7.51-7.40 (m, 1H), 7.24-7.15 (m, 1H), 4.27 (s, 2H), 3.80 (s, 3H).

LCMS (m/z) : 338.1 (M+H).

### Example 5. Preparation of 5-bromo-N-(5-oxo-6,7-dihydro-5H-pyrrolo[3,4-b]pyridine-3-yl)-2,3-dihydrobenzofuran-7-sulfonamide

A target compound (32 mg, yield: 46%) was obtained by reacting 5-bromo-2,3-dihydrobenzofuran-7-sulfonyl chloride with the compound of Intermediate 1 according to the manufacturing method of Example 1.

¹H-NMR (400 MHz, DMSO-*d*₆) δ 10.84 (s, 1H), 8.79 (s, 1H), 8.46-8.42 (m, 1H), 7.67-7.61 (m, 2H), 7.52-7.48 (m, 1H), 4.61 (td, *J* = 8.9, 3.4 Hz, 2H), 4.29 (d, *J* = 3.4 Hz, 2H), 3.23-3.15 (m, 2H).

LCMS (m/z) : 409.9 (M+H).

### Example 6. Preparation of 5-chloro-2-methoxy-N-(5-oxo-5,6,7,8-tetrahydro-1,6-naphthyridine-3-yl)benzenesulfonamide

A target compound (28 mg, yield: 37%) was obtained by reacting 5-chloro-2-methoxybenzenesulfonyl chloride with the compound of Intermediate 2 (3-amino-7,8-dihydro-1,6-naphthyridine-5(6H)-one) according to the manufacturing method of Example 1.

¹H-NMR (400 MHz, DMSO-*d*₆) δ 10.49 (s, 1H), 8.35-8.27 (m, 1H), 8.12-8.06 (m, 1H), 7.89-7.81 (m, 1H), 7.71-7.58 (m, 2H), 7.26-7.17 (m, 1H), 3.82 (s, 3H), 3.41-3.26 (m, 2H), 2.95-2.84 (m, 2H).

LCMS (m/z) : 368.1 (M+H).

### Example 7. Preparation of 2,5-dimethoxy-N-(5-oxo-5,6,7,8-tetrahydro-1,6-naphthyridine-3-yl)benzenesulfonamide

A target compound (24 mg, yield: 31%) was obtained by reacting 2,5-dimethoxybenzenesulfonyl chloride with the compound of Intermediate 2 according to the manufacturing method of Example 1.

¹H-NMR (400 MHz, DMSO-*d*₆) δ 10.36 (s, 1H), 8.34-8.27 (m, 1H), 8.07 (s, 1H), 7.89-7.83 (m, 1H), 7.26-7.19 (m, 1H), 7.18-7.05 (m, 2H), 3.76 (s, 3H), 3.69 (s, 3H), 3.37-3.31 (m, 2H), 2.92-2.83 (m, 2H).

LCMS (m/z) : 364.2 (M+H).

### Example 8. Preparation of 5-fluoro-2-methoxy-N-(5-oxo-5,6,7,8-tetrahydro-1,6-naphthyridine-3-yl)benzenesulfonamide

A target compound (25 mg, yield: 32%) was obtained by reacting 5-fluoro-2-methoxybenzenesulfonyl chloride with the compound of Intermediate 2 according to the manufacturing method of Example 1.

¹H-NMR (400 MHz, DMSO-*d*₆) δ 10.46 (s, 1H), 8.30 (d,*J* = 2.7 Hz, 1H), 8.07 (s, 1H), 7.85 (d, *J* = 2.7 Hz, 1H), 7.54 - 7.40 (m, 2H), 7.19 (dd, *J* = 9.2, 4.0 Hz, 1H), 3.80 (s, 3H), 3.34 (q, *J* = 5.8, 5.3 Hz, 2H), 2.88 (t, *J* = 6.7 Hz, 2H).

LCMS (m/z) : 352.2 (M+H).

### Example 9. Preparation of 5-bromo-2-methoxy-N-(5-oxo-5,6,7,8-tetrahydro-1,6-naphthyridine-3-yl)benzenesulfonamide

A target compound (42 mg, yield: 36%) was obtained by reacting 5-bromo-2-methoxybenzenesulfonyl chloride with the compound of Intermediate 2 according to the manufacturing method of Example 1.

¹H-NMR (400 MHz, DMSO-*d*₆) δ 10.47 (s, 1H), 8.30 (d,*J* = 2.6 Hz, 1H), 8.07 (s, 1H), 7.85 (d, *J* = 2.7 Hz, 1H), 7.79 - 7.70 (m, 2H), 7.15 (d, *J* = 8.8 Hz, 1H), 3.81 (s, 3H), 3.35 (td, *J* = 6.7, 2.8 Hz, 2H), 2.89 (t, *J* = 6.7 Hz, 2H).

LCMS (m/z) : 412.1 (M+H).

### Example 10. Preparation of 5-bromo-N-(5-oxo-5,6,7,8-tetrahydro-1,6-naphthyridine-3-yl)-2,3-dihydrobenzofuran-7-sulfonamide

A target compound (29 mg, yield: 41%) was obtained by reacting 5-bromo-2,3-dihydrobenzofuran-7-sulfonyl chloride with the compound of Intermediate 2 according to the manufacturing method of Example 1.

¹H-NMR (400 MHz, DMSO-*d*₆) δ 10.70 (s, 1H), 8.34-8.25 (m, 1H), 8.12-8.04 (m, 1H), 7.88-7.78 (m, 1H), 7.67-7.58 (m, 1H), 7.52-7.43 (m, 1H), 4.68-4.57 (m, 2H), 3.41-3.31 (m, 2H), 3.23-3.07 (m, 2H), 2.94-2.85 (m, 2H).

LCMS (m/z) : 425.0 (M+H).

### Example 11. Preparation of 2-bromo-5-fluoro-N-(5-oxo-6,7-dihydro-5H-pyrrolo[3,4-b]pyridine-3-yl)benzenesulfonamide

A target compound (110 mg, yield: 80%) was obtained by reacting 2-bromo-5-fluorobenzenesulfonyl chloride with the compound of Intermediate 1 according to the manufacturing method of Example 1.

¹H-NMR (400 MHz, DMSO-d6) δ 11.26 (s, 1H), 8.80 (s, 1H), 8.48 (dt, J = 4.4, 2.2 Hz, 1H), 8.00 - 7.79 (m, 2H), 7.63 (dt, J = 4.4, 2.2 Hz, 1H), 7.50 - 7.41 (m, 1H), 4.29 (s, 2H).

LCMS (m/z) : 387.2 (M+H).

### Example 12. Preparation of 2-bromo-4,6-difluoro-N-(5-oxo-6,7-dihydro-5H-pyrrolo[3,4-b]pyridine-3-yl)benzenesulfonamide

A target compound (55 mg, yield: 49%) was obtained by reacting 2-bromo-4,6-difluorobenzenesulfonyl chloride with the compound of Intermediate 1 according to the manufacturing method of Example 1.

¹H-NMR (400 MHz, DMSO-d6) δ 11.37 (s, 1H), 8.80 (s, 1H), 8.53 - 8.43 (m, 1H), 7.78 - 7.64 (m, 2H), 7.64 - 7.50 (m, 1H), 4.30 (s, 2H).

LCMS (m/z) : 405.1 (M+H).

### Example 13. Preparation of 5-chloro-2-methoxy-N-(5-oxo-6,7,8,9-tetrahydro-5H-pyrido[3,2-c]azepine-3-yl)benzenesulfonamide

A target compound (23 mg, yield: 29%) was obtained by reacting 5-chloro-2-methoxybenzenesulfonyl chloride with the compound of Intermediate 3 according to the manufacturing method of Example 1.

¹H-NMR (400 MHz, DMSO-d6) δ 10.47 (s, 1H), 8.34 - 8.08 (m, 2H), 7.74 - 7.45 (m, 3H), 7.31 - 7.09 (m, 1H), 3.80 (s, 3H), 2.92 - 2.64 (m, 4H), 1.96 - 1.75 (m, 2H).

LCMS (m/z) : 382 (M+H).

### Example 14. Preparation of 5-bromo-2-methoxy-N-(5-oxo-6,7,8,9-tetrahydro-5H-pyrido[3,2-c]azepine-3-yl)benzenesulfonamide

A target compound (25 mg, yield: 33%) was obtained by reacting 5-bromo-2-methoxybenzenesulfonyl chloride with the compound of Intermediate 3 according to the manufacturing method of Example 1.

¹H-NMR (400 MHz, DMSO-d6) δ 10.47 (s, 1H), 8.33 - 8.08 (m, 2H), 7.83 - 7.65 (m, 2H), 7.62 - 7.47 (m, 1H), 7.19 - 6.99 (m, 1H), 3.79 (s, 3H), 2.92 - 2.64 (m, 4H), 1.98 - 1.75 (m, 2H).

LCMS (m/z) : 427 (M+H).

### Example 15. Preparation of 5-fluoro-2-methoxy-N-(5-oxo-6,7,8,9-tetrahydro-5H-pyrido[3,2-c]azepine-3-yl)benzenesulfonamide

A target compound (21 mg, yield: 25%) was obtained by reacting 5-fluoro-2-methoxybenzenesulfonyl chloride with the compound of Intermediate 3 according to the manufacturing method of Example 1.

¹H-NMR (400 MHz, DMSO-d6) δ 10.46 (s, 1H), 8.32 - 8.10 (m, 2H), 7.61 -7.38 (m, 3H), 7.26 - 7.13 (m, 1H), 3.80 (s, 3H), 2.92 - 2.68 (m, 4H), 1.99 - 1.75 (m, 2H).

LCMS (m/z) : 366 (M+H).

### Example 16. Preparation of 2,5-dimethoxy-N-(5-oxo-6,7,8,9-tetrahydro-5H-pyrido[3,2-c]azepine-3-yl)benzenesulfonamide

A target compound (15 mg, yield: 19%) was obtained by reacting 2,5-dimethoxybenzenesulfonyl chloride with the compound of Intermediate 3 according to the manufacturing method of Example 1.

¹H-NMR (400 MHz, DMSO-d6) δ 10.34 (s, 1H), 8.30 - 8.13 (m, 2H), 7.56 (t, J = 2.1 Hz, 1H), 7.22 (dd, J = 3.1, 1.5 Hz, 1H), 7.19 - 7.06 (m, 2H), 3.75 (s, 3H), 3.69 (s, 3H), 2.91 - 2.69 (m, 4H), 1.94 - 1.76 (m, 2H).

LCMS (m/z) : 378 (M+H).

### Example 17. Preparation of 5-bromo-N-(5-oxo-6,7,8,9-tetrahydro-5H-pyrido[3,2-c]azepine-3-yl)-2,3-dihydrobenzofuran-7-sulfonamide

A target compound (18 mg, yield: 24%) was obtained by reacting 5-bromo-2,3-dihydrobenzofuran-7-sulfonyl chloride with the compound of Intermediate 3 according to the manufacturing method of Example 1.

¹H-NMR (400 MHz, DMSO-d6) δ 10.65 (s, 1H), 8.35 - 8.15 (m, 2H), 7.63 (s, 1H), 7.54 (d, J = 3.1 Hz, 1H), 7.47 (d, J = 1.8 Hz, 1H), 4.61 (t, J = 8.7 Hz, 2H), 3.19 (t, J = 8.7 Hz, 2H), 2.82 (dt, J = 30.9, 6.8 Hz, 4H), 1.95 - 1.81 (m, 2H).

LCMS (m/z) : 439 (M+H).

### Example 18. Preparation of N-(5-oxo-6,7-dihydro-5H-pyrrolo[3,4-b]pyridine-3-yl)-2,3-dihydrobenzofuran-7-sulfonamide

A target compound (13 mg, yield: 20%) was obtained by reacting 2,3-dihydrobenzofuran-7-sulfonyl chloride with the compound of Intermediate 1 according to the manufacturing method of Example 1.

¹H-NMR (400 MHz, DMSO-d6) δ 10.58 (s, 1H), 8.77 (s, 1H), 8.44 - 8.37 (m, 1H), 7.65 - 7.56 (m, 2H), 7.48 (d, J = 8.2 Hz, 1H), 6.83 (dt, J = 8.5, 1.7 Hz, 1H), 4.62 - 4.52 (m, 2H), 4.28 (s, 2H), 3.19 - 3.13 (m, 2H).

LCMS (m/z) : 332 (M+H).

### Example 19. Preparation of N-(5-oxo-5,6,7,8-tetrahydro-1,6-naphthyridine-3-yl)-2,3-dihydrobenzofuran-7-sulfonamide

A target compound (9 mg, yield: 14%) was obtained by reacting 2,3-dihydrobenzofuran-7-sulfonyl chloride with the compound of Intermediate 2 according to the manufacturing method of Example 1.

¹H-NMR (400 MHz, DMSO-d6) δ 10.43 (s, 1H), 8.35 - 8.21 (m, 1H), 8.10 (s, 1H), 7.81 (dt, J = 4.6, 2.6 Hz, 1H), 7.59 (d, J = 7.4 Hz, 1H), 7.47 (t, J = 6.1 Hz, 1H), 6.90 - 6.75 (m, 1H), 4.65 - 4.49 (m, 2H), 3.34 (d, J = 8.2 Hz, 2H), 3.16 (d, J = 20.1 Hz, 2H), 2.95 - 2.82 (m, 2H).

LCMS (m/z) : 346 (M+H).

### Example 20. Preparation of 5-chloro-N-(5-oxo-6,7-dihydro-5H-pyrrolo[3,4-b]pyridine-3-yl)-2,3-dihydrobenzofuran-7-sulfonamide

A target compound (17 mg, yield: 23%) was obtained by reacting 5-chloro-2,3-dihydrobenzofuran-7-sulfonyl chloride with the compound of Intermediate 1 according to the manufacturing method of Example 1.

¹H-NMR (400 MHz, DMSO-d6) δ 10.86 (s, 1H), 8.79 (s, 1H), 8.52 - 8.37 (m, 1H), 7.73 - 7.59 (m, 1H), 7.53 (dd, J = 4.9, 2.4 Hz, 1H), 7.39 (q, J = 2.7 Hz, 1H), 4.70 - 4.52 (m, 2H), 4.29 (q, J = 3.5, 2.8 Hz, 2H), 3.19 (d, J = 8.5 Hz, 2H).

LCMS (m/z) : 366 (M+H).

### Example 21. Preparation of 5-fluoro-N-(5-oxo-6,7-dihydro-5H-pyrrolo[3,4-b]pyridine-3-yl)-2,3-dihydrobenzofuran-7-sulfonamide

A target compound (11 mg, yield: 16%) was obtained by reacting 5-fluoro-2,3-dihydrobenzofuran-7-sulfonyl chloride with the compound of Intermediate 1 according to the manufacturing method of Example 1.

¹H-NMR (400 MHz, DMSO-d6) δ 10.85 (s, 1H), 8.78 (s, 1H), 8.44 (dt, J = 7.0, 3.3 Hz, 1H), 7.65 (dt, J = 6.7, 3.3 Hz, 1H), 7.47 - 7.30 (m, 1H), 7.28 -7.12 (m, 1H), 4.70 - 4.48 (m, 2H), 4.29 (q, J = 3.8 Hz, 2H), 3.23-3.10 (m, 2H).

LCMS (m/z) : 350 (M+H).

### Example 22. Preparation of 5-chloro-N-(5-oxo-5,6,7,8-tetrahydro-1,6-naphthyridine-3-yl)-2,3-dihydrobenzofuran-7-sulfonamide

A target compound (13 mg, yield: 18%) was obtained by reacting 5-chloro-2,3-dihydrobenzofuran-7-sulfonyl chloride with the compound of Intermediate 2 according to the manufacturing method of Example 1.

¹H-NMR (400 MHz, DMSO-d6) δ 10.71 (s, 1H), 8.35 - 8.25 (m, 1H), 8.16 - 8.02 (m, 1H), 7.88 - 7.79 (m, 1H), 7.52 (q, J = 3.7, 2.7 Hz, 1H), 7.37 (q, J = 2.9 Hz, 1H), 4.70 - 4.54 (m, 2H), 3.41 - 3.32 (m, 2H), 3.18 (t, J = 8.5 Hz, 2H), 2.89 (tt, J = 6.3, 2.8 Hz, 2H).

LCMS (m/z) : 380 (M+H).

### Example 23. Preparation of 5-fluoro-N-(5-oxo-5,6,7,8-tetrahydro-1,6-naphthyridine-3-yl)-2,3-dihydrobenzofuran-7-sulfonamide

A target compound (9 mg, yield: 14%) was obtained by reacting 5-fluoro-2,3-dihydrobenzofuran-7-sulfonyl chloride with the compound of Intermediate 2 according to the manufacturing method of Example 1.

¹H-NMR (400 MHz, DMSO-d6) δ 10.70 (s, 1H), 8.36 - 8.26 (m, 1H), 8.17 - 8.02 (m, 1H), 7.89 - 7.78 (m, 1H), 7.38 (dt, J = 7.9, 3.0 Hz, 1H), 7.22 - 7.10 (m, 1H), 4.70 - 4.54 (m, 2H), 3.41 - 3.31 (m, 2H), 3.18 (t, J = 8.6 Hz, 2H), 2.89 (td, J = 6.7, 2.8 Hz, 2H).

LCMS (m/z) : 364 (M+H).

### Example 24. Preparation of N-(5-oxo-5,6,7,8-tetrahydro-1,6-naphthyridine-3-yl)-2,3-dihydrobenzo[b][1,4]dioxine-6-sulfonamide

A target compound (3 mg, yield: 3%) was obtained by reacting 2,3-dihydrobenzo[b][1,4]dioxine-6-sulfonyl chloride with the compound of Intermediate 2 according to the manufacturing method of Example 1.

¹H-NMR (400 MHz, Methanol-d4) δ 8.30 (d, J = 2.7 Hz, 1H), 8.01 (d, J = 2.6 Hz, 1H), 7.23 (tt, J = 4.4, 2.3 Hz, 2H), 6.95 - 6.83 (m, 1H), 4.29 - 4.18 (m, 4H), 3.52 (t, J = 6.8 Hz, 2H), 3.03 (t, J = 6.8 Hz, 2H).

LCMS (m/z) : 362 (M+H).

### Example 25. Preparation of 3-oxo-N-(5-oxo-5,6,7,8-tetrahydro-1,6-naphthyridine-3-yl)-3,4-dihydro-2H-benzo[b][1,4]oxazine-6-sulfonamide

A target compound (15 mg, yield: 13%) was obtained by reacting 3-oxo-3,4-dihydro-2H-benzo[b][1,4]oxazine-6-sulfonyl chloride with the compound of Intermediate 2 according to the manufacturing method of Example 1.

¹H-NMR (400 MHz, DMSO-d6) δ 8.24 (d, J = 2.5 Hz, 1H), 7.69 (d, J = 2.6 Hz, 1H), 7.32 - 7.27 (m, 3H), 7.18 - 7.13 (m, 2H), 3.49 - 3.44 (m, 2H), 3.12 - 3.02 (m, 4H).

LCMS (m/z) : 375 (M+H).

### Example 26. Preparation of 2-methoxy-N-(5-oxo-5,6,7,8-tetrahydro-1,6-naphthyridine-3-yl)benzenesulfonamide

A target compound (10 mg, yield: 41%) was obtained by reacting 2-methoxybenzenesulfonyl chloride with the compound of Intermediate 2 according to the manufacturing method of Example 1.

¹H-NMR (400 MHz, DMSO-d6) δ 10.32 (s, 1H), 8.29 (t, J = 4.3 Hz, 1H), 8.05 (s, 1H), 7.88 - 7.79 (m, 1H), 7.76 - 7.67 (m, 1H), 7.60 - 7.46 (m, 1H), 7.18 - 7.07 (m, 1H), 7.05 - 6.91 (m, 1H), 3.81 (s, 3H), 3.32 (t, J = 3.5 Hz, 2H), 2.85 (q, J = 10.2, 8.4 Hz, 3H).

LCMS (m/z) : 364 (M+H).

### Example 27. Preparation of methyl 2-(4'-methoxy-3'-(N-(5-oxo-6,7-dihydro-5H-pyrrolo[3,4-b]pyridine-3-yl)sulfamoyl)-[1,1'-biphenyl]-4-yl)acetate

A mixture of the compound of Example 2 (50 mg, 0.126 mmol), methyl 2-(4-(4,4,5,5-tetramethyl-1,3,2-dioxaborolane-2-yl)phenyl)acetate (39 mg, 0.138 mmol), K₂CO₃ (35 mg, 0.251 mmol), and Pd(PPh₃)₄(15 mg, 0.013 mmol), was added to dioxane:water (4:1) (30 mL) and heated in a sealed tube at 100°C for 2 hours. The solution was diluted with brine (200 mL) and extracted with ethyl acetate. The organic layer was dried over anhydrous sodium sulfate and concentrated in vacuo. The product was purified by column chromatography to give methyl 2-(4'-methoxy-3'-(N-(5-oxo-6,7-dihydro-5H-pyrrolo[3,4-b]pyridine-3-yl)sulfamoyl)-[1,1'-biphenyl]-4-yl)acetate as a white solid (35 mg, 60%).

¹H-NMR (400 MHz, Chloroform-d) δ 8.66 (t, J = 2.2 Hz, 1H), 8.23 (s, 1H), 8.04 (d, J = 2.4 Hz, 1H), 7.96 - 7.90 (m, 1H), 7.71 (dd, J = 8.6, 2.4 Hz, 1H), 7.48 - 7.41 (m, 2H), 7.34 - 7.29 (m, 2H), 7.12 - 7.05 (m, 1H), 6.64 (s, 1H), 4.43 (s, 2H), 4.04 (s, 3H), 3.69 (s, 3H), 3.64 (s, 2H).

LCMS (m/z) : 468 (M+H).

### Example 28. Preparation of 2-(4'-methoxy-3'-(N-(5-oxo-6,7-dihydro-5H-pyrrolo[3,4-b]pyridine-3-yl)sulfamoyl)-[1,1'-biphenyl]-4-yl)acetic acid

NaOH (11 mg, 0.267 mmol) was added to a solution of the compound of Example 27 (25 mg, 0.053 mmol) in THF/water (50 mL, 3:1). The reaction mixture was stirred at ambient temperature for 24 hours. THF was removed under vacuum and the resulting solution was acidified with 1 N hydrochloric acid. More water (50 mL) was added and the aqueous solution was extracted with ethyl acetate (3 x 50 mL). The combined organic layer was washed with brine, dried over anhydrous sodium sulfate, and concentrated. The crude product was purified by column chromatography to give 2-(4'-methoxy-3'-(N-(5-oxo-6,7-dihydro-5H-pyrrolo[3,4-b]pyridine-3-yl)sulfamoyl)-[1,1'-biphenyl]-4-yl)acetic acid (15 mg, 62%).

¹H-NMR (400 MHz, DMSO-d6)δ12.21(s,1H),10.72-10.45(m,1H),8.84-8.71(m,1H),8.53-8.38(m,1H),7.98-7.60(m,3H),7.50(dd,J = 8.2, 3.8 Hz, 1H), 7.34 - 7.11 (m, 2H), 6.83 (q, J = 3.8 Hz, 1H), 6.60 (s, 1H), 4.26 (s, 2H), 3.83 (s, 3H), 3.56 (s, 2H).

LCMS (m/z) : 454 (M+H).

### Example 29. Preparation of 5-(2,3-dihydrobenzo[b][1,4]dioxine-6-yl)-2-methoxy-N-(5-oxo-5,6,7,8-tetrahydro-1,6-naphthyridine-3-yl)benzenesulfonamide

A target compound (12 mg, yield: 21%) was obtained by reacting (2,3-dihydrobenzo[b][1,4]dioxine-6-yl)boronic acid with the compound of Example 9 according to the manufacturing method of Example 27.

¹H-NMR (400 MHz, DMSO-d6) δ 10.44 (s, 1H), 8.32 (dd, J = 7.7, 4.3 Hz, 1H), 8.07 (s, 1H), 7.92 - 7.80 (m, 2H), 7.77 (s, 1H), 7.24 - 7.12 (m, 1H), 7.06 - 6.95 (m, 2H), 6.93 - 6.80 (m, 1H), 4.22 (s, 3H), 3.88 - 3.80 (m, 2H), 3.38-3.20 (m, 2H), 2.91 - 2.81 (m, 2H), 1.75 - 1.62 (m, 2H).

LCMS (m/z) : 468 (M+H).

### Example 30. Preparation of 4,4'-dimethoxy-N-(5-oxo-5,6,7,8-tetrahydro-1,6-naphthyridine-3-yl)-[1,1'-biphenyl]-3-sulfonamide

A target compound (9 mg, yield: 17%) was obtained by reacting (4-methoxyphenyl)boronic acid with the compound of Example 9 according to the manufacturing method of Example 27.

¹H-NMR (400 MHz, DMSO-d6) δ 10.43 (s, 1H), 8.39 - 8.27 (m, 1H), 8.06 (s, 1H), 7.94 - 7.83 (m, 2H), 7.78 (d, J = 8.8 Hz, 1H), 7.53 - 7.46 (m, 2H), 7.04 - 6.90 (m, 3H), 3.85 (s, 3H), 3.75 (s, 3H), 3.37 - 3.31 (m, 2H), 2.91 - 2.80 (m, 2H).

LCMS (m/z) : 440 (M+H).

### Example 31. Preparation of 2-methoxy-N-(5-oxo-5,6,7,8-tetrahydro-1,6-naphthyridine-3-yl)-5-(pyridine-4-yl)benzenesulfonamide

A target compound (16 mg, yield: 32%) was obtained by reacting 4-pyridineboronic acid with the compound of Example 9 according to the manufacturing method of Example 27.

¹H-NMR (400 MHz, DMSO-d6) δ 10.51 (s, 1H), 8.58 (d, J = 5.0 Hz, 2H), 8.39 - 8.28 (m, 1H), 8.13 - 8.04 (m, 2H), 8.05 - 7.98 (m, 1H), 7.89 (d, J = 2.7 Hz, 1H), 7.67 - 7.58 (m, 2H), 7.31 (d, J = 8.7 Hz, 1H), 3.89 (s, 3H), 3.35 - 3.31 (m, 2H), 2.85 (t, J = 6.7 Hz, 2H).

LCMS (m/z) : 411 (M+H).

### Example 32. Preparation of 1-(4'-methoxy-3'-(N-(5-oxo-5,6,7,8-tetrahydro-1,6-naphthyridine-3-yl)sulfamoyl)-[1,1'-biphenyl]-4-yl)cyclopropane-1-carboxylic acid

Methyl 1-(4'-methoxy-3'-(N-(5-oxo-5,6,7,8-tetrahydro-1,6-naphthyridine-3-yl)sulfamoyl)-[1,1'-biphenyl]-4-yl)cyclopropane-1-carboxylate (40 mg, yield: 66%) was obtained by reacting methyl1-(4-(4,4,5,5-tetramethyl-1,3,2-dioxaborolane-2-yl)phenyl)cyclopropane-1-carboxylate with the compound of Example 9 according to the manufacturing method of Example 27.

A target compound (21 mg, yield: 53%) was obtained by reacting methyl 1-(4'-methoxy-3'-(N-(5-oxo-5,6,7,8-tetrahydro-1,6-naphthyridine-3-yl)sulfamoyl)-[1,1'-biphenyl]-4-yl)cyclopropane-1-carboxylate according to the manufacturing method of Example 28.

¹H-NMR (400 MHz, DMSO-d6) δ 10.44 (s, 1H), 8.32 (d, J = 3.0 Hz, 1H), 8.04 (s, 1H), 7.95 - 7.78 (m, 3H), 7.48 (t, J = 5.2 Hz, 2H), 7.38 - 7.31 (m, 2H), 7.25 (d, J = 8.7 Hz, 1H), 3.86 (d, J = 2.3 Hz, 3H), 3.13 (dd, J = 5.3, 2.1 Hz, 3H), 2.85 (d, J = 7.4 Hz, 2H), 1.42 (s, 2H), 1.12 (d, J = 4.2 Hz, 2H).

LCMS (m/z) : 494 (M+H).

### Example 33. Preparation of 2-(4'-methoxy-3'-(N-(5-oxo-5,6,7,8-tetrahydro-1,6-naphthyridine-3-yl)sulfamoyl)-[1,1'-biphenyl]-4-yl)acetic acid

Methyl 2-(4'-methoxy-3'-(N-(5-oxo-5,6,7,8-tetrahydro-1,6-naphthyridine-3-yl)sulfamoyl)-[1,1'-biphenyl]-4-yl)acetate (10 mg, yield: 63%) was obtained by reacting methyl 2-(4-(4,4,5,5-tetramethyl-1,3,2-dioxaborolane-2-yl)phenyl)acetate with the compound of Example 9 according to the manufacturing method of Example 27.

A target compound (5 mg, yield: 8%) was obtained by reacting methyl 2-(4'-methoxy-3'-(N-(5-oxo-5,6,7,8-tetrahydro-1,6-naphthyridine-3-yl)sulfamoyl)-[1,1'-biphenyl]-4-yl)acetate according to the manufacturing method of Example 28.

¹H-NMR (400 MHz, DMSO-d6) δ 8.25 (s, 1H), 8.06 - 7.72 (m, 4H), 7.50 (d, J = 7.7 Hz, 2H), 7.29 (d, J = 7.9 Hz, 2H), 3.82 (s, 3H), 3.54 (s, 3H), 2.83 (s, 3H).

LCMS (m/z) : 468 (M+H).

### Example 34. Preparation of 4'-methoxy-3'-(N-(5-oxo-5,6,7,8-tetrahydro-1,6-naphthyridine-3-yl)sulfamoyl)-[1,1'-biphenyl]-4-carboxylic acid

Methyl 4'-methoxy-3'-(N-(5-oxo-5,6,7,8-tetrahydro-1,6-naphthyridine-3-yl)sulfamoyl)-[1,1'-biphenyl]-4-carboxylate (48 mg, yield: 84%) was obtained by reacting methyl 4-(4,4,5,5-tetramethyl-1,3,2-dioxaborolane-2-yl)benzoate with the compound of Example 9 according to the manufacturing method of Example 27.

A target compound (10 mg, yield: 21%) was obtained by reacting methyl 4'-methoxy-3'-(N-(5-oxo-5,6,7,8-tetrahydro-1,6-naphthyridine-3-yl)sulfamoyl)-[1,1'-biphenyl]-4-carboxylate according to the manufacturing method of Example 28.

¹H-NMR (400 MHz, DMSO-d6) δ 8.31 (s, 1H), 8.10 - 7.85 (m, 5H), 7.67 (d, J = 8.1 Hz, 2H), 7.27 (d, J = 8.9 Hz, 1H), 4.08 (s, 3H), 3.86 (s, 2H), 2.84 (t, J = 6.7 Hz, 2H).

LCMS (m/z) : 454 (M+H).

### Example 35. Preparation of 2-(4'-fluoro-2'-(N-(5-oxo-5,6,7,8-tetrahydro-1,6-naphthyridine-3-yl)sulfamoyl)-[1,1'-biphenyl]-4-yl)acetic acid

Methyl 2-(4'-fluoro-2'-(N-(5-oxo-5,6,7,8-tetrahydro-1,6-naphthyridine-3-yl)sulfamoyl)-[1,1'-biphenyl]-4-yl)acetate (9 mg, yield: 15%) was obtained by reacting 2-methyl 2-(4-(4,4,5,5-tetramethyl-1,3,2-dioxaborolane-2-yl)phenyl)acetate with the compound of Example 56 according to the manufacturing method of Example 27.

A target compound (2 mg, yield: 4%) was obtained by reacting methyl 2-(4'-fluoro-2'-(N-(5-oxo-5,6,7,8-tetrahydro-1,6-naphthyridine-3-yl)sulfamoyl)-[1,1'-biphenyl]-4-yl)acetate according to the manufacturing method of Example 28.

¹H-NMR (400 MHz, Methanol-d4) δ 7.99 - 7.91 (m, 1H), 7.58 - 7.50 (m, 3H), 7.33 (d, J = 7.9 Hz, 3H), 7.27 (d, J = 9.0 Hz, 1H), 7.14 (d, J = 7.9 Hz, 1H), 3.60 (s, 3H), 3.50 (t, J = 7.1 Hz, 1H), 2.99 (t, J = 6.8 Hz, 2H).

LCMS (m/z) : 456 (M+H).

### Example 36. Preparation of 1-(4'-fluoro-2'-(N-(5-oxo-5,6,7,8-tetrahydro-1,6-naphthyridine-3-yl)sulfamoyl)-[1,1'-biphenyl]-4-yl)cyclopropane-1-carboxylic acid

Methyl 1-(4'-fluoro-2'-(N-(5-oxo-5,6,7,8-tetrahydro-1,6-naphthyridine-3-yl)sulfamoyl)-[1,1'-biphenyl]-4-yl)cyclopropane-1-carboxylate (11 mg, yield: 18%) was obtained by reacting methyl 1-(4-(4,4,5,5-tetramethyl-1,3,2-dioxaborolane-2-yl)phenyl)cyclopropane-1-carboxylate and 2-bromo-5-fluoro-N-(5-oxo-5,6,7,8-tetrahydro-1,6-naphthyridine-3-yl)benzenesulfonamide according to the manufacturing method of Example 27.

A target compound (2 mg, yield: 3%) was obtained by reacting methyl 1-(4'-fluoro-2'-(N-(5-oxo-5,6,7,8-tetrahydro-1,6-naphthyridine-3-yl)sulfamoyl)-[1,1'-biphenyl]-4-yl)cyclopropane-1-carboxylate according to the manufacturing method of Example 28.

¹H-NMR (400 MHz, Methanol-d4) δ 8.01 - 7.77 (m, 2H), 7.42 - 7.17 (m, 6H), 7.12 (d, J = 8.0 Hz, 1H), 3.49 (t, J = 6.5 Hz, 2H), 2.99 (t, J = 6.9 Hz, 2H), 1.52 (q, J = 3.9 Hz, 2H), 1.18 - 1.11 (m, 2H).

LCMS (m/z) : 482 (M+H).

### Example 37. Preparation of 1-(3',5'-difluoro-2'-(N-(5-oxo-5,6,7,8-tetrahydro-1,6-naphthyridine-3-yl)sulfamoyl)-[1,1'-biphenyl]-4-yl)cyclopropane-1-carboxylic acid

2-Bromo-4,6-difluoro-N-(5-oxo-5,6,7,8-tetrahydro-1,6-naphthyridine-3-yl)benzenesulfonamide (510 mg, yield: 66%) was obtained by reacting 2-bromo-4,6-difluorobenzenesulfonylchloride with the compound of Intermediate 2 according to the manufacturing method of Example 1.

Methyl 1-(3',5'-difluoro-2'-(N-(5-oxo-5,6,7,8-tetrahydro-1,6-naphthyridine-3-yl)sulfamoyl)-[1,1'-biphenyl]-4-yl)cyclopropane-1-carboxylate (16 mg, yield: 32%) was obtained by reacting methyl 1-(4-(4,4,5,5-tetramethyl-1,3,2-dioxaborolane-2-yl)phenyl)cyclopropane-1-carboxylate and 2-bromo-4,6-difluoro-N-(5-oxo-5,6,7,8-tetrahydro-1,6-naphthyridine-3-yl)benzenesulfonamide according to the manufacturing method of Example 27.

A target compound (2 mg, yield: 3%) was obtained by reacting methyl 1-(3',5'-difluoro-2'-(N-(5-oxo-5,6,7,8-tetrahydro-1,6-naphthyridine-3-yl)sulfamoyl)-[1,1'-biphenyl]-4-yl)cyclopropane-1-carboxylate according to the manufacturing method of Example 28.

¹H-NMR (400 MHz, DMSO-d6) Methanol-d4) δ 7.86 (s, 1H), 7.52 (s, 1H), 7.42 - 7.02 (m, 6H), 3.32 (d, J = 2.4 Hz, 4H), 1.52 (q, J = 3.8 Hz, 2H), 1.12 (p, J = 3.8 Hz, 2H).

LCMS (m/z) : 500 (M+H).

### Example 38. Preparation of 1-(4-(7-(N-(5-oxo-5,6,7,8-tetrahydro-1,6-naphthyridine-3-yl)sulfamoyl)-2,3-dihydrobenzofuran-5-yl)phenyl)cyclopropane-1-carboxylic acid

Methyl 1-(4-(7-(N-(5-oxo-5,6,7,8-tetrahydro-1,6-naphthyridine-3-yl)sulfamoyl)-2,3-dihydrobenzofuran-5-yl)phenyl)cyclopropane-1-carboxylate (31 mg, yield: 47%) was obtained by reacting methyl 1-(4-(4,4,5,5-tetramethyl-1,3,2-dioxaborolane-2-yl)phenyl)cyclopropane-1-carboxylate with the compound of Example 5 according to the manufacturing method of Example 27.

A target compound (2 mg, yield: 3%) was obtained by reacting methyl 1-(4-(7-(N-(5-oxo-5,6,7,8-tetrahydro-1,6-naphthyridine-3-yl)sulfamoyl)-2,3-dihydrobenzofuran-5-yl)phenyl)cyclopropane-1-carboxylate according to the manufacturing method of Example 28.

¹H-NMR (400 MHz, Methanol-d4) δ 8.39 (d, J = 2.6 Hz, 1H), 8.13 - 8.02 (m, 1H), 7.67 (dd, J = 15.8, 2.2 Hz, 1H), 7.46 - 7.34 (m, 2H), 7.34 - 7.16 (m, 3H), 4.72 (t, J = 8.4 Hz, 2H), 3.59 - 3.40 (m, 2H), 3.32 (d, J = 2.2 Hz, 2H), 3.05 - 2.88 (m, 2H), 1.52 (ddd, J = 6.8, 4.3, 2.3 Hz, 2H), 1.16 - 1.05 (m, 2H).

LCMS (m/z) : 506 (M+H).

### Example 39. Preparation of 4'-(aminomethyl)-4-fluoro-N-(5-oxo-5,6,7,8-tetrahydro-1,6-naphthyridine-3-yl)-[1,1'-biphenyl]-2-sulfonamide hydrochloride

Tert-butyl((4'-fluoro-2'-(N-(5-oxo-5,6,7,8-tetrahydro-1,6-naphthyridine-3-yl)sulfamoyl)-[1,1'-biphenyl]-4-yl)methyl)carbamate (108 mg, yield: 82%) was obtained by reacting tert-butyl(4-(4,4,5,5-tetramethyl-1,3,2-dioxaborolane-2-yl)benzyl)carbamate and 2-bromo-5-fluoro-N-(5-oxo-5,6,7,8-tetrahydro-1,6-naphthyridine-3-yl)benzenesulfonamide according to the manufacturing method of Example 27.

Tert-butyl((4'-fluoro-2'-(N-(5-oxo-5,6,7,8-tetrahydro-1,6-naphthyridine-3-yl)sulfamoyl)-[1,1'-biphenyl]-4-yl)methyl)carbamate was dissolved in 1 ml of methanol, to which 0.5 ml of HCl (in dioxane) was added, and the mixture was stirred at room temperature for 1 hour. Upon completion of the reaction, the reactant was concentrated and washed with diethyl ether to give a target compound (63 mg, yield: 71%).

¹H-NMR (400 MHz, Methanol-d4) δ 8.17 (d, J = 2.7 Hz, 1H), 7.98 - 7.79 (m, 2H), 7.49 - 7.38 (m, 3H), 7.34 - 7.24 (m, 3H), 4.16 (s, 2H), 3.61 - 3.53 (m, 3H), 3.15 (t, J = 6.7 Hz, 2H).

LCMS (m/z) : 467 (M+H).

### Example 40. Preparation of 4'-(aminomethyl)-3,5-difluoro-N-(5-oxo-5,6,7,8-tetrahydro-1,6-naphthyridine-3-yl)-[1,1'-biphenyl]-2-sulfonamide hydrochloride

Tert-butyl((3',5'-difluoro-2'-(N-(5-oxo-5,6,7,8-tetrahydro-1,6-naphthyridine-3-yl)sulfamoyl)-[1,1'-biphenyl]-4-yl)methyl)carbamate (46 mg, yield: 35%) was obtained by reacting tert-butyl(4-(4,4,5,5-tetramethyl-1,3,2-dioxaborolane-2-yl)benzyl)carbamate and 2-bromo-4,6-difluoro-N-(5-oxo-5,6,7,8-tetrahydro-1,6-naphthyridine-3-yl)benzenesulfonamide according to the manufacturing method of Example 27.

Tert-butyl((3',5'-difluoro-2'-(N-(5-oxo-5,6,7,8-tetrahydro-1,6-naphthyridine-3-yl)sulfamoyl)-[1,1'-biphenyl]-4-yl)methyl)carbamate was dissolved in 1 ml of methanol, to which 0.5 ml of HCl (in dioxane) was added, and the mixture was stirred at room temperature for 1 hour. Upon completion of the reaction, the reactant was concentrated and washed with diethyl ether to give a target compound (25 mg, yield: 22%).

¹H-NMR (400 MHz, Methanol-d4) δ 8.32 (d, J = 12.5 Hz, 2H), 7.61 - 7.18 (m, 5H), 6.96 (d, J = 7.6 Hz, 1H), 4.17 (s, 2H), 3.62 (d, J = 4.6 Hz, 3H).

LCMS (m/z) : 445 (M+H).

### Example 41. Preparation of 5-(4-(aminomethyl)phenyl)-N-(5-oxo-5,6,7,8-tetrahydro-1,6-naphthyridine-3-yl)-2,3-dihydrobenzofuran-7-sulfonamide

Tert-butyl(4-(7-(N-(5-oxo-5,6,7,8-tetrahydro-1,6-naphthyridine-3-yl)sulfamoyl)-2,3-dihydrobenzofuran-5-yl)benzyl)carbamate (28 mg, yield: 43%) was obtained by reacting tert-butyl(4-(4,4,5,5-tetramethyl-1,3,2-dioxaborolane-2-yl)benzyl)carbamate with the compound of Example 5 according to the manufacturing method of Example 27.

Tert-butyl(4-(7-(N-(5-oxo-5,6,7,8-tetrahydro-1,6-naphthyridine-3-yl)sulfamoyl)-2,3-dihydrobenzofuran-5-yl)benzyl)carbamate was dissolved in 1 ml of methanol, to which 0.5 ml of HCl (in dioxane) was added, and the mixture was stirred at room temperature for 1 hour. Upon completion of the reaction, the reactant was concentrated and the pH was adjusted to 9-10 with 1 N NaOH aqueous solution. More water (50 mL) was added and the aqueous solution was extracted with ethyl acetate (3 x 50 mL). The combined organic layer was washed with brine, dried over anhydrous sodium sulfate, and concentrated. The mixture was purified by column chromatography to give a target compound (10 mg, 19%).

¹H-NMR (400 MHz, Methanol-d4) δ 8.33 (s, 1H), 8.06 (s, 1H), 7.75 (s, 1H), 7.67 - 7.29 (m, 4H), 4.01 (s, 1H), 3.71 - 3.39 (m, 6H), 2.97 (s, 3H).

LCMS (m/z) : 451 (M+H).

### Example 42. Preparation of ethyl 2-(4-(4-methoxy-3-(N-(5-oxo-5,6,7,8-tetrahydro-1,6-naphthyridine-3-yl)sulfamoyl)phenyl)-1H-pyrazole-1-yl)propanoate

A target compound (21 mg, yield: 17%) was obtained by reacting ethyl 2-(4-(4,4,5,5-tetramethyl-1,3-dioxolane-2-yl)-1H-pyrazole-1-yl)propanoate with the compound of Example 9 according to the manufacturing method of Example 27.

¹H-NMR (400 MHz, DMSO-d6) δ 10.51 (s, 1H), 8.31 - 8.27 (m, 1H), 8.10 (s, 1H), 7.85 - 7.75 (m, 1H), 7.79 - 7.70 (m, 3H), 7.54 - 7.49 (m, 1H), 7.15 - 7.11 (m, 1H), 4.50-4.40 (m, 1H), 4.20-4.10 (m, 2H), 3.80 (s, 3H), 3.40 - 3.29 (m, 2H), 3.25 - 3.18 (m, 2H), 1.45 (s, 3H), 1.25-1.10 (m, 3H).

LCMS (m/z) : 500(M+H).

### Example 43. Preparation of 2-(4-(4-methoxy-3-(N-(5-oxo-5,6,7,8-tetrahydro-1,6-naphthyridine-3-yl)sulfamoyl)phenyl)-1H-pyrazole-1-yl)propanoic acid

The compound of Example 42 was reacted according to the manufacturing method of Example 28 to give a target compound (11 mg, yield: 65%).

¹H-NMR (400 MHz, DMSO-d6) δ 10.36 (s, 1H), 8.30 (d, J = 23.1 Hz, 2H), 8.07 (s, 1H), 7.89 - 7.71 (m, 3H), 7.50 - 7.45 (m, 1H), 7.16 (d, J = 8.7 Hz, 1H), 5.12 - 4.99 (m, 1H), 3.84 (s, 3H), 3.39 - 3.23 (m, 2H), 2.90 - 2.80 (m, 2H), 1.63 (s, 3H).

LCMS (m/z) : 472 (M+H).

### Example 44. Preparation of 5-(2-aminopyrimidine-5-yl)-2-methoxy-N-(5-oxo-5,6,7,8-tetrahydro-1,6-naphthyridine-3-yl)benzenesulfonamide

A target compound (14 mg, yield: 45%) was obtained by reacting 5-(4,4,5,5-tetramethyl-1,3,2-dioxaborolane-2-yl)pyrimidine-2-amine with the compound of Example 9 according to the manufacturing method of Example 27.

¹H-NMR (400 MHz, DMSO-d6) δ 10.46 (s, 1H), 8.53 - 8.41 (m, 2H), 8.32 (t, J = 2.2 Hz, 1H), 8.08 (s, 1H), 7.88 (t, J = 2.9 Hz, 2H), 7.83 - 7.72 (m, 1H), 7.22 (dd, J = 8.8, 1.8 Hz, 1H), 6.83 (s, 2H), 3.84 (s, 3H), 3.32 (dt, J = 6.9, 3.0 Hz, 3H), 2.86 (t, J = 6.6 Hz, 2H).

LCMS (m/z) : 427 (M+H).

### Example 45. Preparation of 2-methoxy-N-(5-oxo-5,6,7,8-tetrahydro-1,6-naphthyridine-3-yl)-5-(pyrazolo[1,5-a]pyridine-3-yl)benzenesulfonamide

A target compound (6 mg, yield: 18%) was obtained by reacting 3-(4,4,5,5-tetramethyl-1,3,2-dioxaborolane-2-yl)pyrazolo[1,5-a]pyridine with the compound of Example 9 according to the manufacturing method of Example 27.

¹H-NMR (400 MHz, Chloroform-d) δ 8.54 (d, J = 2.7 Hz, 1H), 8.46 (d, J = 7.2 Hz, 1H), 8.05 (s, 1H), 8.04 - 8.00 (m, 2H), 7.70 (dd, J = 8.7, 2.5 Hz, 2H), 7.20 (d, J = 7.8 Hz, 1H), 7.11 (d, J = 8.6 Hz, 2H), 6.80 (t, J = 6.9 Hz, 1H), 5.94 (s, 1H), 4.09 (s, 3H), 3.61 - 3.53 (m, 2H), 3.08 (t, J = 6.7 Hz, 2H).

LCMS (m/z) : 450 (M+H).

### Example 46. Preparation of 3',5'-difluoro-4'-hydroxy-4-methoxy-N-(5-oxo-5,6,7,8-tetrahydro-1,6-naphthyridine-3-yl)-[1,1'-biphenyl]-3-sulfonamide

A target compound (16 mg, yield: 47%) was obtained by reacting 2,6-difluoro-4-(4,4,5,5-tetramethyl-1,3,2-dioxaborolane-2-yl)phenol with the compound of Example 9 according to the manufacturing method of Example 27.

¹H-NMR (400 MHz, DMSO-d6)δ10.47(s,1H),10.32(s,1H),8.36-8.28(m,1H),8.09(s,1H),7.92-7.84(m,2H),7.87-7.78(m,1H),7.34-7.26(m,2H),7.21(dt,J = 7.5, 3.4 Hz,1H), 3.85 (s, 3H), 3.34 - 3.26 (m, 2H), 2.89 - 2.81 (m, 2H).

LCMS (m/z) : 462 (M+H).

### Example 47. Preparation of tert-butyl 2-(4-(4-methoxy-3-(N-(5-oxo-5,6,7,8-tetrahydro-1,6-naphthyridine-3-yl)sulfamoyl)phenyl)-1H-pyrazole-1-yl)acetate

A target compound (19 mg, yield: 15%) was obtained by reacting tert-butyl 2-(4-(4,4,5,5-tetramethyl-1,3-dioxolane-2-yl)-1H-pyrazole-1-yl)acetate with the compound of Example 9 according to the manufacturing method of Example 27.

¹H-NMR (400 MHz, DMSO-d6) δ 10.37 (s, 1H), 8.32 (t, J = 2.7 Hz, 1H), 8.16 - 8.10 (m, 1H), 8.06 (s, 1H), 7.90 - 7.79 (m, 3H), 7.79 - 7.71 (m, 1H), 7.20 - 7.13 (m, 1H), 4.89 (s, 2H), 3.83 (s, 3H), 3.34 - 3.26 (m, 2H), 2.86 (t, J = 6.3 Hz, 2H), 1.38 (s, 9H).

LCMS (m/z) : 514 (M+H).

### Example 48. Preparation of 2-(4-(4-methoxy-3-(N-(5-oxo-5,6,7,8-tetrahydro-1,6-naphthyridine-3-yl)sulfamoyl)phenyl)-1H-pyrazole-1-yl)acetic acid

The compound of Example 47 was dissolved in 1 ml of methanol, to which 1 ml of hydrochloric acid was added. Check the mixture by TLC and adjust the PH to 5 with an aqueous solution of 1 N-NaOH. The reactant was purified by column chromatography to give a target compound (9 mg, yield: 63%).

¹H-NMR (400 MHz, DMSO-d6) δ 10.37 (s, 1H), 8.33 (t, J = 3.0 Hz, 1H), 8.16 - 8.10 (m, 1H), 8.07 (s, 1H), 7.90 - 7.85 (m, 1H), 7.82 (q, J = 3.3, 2.7 Hz, 2H), 7.78 - 7.71 (m, 1H), 7.20 - 7.13 (m, 1H), 4.90 (s, 2H), 3.82 (s, 3H), 3.35 - 3.28 (m, 2H), 2.86 (t, J = 6.5 Hz, 2H).

LCMS (m/z) : 458 (M+H).

### Example 49. Preparation of ethyl 3-(4'-methoxy-3'-(N-(5-oxo-5,6,7,8-tetrahydro-1,6-naphthyridine-3-yl)sulfamoyl)-[1,1'-biphenyl]-4-yl)propanoate

A target compound (20 mg, yield: 54%) was obtained by reacting ethyl 3-(4-(4,4,5,5-tetramethyl-1,3,2-dioxaborolane-2-yl)phenyl)propanoate with the compound of Example 9 according to the manufacturing method of Example 27.

¹H-NMR (400 MHz, DMSO-d6) δ 10.47 (s, 1H), 8.35 - 8.31 (m, 1H), 8.09 (s, 1H), 7.94 - 7.87 (m, 2H), 7.87 - 7.80 (m, 1H), 7.51 - 7.43 (m, 2H), 7.31 - 7.19 (m, 3H), 4.00 (qt, J = 7.1, 1.6 Hz, 2H), 3.85 (s, 3H), 2.91 - 2.77 (m, 4H), 2.59 (t, J = 7.6 Hz, 2H), 1.10 (t, J = 1.6 Hz, 3H).

LCMS (m/z) : 510 (M+H).

### Example 50. Preparation of 3-(4'-methoxy-3'-(N-(5-oxo-5,6,7,8-tetrahydro-1,6-naphthyridine-3-yl)sulfamoyl)-[1,1'-biphenyl]-4-yl)propanoic acid

The compound of Example 49 was reacted according to the manufacturing method of Example 28 to give a target compound (12 mg, yield: 85%).

¹H-NMR (400 MHz, DMSO-d6) δ 12.10 (s, 1H), 10.46 (s, 1H), 8.35 - 8.27 (m, 1H), 8.08 (s, 1H), 7.96 - 7.78 (m, 3H), 7.52 - 7.40 (m, 2H), 7.33 - 7.18 (m, 3H), 3.84 (s, 3H), 2.92 - 2.72 (m, 4H), 2.56 - 2.48 (m, 2H)

LCMS (m/z) : 482 (M+H).

### Example 51. Preparation of 5-(3,6-dihydro-2H-pyran-4-yl)-2-methoxy-N-(5-oxo-5,6,7,8-tetrahydro-1,6-naphthyridine-3-yl)benzenesulfonamide

A target compound (16 mg, yield: 53%) was obtained by reacting 2-(3,6-dihydro-2H-pyran-4-yl)-4,4,5,5-tetramethyl-1,3,2-dioxaborolane with the compound of Example 9 according to the manufacturing method of Example 27.

¹H-NMR (400 MHz, DMSO-d6) δ 10.37 (s, 1H), 8.36 - 8.26 (m, 1H), 8.06 (s, 1H), 7.92 - 7.82 (m, 1H), 7.79 - 7.71 (m, 1H), 7.69 - 7.59 (m, 1H), 7.19 - 7.08 (m, 1H), 6.23 - 6.11 (m, 1H), 4.21 - 4.12 (m, 1H), 3.95 - 3.88 (m, 1H), 3.83 (s, 3H), 3.80 - 3.68 (m, 2H), 3.41 - 3.32 (m, 2H), 2.94 - 2.77 (m, 2H), 2.42 - 2.24 (m, 2H).

LCMS (m/z) : 416 (M+H).

### Example 52. Preparation of tert-butyl 4-(4-methoxy-3-(N-(5-oxo-5,6,7,8-tetrahydro-1,6-naphthyridine-3-yl)sulfamoyl)phenyl)-3,6-dihydropyridine-1(2H)-carboxylate

A target compound (34 mg, yield: 55%) was obtained by reacting tert-butyl 4-(4,4,5,5-tetramethyl-1,3,2-dioxaborolane-2-yl)-3,6-dihydropyridine-1(2H)-carboxylate with the compound of Example 9 according to the manufacturing method of Example 27.

¹H-NMR (400 MHz, DMSO-d6) δ 10.37 (s, 1H), 8.35 - 8.22 (m, 1H), 8.07 (s, 1H), 7.89 - 7.80 (m, 1H), 7.77 - 7.67 (m, 1H), 7.62 (dd, J = 9.2, 2.9 Hz, 1H), 7.13 (d, J = 8.2 Hz, 1H), 6.06 (s, 1H), 3.93 (s, 2H), 3.81 (s, 3H), 3.47 (d, J = 5.7 Hz, 2H), 3.32 (dd, J = 6.9, 2.8 Hz, 2H), 2.86 (t, J = 6.6 Hz, 2H), 2.39-2.31 (m, 2H), 1.38 (s, 9H).

LCMS (m/z) : 515 (M+H).

### Example 53. Preparation of 2-methoxy-N-(5-oxo-5,6,7,8-tetrahydro-1,6-naphthyridine-3-yl)-5-(1,2,3,6-tetrahydropyridine-4-yl)benzenesulfonamide hydrochloride

The compound of Example 52 was dissolved in 1 ml of methanol, to which 0.5 ml of HCl (in dioxane) was added, and the mixture was stirred at room temperature for 1 hour. Upon completion of the reaction, the reactant was concentrated and washed with diethyl ether to give a target compound (35 mg, yield: 83%).

¹H-NMR (400 MHz, DMSO-d6) δ 10.52 (s, 1H), 9.19 (s, 2H), 8.32 (t, J = 3.0 Hz, 1H), 8.11 (s, 1H), 7.89 (d, J = 3.9 Hz, 1H), 7.82 - 7.73 (m, 1H), 7.72 - 7.57 (m, 1H), 7.17 (dd, J = 9.5, 3.4 Hz, 1H), 6.17 - 6.01 (m, 1H), 3.83 (s, 3H), 3.72 - 3.58 (m, 2H), 3.39 - 3.13 (m, 4H), 2.94 - 2.79 (m, 2H), 2.64 - 2.52 (m, 2H).

LCMS (m/z) : 415 (M+H).

**Example 54. Preparation of 2-methoxy-N-(5-oxo-5,6,7,8-tetrahydro-1,6-naphthyridine-3-yl)-5-(1-(tetrahydro-2H-pyran-2-yl)-1H-pyrazole-4-yl)benzenesulfonamide**

A target compound (32 mg, yield: 55%) was obtained by reacting 1-(tetrahydro-2H-pyran-2-yl)-4-(4,4,5,5-tetramethyl-1,3,2-dioxaborolane-2-yl)-1H-pyrazole with the compound of Example 9 according to the manufacturing method of Example 27.

¹H-NMR (400 MHz, DMSO-d6) δ 10.33 (s, 1H), 8.40 - 8.27 (m, 2H), 8.03 (s, 1H), 7.94 - 7.68 (m, 5H), 7.16 (t, J = 7.3 Hz, 1H), 5.34 (d, J = 10.5 Hz, 1H), 3.96 - 3.86 (m, 1H), 3.83 (s, 3H), 3.70 - 3.52 (m, 1H), 2.94 - 2.80 (m, 2H), 2.18 - 1.82 (m, 4H), 1.74 - 1.43 (m, 4H).

LCMS (m/z) : 484 (M+H).

### Example 55. Preparation of 2-methoxy-N-(5-oxo-5,6,7,8-tetrahydro-1,6-naphthyridine-3-yl)-5-(1H-pyrazole-4-yl)benzenesulfonamide

The compound of Example 54 was dissolved in 1 ml of methanol, to which 0.5 ml of HCl (in dioxane) was added, and the mixture was stirred at room temperature for 1 hour. Upon completion of the reaction, the pH of the reactant was adjusted to 9-10 with 1 N-NaOH. More water (50 mL) was added and the aqueous solution was extracted with ethyl acetate (3 x 50 mL). The combined organic layer was washed with brine, dried over anhydrous sodium sulfate, and concentrated. The mixture was purified by column chromatography to give a target compound (15 mg, 31%).

¹H-NMR (400 MHz, DMSO-d6) δ 10.52 (s, 1H), 9.19 (s, 2H), 8.32 (t, J = 3.0 Hz, 1H), 8.11 (s, 1H), 7.89 (d, J = 3.9 Hz, 1H), 7.82 - 7.73 (m, 1H), 7.72 - 7.57 (m, 1H), 7.17 (dd, J = 9.5, 3.4 Hz, 1H), 6.17 - 6.01 (m, 1H), 3.83 (s, 3H), 3.72 - 3.58 (m, 2H), 3.39 - 3.13 (m, 4H), 2.94 - 2.79 (m, 2H), 2.64 - 2.52 (m, 2H).

LCMS (m/z) : 400 (M+H).

### Example 56. Preparation of 2-bromo-5-fluoro-N-(5-oxo-5,6,7,8-tetrahydro-1,6-naphthyridine-3-yl)benzenesulfonamide

A target compound (80 mg, yield: 55%) was obtained by reacting 2-bromo-5-fluorobenzenesulfonylchloride with the compound of Intermediate 2 according to the manufacturing method of Example 1.

¹H-NMR (400 MHz, DMSO-d6) δ 11.11 (s, 1H), 8.46 - 8.31 (m, 1H), 8.10 (t, J = 5.4 Hz, 1H), 7.96 - 7.77 (m, 3H), 7.56 - 7.37 (m, 1H), 3.44 - 3.33 (m, 2H), 2.99 - 2.83 (m, 2H).

LCMS (m/z) : 401 (M+H).

### Example 57. Preparation of 2-methoxy-5-(1-methyl-1H-pyrazole-4-yl)-N-(5-oxo-5,6,7,8-tetrahydro-1,6-naphthyridine-3-yl)benzenesulfonamide

A target compound (12 mg, yield: 40%) was obtained by reacting 1-methyl-4-(4,4,5,5-tetramethyl-1,3-dioxolane-2-yl)-1H-pyrazole with the compound of Example 9 according to the manufacturing method of Example 27.

¹H-NMR (400 MHz, DMSO-d6) δ 10.33 (s, 1H), 8.37 - 8.27 (m, 1H), 8.09 (d, J = 5.5 Hz, 1H), 8.04 (s, 1H), 7.92 - 7.67 (m, 3H), 7.22 - 7.10 (m, 1H), 3.82 (s, 3H), 3.79 (s, 3H), 3.39 - 3.30 (m, 2H), 2.92 - 2.79 (m, 2H).

LCMS (m/z) : 414 (M+H).

### Example 58. Preparation of 2-(4-(4-methoxy-3-(N-(5-oxo-5,6,7,8-tetrahydro-1,6-naphthyridine-3-yl)sulfamoyl)phenyl)-1H-pyrazole-1-yl)-2-methylpropanamide

A target compound (8 mg, yield: 23%) was obtained by reacting 2-methyl-2-(4-(4,4,5,5-tetramethyl-1,3,2-dioxaborolane-2-yl)-1H-pyrazole-1-yl)propanamide with the compound of Example 9 according to the manufacturing method of Example 27.

¹H-NMR (400 MHz, DMSO-d6) δ 10.31 (s, 1H), 8.40 - 8.23 (m, 2H), 8.01 (s, 1H), 7.93 - 7.66 (m, 4H), 7.26 (s, 2H), 7.22 - 7.05 (m, 1H), 3.81 (s, 3H), 3.41 - 3.30 (m, 2H), 2.91 - 2.82 (m, 2H), 1.71 (s, 6H).

LCMS (m/z) : 485 (M+H).

### Example 59. Preparation of ethyl 2-(4-(4-methoxy-3-(N-(5-oxo-5,6,7,8-tetrahydro-1,6-naphthyridine-3-yl)sulfamoyl)phenyl)-1H-pyrazole-1-yl)-2-methylpropanoate

A target compound (28 mg, yield: 45%) was obtained by reacting ethyl 2-methyl-2-(4-(4,4,5,5-tetramethyl-1,3,2-dioxaborolane-2-yl)-1H-pyrazole-1-yl)propanoate with the compound of Example 9 according to the manufacturing method of Example 27.

¹H-NMR (400 MHz, DMSO-d6) δ 10.30 (s, 1H), 8.42 - 8.25 (m, 2H), 8.04 (s, 1H), 7.95 - 7.68 (m, 5H), 7.23 - 7.09 (m, 1H), 4.11 - 3.98 (m, 2H), 3.83 (s, 3H), 3.39-3.29 (m, 2H), 2.93 - 2.83 (m, 2H), 1.74 (s, 6H), 1.15 - 1.00 (m, 3H).

LCMS (m/z) : 514 (M+H).

### Example 60. Preparation of 2-(4-(4-methoxy-3-(N-(5-oxo-5,6,7,8-tetrahydro-1,6-naphthyridine-3-yl)sulfamoyl)phenyl)-1H-pyrazole-1-yl)-2-methylpropanoic acid

The compound of Example 59 was reacted according to the manufacturing method of Example 28 to give a target compound (12 mg, yield: 20%).

¹H-NMR (400 MHz, DMSO-d6) δ 12.88 (s, 1H), 10.32 (s, 1H), 8.43 - 8.25 (m, 2H), 8.04 (s, 1H), 7.96 - 7.69 (m, 4H), 7.24 - 7.07 (m, 1H), 3.83 (s, 3H), 3.42 - 3.32 (m, 2H), 2.94 - 2.81 (m, 2H), 1.73 (s, 6H).

LCMS (m/z) : 486 (M+H).

### Example 61. Preparation of 5-(1-acetyl-1,2,3,6-tetrahydropyridine-4-yl)-2-methoxy-N-(5-oxo-5,6,7,8-tetrahydro-1,6-naphthyridine-3-yl)benzenesulfonamide

A target compound (14 mg, yield: 46%) was obtained by reacting 1-(4-(4,4,5,5-tetramethyl-1,3,2-dioxaborolane-2-yl)-3,6-dihydropyridine-1(2H)-yl)ethane-1-one with the compound of Example 9 according to the manufacturing method of Example 27.

¹H-NMR (400 MHz, DMSO-d6) δ 10.35 (s, 1H), 8.33 - 8.20 (m, 1H), 8.05 (s, 1H), 7.86 - 7.78 (m, 1H), 7.76 - 7.63 (m, 1H), 7.60 (dd, J = 9.2, 2.9 Hz, 1H), 7.12 (d, J = 8.2 Hz, 1H), 6.04 (s, 1H), 3.91 (s, 2H), 3.80 (s, 3H), 3.48 (d, J = 5.7 Hz, 2H), 3.31 (dd, J = 6.9, 2.8 Hz, 2H), 2.84 (t, J = 6.6 Hz, 2H), 2.38 (s, 3H), 2.32-2.28 (m, 2H).

LCMS (m/z) : 457 (M+H).

### Example 62. Preparation of 5-(5,6-dihydro-2H-pyran-3-yl)-2-methoxy-N-(5-oxo-5,6,7,8-tetrahydro-1,6-naphthyridine-3-yl)benzenesulfonamide

A target compound (14 mg, yield: 46%) was obtained by reacting 2-(5,6-dihydro-2H-pyran-3-yl)-4,4,5,5-tetramethyl-1,3,2-dioxaborolane with the compound of Example 9 according to the manufacturing method of Example 27.

¹H-NMR (400 MHz, DMSO-d6) δ 10.39 (s, 1H), 8.28 (d, J = 7.0 Hz, 1H), 8.07 (s, 1H), 7.90 - 7.79 (m, 1H), 7.69 - 7.59 (m, 1H), 7.59 - 7.41 (m, 1H), 7.20 - 7.04 (m, 1H), 6.24 - 6.11 (m, 1H), 4.36 - 4.25 (m, 2H), 3.80 (s, 3H), 3.70 - 3.58 (m, 2H), 3.40 - 3.29 (m, 2H), 2.86 (t, J = 7.3 Hz, 2H), 2.25 - 2.10 (m, 2H).

LCMS (m/z) : 416 (M+H).

### Example 63. Preparation of 4-methoxy-N-(5-oxo-5,6,7,8-tetrahydro-1,6-naphthyridine-3-yl)-2',3',4',5'-tetrahydro-[1,1'-biphenyl]-3-sulfonamide

A target compound (15 mg, yield: 30%) was obtained by reacting 2-(cyclohex-1-en-1-yl)-4,4,5,5-tetramethyl-1,3,2-dioxaborolane with the compound of Example 9 according to the manufacturing method of Example 27.

¹H-NMR (400 MHz, DMSO-d6) δ 10.35 (s, 1H), 8.35 - 8.21 (m, 1H), 8.06 (s, 1H), 7.92 - 7.77 (m, 1H), 7.69 (dt, J = 7.8, 3.8 Hz, 1H), 7.62 - 7.47 (m, 1H), 7.09 (dd, J = 8.9, 5.0 Hz, 1H), 6.13 - 5.99 (m, 1H), 3.80 (s, 3H), 3.32 (dd, J = 7.1, 3.2 Hz, 2H), 2.85 (q, J = 6.0, 5.5 Hz, 2H), 2.30 - 2.17 (m, 2H), 2.16 - 2.03 (m, 2H), 1.73 - 1.59 (m, 2H), 1.58 - 1.43 (m, 2H).

LCMS (m/z) : 414 (M+H).

### Example 64. Preparation of 4',4'-difluoro-4-methoxy-N-(5-oxo-5,6,7,8-tetrahydro-1,6-naphthyridine-3-yl)-2',3',4',5'-tetrahydro-[1,1'-biphenyl]-3-sulfonamide

A target compound (8 mg, yield: 25%) was obtained by reacting 2-(4,4-difluorocyclohex-1-en-1-yl)-4,4,5,5-tetramethyl-1,3,2-dioxaborolane with the compound of Example 9 according to the manufacturing method of Example 27.

¹H-NMR (400 MHz, DMSO-d6) δ 10.37 (s, 1H), 8.39 - 8.24 (m, 1H), 8.06 (s, 1H), 7.91 - 7.81 (m, 1H), 7.78 - 7.71 (m, 1H), 7.68 - 7.57 (m, 1H), 7.13 (tt, J = 8.9, 5.0 Hz, 1H), 5.91 (s, 1H), 3.82 (s, 3H), 3.38 - 3.29 (m, 2H), 2.93 - 2.80 (m, 2H), 2.76 - 2.50 (m, 4H), 2.23 - 2.02 (m, 2H).

LCMS (m/z) : 450 (M+H).

### Example 65. Preparation of 2-methoxy-5-(1-(1-methylpiperidine-4-yl)-1H-pyrazole-4-yl)-N-(5-oxo-5,6,7,8-tetrahydro-1,6-naphthyridine-3-yl)benzenesulfonamide

A target compound (12 mg, yield: 33%) was obtained by reacting 1-methyl-4-(4-(4,4,5,5-tetramethyl-1,3-dioxolane-2-yl)-1H-pyrazole-1-yl)piperidine with the compound of Example 9 according to the manufacturing method of Example 27.

¹H-NMR (400 MHz, DMSO-d6) δ 8.49 (s, 1H), 8.01 (s, 1H), 7.92 (d, J = 2.8 Hz, 1H), 7.85 (d, J = 2.4 Hz, 1H), 7.69 - 7.62 (m, 3H), 7.42 (dd, J = 8.4, 2.5 Hz, 1H), 6.91 (d, J = 8.4 Hz, 1H), 4.10-3.98 (m, 1H), 3.65 (s, 3H), 2.82 - 2.68 (m, 4H), 2.15 (s, 3H), 1.98 - 1.91 (m, 4H).

LCMS (m/z) : 497 (M+H).

### Example 66. Preparation of N-ethyl-2-(4-(4-methoxy-3-(N-(5-oxo-5,6,7,8-tetrahydro-1,6-naphthyridine-3-yl)sulfamoyl)phenyl)-1H-pyrazole-1-yl)acetamide

A mixture of the compound of Example 48 (20 mg, 0.044 mmol), EDCI (13 mg, 0.066 mmol), ethaneamine (4 mg, 0.087 mmol), HOBt (6 mg, 0.044 mmol), and DIPEA (9 mg, 0.066 mmol) was stirred in dichloromethane (20 mL) for 24 hours. The reaction mixture was diluted with water and extracted with dichloromethane. The extract was washed with brine, dried over anhydrous sodium sulfate and concentrated in vacuo. The residue was purified by column chromatography to give a target compound (8 mg, yield: 38%).

¹H-NMR (400 MHz, DMSO-d6) δ 10.36 (s, 1H), 8.33 (d, J = 2.9 Hz, 1H), 8.08 (s, 1H), 7.96 - 7.63 (m, 4H), 7.54 - 7.45 (m, 1H), 7.37 (t, J = 7.8 Hz, 1H), 7.16 (d, J = 8.7 Hz, 1H), 4.71 (s, 2H), 3.82 (s, 3H), 2.89 - 2.72 (m, 6H), 1.02 - 0.97 (m, 3H).

LCMS (m/z) : 485 (M+H).

### Example 67. Preparation of N-ethyl-3-(4'-methoxy-3'-(N-(5-oxo-5,6,7,8-tetrahydro-1,6-naphthyridine-3-yl)sulfamoyl)-[1,1'-biphenyl]-4-yl)propanamide

The compound of Example 50 was reacted according to the manufacturing method of Example 66 to give a target compound (11 mg, yield: 52%).

¹H-NMR (400 MHz, DMSO-d6) δ 10.46 (s, 1H), 8.36 - 8.28 (m, 1H), 8.10 - 8.02 (m, 1H), 7.94 - 7.86 (m, 2H), 7.86 - 7.77 (m, 2H), 7.47 - 7.40 (m, 2H), 7.25 - 7.21 (m, 3H), 3.84 (s, 3H), 2.91 - 2.72 (m, 6H), 2.42-2.38 (m, 2H), 2.34 - 2.29 (m, 2H), 0.96 - 0.90 (m, 3H).

LCMS (m/z) : 509 (M+H).

### Example 68. Preparation of ethyl 2-(4-(4-methoxy-3-(N-(5-oxo-5,6,7,8-tetrahydro-1,6-naphthyridine-3-yl)sulfamoyl)phenyl)-1H-pyrazole-1-yl)acetate

A target compound (11 mg, yield: 31%) was obtained by reacting ethyl 2-(4-(4,4,5,5-tetramethyl-1,3,2-dioxaborolane-2-yl)-1H-pyrazole-1-yl)acetate with the compound of Example 9 according to the manufacturing method of Example 27.

¹H-NMR (400 MHz, DMSO-d6) δ 10.32 (s, 1H), 8.39 - 8.26 (m, 1H), 8.21 - 8.08 (m, 1H), 8.03 (s, 1H), 7.93 - 7.67 (m, 4H), 7.24 - 7.09 (m, 1H), 5.07 - 4.95 (m, 2H), 4.20 - 4.03 (m, 2H), 3.82 (s, 3H), 3.40 - 3.29 (m, 2H), 2.94 - 2.77 (m, 2H), 1.21 - 1.15 (m, 3H).

LCMS (m/z) : 486 (M+H).

### Example 69. Preparation of 5-(1-(1-(3-isopropyl-1,2,4-oxadiazole-5-yl)ethyl)-1H-pyrazole-4-yl)-2-methoxy-N-(5-oxo-5,6,7,8-tetrahydro-1,6-naphthyridine-3-yl)benzenesulfonamide

A mixture of the compound of Example 55 (50 mg, 0.125 mmol), K₂CO₃ (35 mg, 0.250 mmol), and 5-(1-chloroethyl)-3-isopropyl-1,2,4-oxadiazole (24 mg, 0.138 mmol) was stirred in DMF (10 mL) for 24 hours. The reaction mixture was diluted with water and extracted with ethyl acetate. The extract was washed with brine, dried over anhydrous sodium sulfate and concentrated in vacuo. The residue was purified by column chromatography to give a target compound (22 mg, yield: 39%).

¹H-NMR (400 MHz, DMSO-d6) δ 10.31 (s, 1H), 8.42 (d, J = 7.7 Hz, 1H), 8.36 - 8.25 (m, 1H), 8.03 (s, 1H), 7.92 - 7.84 (m, 4H), 7.25 - 7.09 (m, 1H), 6.09 - 5.93 (m, 1H), 3.84 (s, 3H), 2.89 - 2.83 (m, 4H), 2.72 - 2.68 (m, 1H), 1.87 (s, 3H), 1.20 (s, 6H).

LCMS (m/z) : 538 (M+H).

### Example 70. Preparation of 2-methoxy-5-(1-(1-(3-methyl-1,2,4-oxadiazole-5-yl)ethyl)-1H-pyrazole-4-yl)-N-(5-oxo-5,6,7,8-tetrahydro-1,6-naphthyridine-3-yl)benzenesulfonamide

A target compound (6 mg, yield: 43%) was obtained by reacting 5-(1-chloroethyl)-3-methyl-1,2,4-oxadiazole with the compound of Example 55 according to the manufacturing method of Example 69.

¹H-NMR (400 MHz, DMSO-d6) δ 10.32 (s, 1H), 8.37 - 8.28 (m, 1H), 8.03 (s, 1H), 7.95 - 7.82 (m, 3H), 7.81 - 7.68 (m, 2H), 7.21 - 7.13 (m, 1H), 5.90-5.75 (m, 1H), 3.83 (s, 3H), 2.88 - 2.83 (m, 4H), 2.70 (s, 3H), 1.71 (s, 3H).

LCMS (m/z) : 510 (M+H).

### Example 71. Preparation of 5-(1-(1-(3-(4-fluorophenyl)-1,2,4-oxadiazole-5-yl)ethyl)-1H-pyrazole-4-yl)-2-methoxy-N-(5-oxo-5,6,7,8-tetrahydro-1,6-naphthyridine-3-yl)benzenesulfonamide

A target compound (11 mg, yield: 31%) was obtained by reacting 5-(1-chloroethyl)-3-(4-fluorophenyl)-1,2,4-oxadiazole with the compound of Example 55 according to the manufacturing method of Example 69.

¹H-NMR (400 MHz, DMSO-d6) δ 10.32 (s, 1H), 8.54 - 8.40 (m, 1H), 8.32 (s, 1H), 8.12 - 7.67 (m, 5H), 7.56 - 7.34 (m, 2H), 7.21 - 7.14 (m, 1H), 6.18 - 6.10 (m, 1H), 5.89 - 5.74 (m, 1H), 4.47 - 4.30 (m, 1H), 3.84 (s, 3H), 2.94 - 2.80 (m, 2H), 2.77 - 2.59 (m, 2H), 1.94 (s, 3H).

LCMS (m/z) : 590 (M+H).

### Example 72. Preparation of 5-(1-isopropyl-1H-pyrazole-4-yl)-2-methoxy-N-(5-oxo-5,6,7,8-tetrahydro-1,6-naphthyridine-3-yl)benzenesulfonamide

A target compound (18 mg, yield: 34%) was obtained by reacting 1-isopropyl-4-(4,4,5,5-tetramethyl-1,3-dioxolane-2-yl)-1H-pyrazole with the compound of Example 9 according to the manufacturing method of Example 27.

¹H-NMR (400 MHz, DMSO-d6) δ 10.31 (s, 1H), 8.32 (t, J = 2.9 Hz, 1H), 8.19 (d, J = 8.4 Hz, 1H), 8.03 (s, 1H), 7.93 - 7.67 (m, 4H), 7.22 - 7.10 (m, 1H), 4.52 - 4.37 (m, 1H), 3.84 (s, 3H), 3.40-3.30 (m, 2H), 2.95 - 2.80 (m, 2H), 1.39 (s, 6H).

LCMS (m/z) : 442 (M+H).

### Example 73. Preparation of 2-methoxy-N-(5-oxo-5,6,7,8-tetrahydro-1,6-naphthyridine-3-yl)-5-(1-(trifluoromethyl)-1H-pyrazole-4-yl)benzenesulfonamide

A target compound (21 mg, yield: 37%) was obtained by reacting 4-(4,4,5,5-tetramethyl-1,3-dioxolane-2-yl)-1-(trifluoromethyl)-1H-pyrazole with the compound of Example 9 according to the manufacturing method of Example 27.

¹H-NMR (400 MHz, DMSO-d6) δ 10.37 (s, 1H), 8.98 (d, J = 5.2 Hz, 1H), 8.46 - 8.28 (m, 2H), 8.12 - 8.00 (m, 2H), 7.97 - 7.81 (m, 2H), 7.30 - 7.17 (m, 1H), 3.86 (s, 3H), 3.39 - 3.30 (m, 2H), 2.92 - 2.80 (m, 2H).

LCMS (m/z) : 468 (M+H).

### Example 74. Preparation of 5-(1-cyclopropyl-1H-pyrazole-4-yl)-2-methoxy-N-(5-oxo-5,6,7,8-tetrahydro-1,6-naphthyridine-3-yl)benzenesulfonamide

A target compound (23 mg, yield: 43%) was obtained by reacting 1-cyclopropyl-4-(4,4,5,5-tetramethyl-1,3-dioxolane-2-yl)-1H-pyrazole with the compound of Example 9 according to the manufacturing method of Example 27.

¹H-NMR (400 MHz, DMSO-d6) δ 10.31 (s, 1H), 8.37 - 8.24 (m, 1H), 8.22 - 8.12 (m, 1H), 8.02 (s, 1H), 7.84 (d, J = 6.7 Hz, 2H), 7.73 (t, J = 8.4 Hz, 2H), 7.13 (d, J = 8.2 Hz, 1H), 3.82 (s, 3H), 3.73 - 3.61 (m, 1H), 3.36-3.30 (m, 2H), 2.92 - 2.77 (m, 2H), 1.09 - 0.98 (m, 2H), 0.96 - 0.85 (m, 2H).

LCMS (m/z) : 440 (M+H).

### Example 75. Preparation of 2-methoxy-5-(1-(methylsulfonyl)-1H-pyrazole-4-yl)-N-(5-oxo-5,6,7,8-tetrahydro-1,6-naphthyridine-3-yl)benzenesulfonamide

A target compound (21 mg, yield: 36%) was obtained by reacting 1-(methylsulfonyl)-4-(4,4,5,5-tetramethyl-1,3,2-dioxaborolane-2-yl)-1H-pyrazole with the compound of Example 9 according to the manufacturing method of Example 27.

¹H-NMR (400 MHz, DMSO-d6) δ 10.37 (s, 1H), 8.75 (dd, J = 7.8, 4.7 Hz, 1H), 8.45 - 8.28 (m, 2H), 8.10 - 7.99 (m, 2H), 7.99 - 7.82 (m, 2H), 7.28 - 7.15 (m, 1H), 3.86 (s, 3H), 3.52 (s, 3H), 3.38 - 3.29 (m, 2H), 2.92 - 2.78 (m, 2H).

LCMS (m/z) : 478 (M+H).

### Example 76. Preparation of 2-methoxy-N-(5-oxo-5,6,7,8-tetrahydro-1,6-naphthyridine-3-yl)-5-(1-(tetrahydro-2H-pyran-4-yl)-1H-pyrazole-4-yl)benzenesulfonamide

A target compound (17 mg, yield: 29%) was obtained by reacting 1-(tetrahydro-2H-pyran-4-yl)-4-(4,4,5,5-tetramethyl-1,3-dioxolane-2-yl)-1H-pyrazole with the compound of Example 9 according to the manufacturing method of Example 27.

¹H-NMR (400 MHz, DMSO-d6) δ 10.32 (s, 1H), 8.36 - 8.28 (m, 1H), 8.24 (d, J = 3.9 Hz, 1H), 8.03 (s, 1H), 7.94 - 7.83 (m, 2H), 7.83 - 7.68 (m, 2H), 7.15 (dd, J = 8.5, 4.1 Hz, 1H), 4.42 - 4.27 (m, 1H), 3.99 - 3.86 (m, 2H), 3.83 (s, 3H), 3.50 - 3.36 (m, 2H), 3.36 - 3.29 (m, 2H), 2.93 - 2.78 (m, 2H), 1.99 - 1.87 (m, 4H).

LCMS (m/z) : 484 (M+H).

### Example 77. Preparation of 5-(6-fluoropyridine-3-yl)-2-methoxy-N-(5-oxo-5,6,7,8-tetrahydro-1,6-naphthyridine-3-yl)benzenesulfonamide

A target compound (22 mg, yield: 42%) was obtained by reacting 2-fluoro-5-(4,4,5,5-tetramethyl-1,3,2-dioxaborolane-2-yl)pyridine with the compound of Example 9 according to the manufacturing method of Example 27.

¹H-NMR (400 MHz, DMSO-d6) δ 10.48 (s, 1H), 8.45 (t, J = 4.5 Hz, 1H), 8.38 - 8.29 (m, 1H), 8.19 (t, J = 7.7 Hz, 1H), 8.05 (s, 1H), 8.01 - 7.95 (m, 1H), 7.94 - 7.85 (m, 2H), 7.34 - 7.16 (m, 2H), 3.87 (s, 3H), 3.40-3.35 (m, 2H), 2.91 - 2.80 (m, 2H).

LCMS (m/z) : 429 (M+H).

### Example 78. Preparation of 5-(2-amino-4-(trifluoromethyl)pyrimidine-5-yl)-2-methoxy-N-(5-oxo-5,6,7,8-tetrahydro-1,6-naphthyridine-3-yl)benzenesulfonamide

A target compound (18 mg, yield: 30%) was obtained by reacting 5-(4,4,5,5-tetramethyl-1,3,2-dioxaborolane-2-yl)-4-(trifluoromethyl)pyrimidine-2-amine with the compound of Example 9 according to the manufacturing method of Example 27.

¹H-NMR (400 MHz, DMSO-d6) δ 10.40 (s, 1H), 8.37 - 8.21 (m, 2H), 8.06 (s, 1H), 7.84 (tt, J = 8.4, 4.2 Hz, 1H), 7.61 (dd, J = 7.1, 4.6 Hz, 1H), 7.54 - 7.42 (m, 1H), 7.33 (s, 2H), 7.28 - 7.15 (m, 1H), 3.86 (s, 3H), 3.39 - 3.30 (m, 2H), 2.94 - 2.77 (m, 2H).

LCMS (m/z) : 495 (M+H).

### Example 79. Preparation of 2-methoxy-5-(1-(oxetane-3-yl)-1H-pyrazole-4-yl)-N-(5-oxo-5,6,7,8-tetrahydro-1,6-naphthyridine-3-yl)benzenesulfonamide

A target compound (16 mg, yield: 29%) was obtained by reacting 1-(oxetane-3-yl)-4-(4,4,5,5-tetramethyl-1,3-dioxolane-2-yl)-1H-pyrazole with the compound of Example 9 according to the manufacturing method of Example 27.

¹H-NMR (400 MHz, DMSO-d6) δ 10.33 (s, 1H), 8.42 - 8.24 (m, 2H), 8.03 (s, 1H), 7.98 - 7.82 (m, 3H), 7.83 - 7.71 (m, 1H), 7.26 - 7.09 (m, 1H), 5.59 - 5.42 (m, 1H), 4.95 - 4.77 (m, 4H), 3.83 (s, 3H), 3.41 - 3.29 (m, 2H), 2.94 - 2.74 (m, 2H).

LCMS (m/z) : 456 (M+H).

### Example 80. Preparation of 5-(1-(2-hydroxyethyl)-1H-pyrazole-4-yl)-2-methoxy-N-(5-oxo-5,6,7,8-tetrahydro-1,6-naphthyridine-3-yl)benzenesulfonamide

A target compound (25 mg, yield: 47%) was obtained by reacting 2-(4-(4,4,5,5-tetramethyl-1,3-dioxolane-2-yl)-1H-pyrazole-1-yl)ethane-1-ol with the compound of Example 9 according to the manufacturing method of Example 27.

¹H-NMR (400 MHz, DMSO-d6) δ 10.32 (s, 1H), 8.40 - 8.27 (m, 1H), 8.17 - 8.07 (m, 1H), 8.03 (s, 1H), 7.89 - 7.73 (m, 3H), 7.64 - 7.43 (m, 1H), 7.23 -7.09 (m, 1H), 4.85 (s, 1H), 4.14 - 4.06 (m, 2H), 3.83 (s, 3H), 3.74 - 3.66 (m, 2H), 3.40 - 3.30 (m, 2H), 2.92 - 2.77 (m, 2H).

LCMS (m/z) : 444 (M+H).

### Example 81. Preparation of 5-(1-isopropyl-3-(trifluoromethyl)-1H-pyrazole-4-yl)-2-methoxy-N-(5-oxo-5,6,7,8-tetrahydro-1,6-naphthyridine-3-yl)benzenesulfonamide

A target compound (15 mg, yield: 41%) was obtained by reacting 1-isopropyl-4-(4,4,5,5-tetramethyl-1,3,2-dioxaborolane-2-yl)-3-(trifluoromethyl)-1H-pyrazole with the compound of Example 9 according to the manufacturing method of Example 27.

¹H-NMR (400 MHz, DMSO-d6) δ 10.40 (s, 1H), 8.37 - 8.18 (m, 2H), 8.05 (s, 1H), 7.92 - 7.79 (m, 1H), 7.75 (d, J = 5.6 Hz, 1H), 7.62 - 7.51 (m, 1H), 7.29 - 7.17 (m, 1H), 4.64 - 4.47 (m, 1H), 3.85 (s, 3H), 3.40 - 3.30 (m, 2H), 2.95 - 2.80 (m, 2H), 1.55 - 1.26 (m, 6H).

LCMS (m/z) : 510 (M+H).

### Example 82. Preparation of 5-(1-ethyl-3-(trifluoromethyl)-1H-pyrazole-4-yl)-2-methoxy-N-(5-oxo-5,6,7,8-tetrahydro-1,6-naphthyridine-3-yl)benzenesulfonamide

A target compound (9 mg, yield: 25%) was obtained by reacting 1-ethyl-4-(4,4,5,5-tetramethyl-1,3,2-dioxaborolane-2-yl)-3-(trifluoromethyl)-1H-pyrazole with the compound of Example 9 according to the manufacturing method of Example 27.

¹H-NMR (400 MHz, DMSO-d6) δ 10.43 (s, 1H), 8.33 - 8.24 (m, 1H), 8.18 (d, J = 6.7 Hz, 1H), 8.06 (s, 1H), 7.91 - 7.80 (m, 1H), 7.77 - 7.68 (m, 1H), 7.56 (d, J = 8.8 Hz, 1H), 7.31 - 7.09 (m, 1H), 4.29 - 4.03 (m, 2H), 3.83 (s, 3H), 3.40 - 3.31 (m, 2H), 2.96 - 2.82 (m, 2H), 1.47 - 1.31 (m, 3H).

LCMS (m/z) : 496 (M+H).

### Example 83. Preparation of 5-(isoxazole-4-yl)-2-methoxy-N-(5-oxo-5,6,7,8-tetrahydro-1,6-naphthyridine-3-yl)benzenesulfonamide

A target compound (13 mg, yield: 45%) was obtained by reacting 4-(4,4,5,5-tetramethyl-1,3,2-dioxaborolane-2-yl)isoxazole with the compound of Example 9 according to the manufacturing method of Example 27.

¹H-NMR (400 MHz, DMSO-d6) δ 10.38 (s, 1H), 8.36 - 8.15 (m, 2H), 8.02 (s, 1H), 7.90 - 7.76 (m, 2H), 7.70 (d, J = 5.6 Hz, 1H), 7.60 - 7.49 (m, 1H), 7.30 - 7.18 (m, 1H), 3.82 (s, 3H), 3.39 - 3.28 (m, 2H), 2.93 - 2.78 (m, 2H).

LCMS (m/z) : 401 (M+H).

### Example 84. Preparation of 5-(3,5-dimethylisoxazole-4-yl)-2-methoxy-N-(5-oxo-5,6,7,8-tetrahydro-1,6-naphthyridine-3-yl)benzenesulfonamide

A target compound (14 mg, yield: 45%) was obtained by reacting 3,5-dimethyl-4-(4,4,5,5-tetramethyl-1,3,2-dioxaborolane-2-yl)isoxazole with the compound of Example 9 according to the manufacturing method of Example 27.

¹H-NMR (400 MHz, DMSO-d6) δ 10.34 (s, 1H), 8.37 - 8.16 (m, 1H), 8.04 (s, 1H), 7.88 - 7.75 (m, 1H), 7.72 (d, J = 5.6 Hz, 1H), 7.58 - 7.47 (m, 1H), 7.28 - 7.15 (m, 1H), 3.80 (s, 3H), 3.37 - 3.25 (m, 2H), 2.90 - 2.75 (m, 2H), 2.3 (s, 3H), 2.1 (s, 3H).

LCMS (m/z) : 429 (M+H).

### Example 85. Preparation of tert-butyl 4-(4-(4-methoxy-3-(N-(5-oxo-5,6,7,8-tetrahydro-1,6-naphthyridine-3-yl)sulfamoyl)phenyl)-1H-pyrazole-1-yl)piperidine-1-carboxylate

A target compound (25 mg, yield: 59%) was obtained by reacting tert-butyl 4-(4-(4,4,5,5-tetramethyl-1,3-dioxolane-2-yl)-1H-pyrazole-1-yl)piperidine-1-carboxylate with the compound of Example 9 according to the manufacturing method of Example 27.

¹H-NMR (400 MHz, DMSO-d6) δ 10.29 (s, 1H), 8.37 - 8.17 (m, 1H), 8.00 (s, 1H), 7.90 - 7.81 (m, 1H), 7.77 (s, 1H), 7.74 - 7.67 (m, 1H), 7.58 - 7.40 (m, 2H), 7.13 (d, J = 8.6 Hz, 1H), 4.51 - 4.38 (m, 1H), 4.35 - 4.17 (m, 2H), 4.04 - 3.96 (m, 2H), 3.81 (s, 3H), 3.35-3.30 (m, 2H), 2.87 - 2.79 (m, 2H), 2.04 -1.92 (m, 2H), 1.82 - 1.71 (m, 2H), 1.38 (s, 9H).

LCMS (m/z) : 583 (M+H).

### Example 86. Preparation of 2-methoxy-N-(5-oxo-5,6,7,8-tetrahydro-1,6-naphthyridine-3-yl)-5-(1-(piperidine-4-yl)-1H-pyrazole-4-yl)benzenesulfonamide hydrochloride

The compound of Example 85 was reacted according to the manufacturing method of Example 53 to give a target compound (14 mg, yield: 37%).

¹H-NMR (400 MHz, DMSO-d6) δ 10.47 (s, 1H), 9.16 (s, 1H), 8.89 (s, 1H), 8.40 - 8.30 (m, 1H), 8.22 (dd, J = 7.6, 4.9 Hz, 1H), 8.11 (s, 1H), 8.01 - 7.82 (m, 3H), 7.75 (dt, J = 8.4, 4.3 Hz, 1H), 7.15 (dd, J = 9.1, 5.8 Hz, 1H), 4.52 - 4.38 (m, 1H), 3.83 (s, 3H), 3.41 - 3.21 (m, 4H), 3.14 - 2.95 (m, 2H), 2.94 - 2.81 (m, 2H), 2.24 - 2.04 (m, 4H).

LCMS (m/z) : 483 (M+H).

### Example 87. Preparation of 5-bromo-2-methoxy-N-(6-methyl-5-oxo-5,6,7,8-tetrahydro-1,6-naphthyridine-3-yl)benzenesulfonamide

A target compound (2 mg, yield: 8%) was obtained by reacting 5-bromo-2-methoxybenzenesulfonyl chloride and 3-amino-6-methyl-7,8-dihydro-1,6-naphthyridine-5(6H)-one according to the manufacturing method of Example 1.

¹H-NMR (400 MHz, DMSO-d6) δ 10.47 (s, 1H), 8.30 (d,J = 2.6 Hz, 1H), 8.07 (s, 1H), 7.85 (d, J = 2.7 Hz, 1H), 7.79 - 7.70 (m, 2H), 7.15 (d, J = 8.8 Hz, 1H), 3.81 (s, 3H), 3.35 (td, J = 6.7, 2.8 Hz, 2H), 3.2 (s, 3H), 2.89 (t, J = 6.7 Hz, 2H).

LCMS (m/z) : 427 (M+H).

### Example 88. Preparation of tert-butyl 3-((5-bromo-2-methoxyphenyl)sulfonamido)-7,8-dihydro-1,6-naphthyridine-6(5H)-carboxylate

### Step 1. Preparation of tert-butyl 3-amino-7,8-dihydro-1,6-naphthyridine-6(5H)-carboxylate

A target compound (840 mg, 94%) was obtained by reacting tert-butyl 3-nitro-7,8-dihydro-1,6-naphthyridine-6(5H)-carboxylate according to the manufacturing method of step 2 of Intermediate 4.

### Step 2. Preparation of tert-butyl 3-((5-bromo-2-methoxyphenyl)sulfonamido)-7,8-dihydro-1,6-naphthyridine-6(5H)-carboxylate

A target compound (201 mg, yield: 39%) was obtained by reacting 5-bromo-2-methoxybenzenesulfonyl chloride and tert-butyl 3-amino-7,8-dihydro-1,6-naphthyridine-6(5H)-carboxylate according to the manufacturing method of Example 1.

¹H-NMR (400 MHz, DMSO-d6) δ 10.28 (s, 1H), 8.05 (d, J = 2.6 Hz, 1H), 7.87 - 7.67 (m, 2H), 7.25 (d, J = 2.5 Hz, 1H), 7.14 (d, J = 8.8 Hz, 1H), 4.40 (s, 2H), 3.82 (s, 3H), 3.54 (t, J = 5.9 Hz, 2H), 1.36 (s, 9H).

LCMS (m/z) : 499 (M+H).

### Example 89. Preparation of tert-butyl 3-((2,5-dimethoxyphenyl)sulfonamido)-7,8-dihydro-1,6-naphthyridine-6(5H)-carboxylate

A target compound (125 mg, yield: 69%) was obtained by reacting 2,5-dimethoxybenzenesulfonyl chloride and tert-butyl 3-amino-7,8-dihydro-1,6-naphthyridine-6(5H)-carboxylate according to the manufacturing method of Example 1.

¹H-NMR (400 MHz, DMSO-d6) δ 10.15 (s, 1H), 8.05 (d, J = 2.5 Hz, 1H), 7.23 (dd, J = 11.4, 2.7 Hz, 2H), 7.17 - 7.06 (m, 2H), 4.39 (s, 2H), 3.77 (s, 3H), 3.68 (s, 3H), 3.53 (t, J = 6.0 Hz, 2H), 2.69 (t, J = 6.0 Hz, 2H), 1.36 (s, 9H).

LCMS (m/z) : 450 (M+H).

### Example 90. Preparation of 5-fluoro-2-methoxy-N-(5,6,7,8-tetrahydro-1,6-naphthyridine-3-yl)benzenesulfonamide hydrochloride

A target compound (34 mg, yield: 98%) was obtained by reacting tert-butyl 3-((5-fluoro-2-methoxyphenyl)sulfonamido)-7,8-dihydro-1,6-naphthyridine-6(5H)-carboxylate according to the manufacturing method of Example 53.

¹H-NMR (400 MHz, DMSO-d6) δ 10.47 (s, 1H), 9.23 (s, 2H), 8.14 (d, J = 2.5 Hz, 1H), 7.53 (dd, J = 8.0, 3.2 Hz, 1H), 7.45 (ddd, J = 9.1, 8.0, 3.2 Hz, 1H), 7.37 (d, J = 2.5 Hz, 1H), 7.20 (dd, J = 9.2, 4.0 Hz, 1H), 4.21 (s, 2H), 3.82 (s, 3H), 3.53 (s, 1H), 3.37 (d, J = 6.4 Hz, 2H), 2.93 (t, J = 6.3 Hz, 2H).

LCMS (m/z) : 338 (M+H).

### Example 91. Preparation of 5-fluoro-2-bromo-N-(5,6,7,8-tetrahydro-1,6-naphthyridine-3-yl)benzenesulfonamide hydrochloride

A target compound (128 mg, yield: 95%) was obtained by reacting tert-butyl 3-((5-fluoro-2-bromo)sulfonamido)-7,8-dihydro-1,6-naphthyridine-6(5H)-carboxylate according to the manufacturing method of Example 53.

¹H-NMR (400 MHz, DMSO-d6) δ 11.13 (s, 1H), 9.27 (s, 2H), 8.20 (d, J = 2.6 Hz, 1H), 7.99 - 7.71 (m, 2H), 7.52 - 7.30 (m, 2H), 4.22 (s, 2H), 3.37 (s, 2H), 2.94 (t, J = 6.3 Hz, 2H).

LCMS (m/z) : 387 (M+H).

### Example 92. Preparation of 5-chloro-2-methoxy-N-(5,6,7,8-tetrahydro-1,6-naphthyridine-3-yl)benzenesulfonamide hydrochloride

A target compound (22 mg, yield: 99%) was obtained by reacting tert-butyl 3-((5-chloro-2-methoxyphenyl)sulfonamido)-7,8-dihydro-1,6-naphthyridine-6(5H)-carboxylate according to the manufacturing method of Example 53.

¹H-NMR (400 MHz, DMSO-d6) δ 10.50 (s, 1H), 9.37 (s, 2H), 8.15 (d, J = 2.5 Hz, 1H), 7.80 - 7.50 (m, 2H), 7.40 (d, J = 2.5 Hz, 1H), 7.21 (d, J = 9.0 Hz, 1H), 3.83 (s, 3H), 3.37 (d, J = 6.8 Hz, 2H), 2.95 (t, J = 6.3 Hz, 2H).

LCMS (m/z) : 354 (M+H).

### Example 93. Preparation of 5-bromo-2-methoxy-N-(5,6,7,8-tetrahydro-1,6-naphthyridine-3-yl)benzenesulfonamide hydrochloride

A target compound (169 mg, yield: 96%) was obtained by reacting tert-butyl 3-((5-bromo-2-methoxyphenyl)sulfonamido)-7,8-dihydro-1,6-naphthyridine-6(5H)-carboxylate according to the manufacturing method of Example 53.

¹H-NMR (400 MHz, DMSO-d6) δ 10.46 (s, 1H), 9.26 (s, 2H), 8.14 (d, J = 2.5 Hz, 1H), 7.83 - 7.68 (m, 2H), 7.39 (d, J = 2.5 Hz, 1H), 7.16 (d, J = 8.9 Hz, 1H), 4.22 (s, 2H), 3.83 (s, 3H), 3.37 (d, J = 6.9 Hz, 2H), 2.94 (t, J = 6.3 Hz, 2H).

LCMS (m/z) : 399 (M+H).

### Example 94. Preparation of 2,5-dimethoxy-N-(5,6,7,8-tetrahydro-1,6-naphthyridine-3-yl)benzenesulfonamide hydrochloride

A target compound (111 mg, yield: 99%) was obtained by reacting tert-butyl 3-((2,5-dimethoxyphenyl)sulfonamido)-7,8-dihydro-1,6-naphthyridine-6(5H)-carboxylate according to the manufacturing method of Example 53.

¹H-NMR (400 MHz, DMSO-d6) δ 10.35 (s, 1H), 9.26 (s, 2H), 8.15 (d, J = 2.5 Hz, 1H), 7.38 (d, J = 2.5 Hz, 1H), 7.23 (d, J = 2.9 Hz, 1H), 7.18 - 7.06 (m, 2H), 4.21 (s, 2H), 3.77 (s, 3H), 3.69 (s, 3H), 3.36 (s, 2H), 2.92 (t, J = 6.3 Hz, 2H).

LCMS (m/z) : 350 (M+H).

### Example 95. Preparation of ethyl3-((5-fluoro-2-methoxyphenyl)sulfonamido)-7,8-dihydro-1,6-naphthyridine-6(5H)-carboxylate

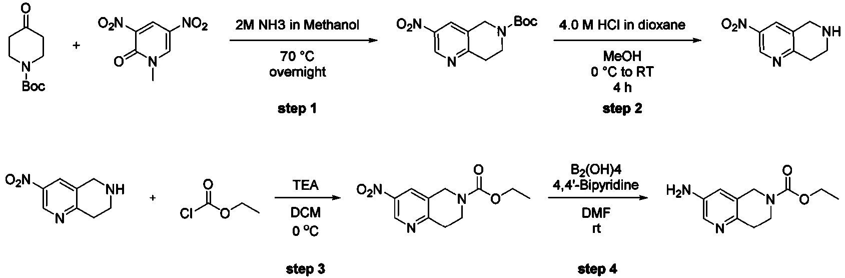

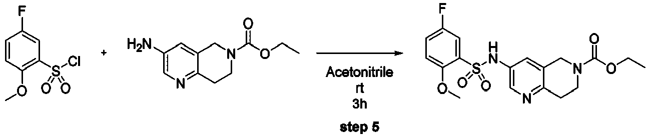

### Step 1. Preparation of tert-butyl 3-nitro-7,8-dihydro-1,6-naphthyridine-6(5H)-carboxylate

Tert-butyl 4-oxopiperidine-1-carboxylate (8.3 g, 41.656 mmol, 1.0 eq) and 1-methyl-3,5-dinitropyridine-2(1H)-one (9.124 g, 45.822 mmol, 1.1 q) were added to 80 mL of methanol at room temperature, to which 20 mL of 2 M ammonia solution (2 M in MeOH) was additionally added. The mixture was stirred at 70°C for more than 12 hours. The progress of the reaction was confirmed using TLC. Upon completion of the reaction, the temperature of the reactant was lowered to room temperature. The mixture was concentrated under reduced pressure. The concentrated mixture was extracted using water and ethyl acetate. The organic layer was washed with brine, treated with sodium sulfate, and the filtrate was concentrated. The concentrated mixture was purified by silica gel column chromatography using 10% ethyl acetate/hexane as a developing solvent to give a target compound as a solid (7.732 g, yield: 66%).

LCMS (m/z) : 280.20 (M+H).

### Step 2. Preparation of 3-nitro-5,6,7,8-tetrahydro-1,6-naphthyridine

While stirring a solution of tert-butyl 3-nitro-7,8-dihydro-1,6-naphthyridine-6(5H)-carboxylate (1.0 g, 3.58 mmol, 1.0 eq) mixed in 10 mL of methanol solution, a dioxane solution (4.47 ml, 17.90 mmol, 5.0 eq) containing 4 M HCl was slowly added thereto at 0°C. The mixture was stirred at room temperature for 4 hours. The progress of the reaction was confirmed using TLC. Upon completion of the reaction, the mixture was concentrated and alkalized using 2 M ammonia solution (2 M in MeOH). The alkalized mixture was concentrated under reduced pressure and extracted with water and ethyl acetate. The organic layer was washed with brine, treated with sodium sulfate, and the filtrate was concentrated to give a target compound as a solid (0.61 g, yield: 95%).

LCMS (m/z) : 180.1 (M+H).

### Step 3. Preparation of ethyl-3-nitro-7,8-dihydro-1,6-naphthylpyridine-6(5H)-carboxylate

Ethyl chloroformate (227 mg, 2.095 mmol, 1.2 eq) was slowly added to a solution of 3-nitro-5,6,7,8-tetrahydro-1,6-naphthyridine (313 mg, 1.746 mmol, 1.0 eq) and triethylamine (212 mg, 2.095 mmol, 1.2 eq) mixed in 6 mL of dichloromethane solution at 0°C while stirring. The mixture was stirred at room temperature for 1 hour. The progress of the reaction was confirmed using TLC. The mixture was extracted using water and ethyl acetate. The organic layer was washed with brine, treated with sodium sulfate, and the filtrate was concentrated. The concentrated mixture was purified by silica gel column chromatography using 20% ethyl acetate/hexane as a developing solvent to give a target compound as a solid (7.732 g, yield: 66%).

LCMS (m/z) : 252.2 (M+H).

### Step 4. Preparation of ethyl-3-amino-7,8-dihydro-1,6-naphthylpyridine-6(5H)-carboxylate

4,4'-Bipyridine (23 mg, 0.150 mmol, 0.2 eq) was slowly added to a solution of ethyl-3-nitro-7,8-dihydro-1,6-naphthylpyridine-6(5H)-carboxylate (188 mg, 0.748 mmol, 1.0 eq) and tetrahydroxydiboron (201 mg, 2.245 mmol, 3.0 eq) in 2 mL of dimethylformamide solution at 0°C while stirring. The mixture was additionally stirred for 30 minutes, then warmed to room temperature and stirred for more than 1 hour. The progress of the reaction was confirmed using TLC. The mixture was extracted using water and ethyl acetate. The organic layer was washed with brine, treated with sodium sulfate, and the filtrate was concentrated. The concentrated mixture was purified by silica gel column chromatography using 5% methanol/dichloromethane as a developing solvent to give a target compound as a solid (135 mg, yield: 81%).

LCMS (m/z) : 222.2 (M+H).

### Step 5. Preparation of ethyl3-((5-fluoro-2-methoxyphenyl)sulfonamido)-7,8-dihydro-1,6-naphthyridine-6(5H)-carboxylate

Triethylamine (0.03 mL, 0.249 mmol, 2.0 eq) was added to a solution of 5-fluoro-2-methoxybenzenesulfonyl chloride (30 mg, 0.134 mmol, 1.0 eq) and ethyl-3-amino-7,8-dihydro-1,6-naphthylpyridine-6(5H)-carboxylate (32 mg, 0.147 mmol, 1.1 eq) mixed in 2 mL of dichloromethane at 0°C. The mixture was stirred at room temperature for 12 hours. The progress of the reaction was confirmed using TLC. Upon completion of the reaction, the mixture was concentrated. The concentrated mixture was purified by silica gel column chromatography using methanol/dichloromethane as a developing solvent to give a target compound as a solid (19 mg, yield: 35%).

¹H-NMR (400 MHz, Chloroform-d) δ 7.97 (s, 1H), 7.49 (dd, J = 7.5, 3.3 Hz, 1H), 7.35 (s, 1H), 7.25 (d, J = 1.4 Hz, 1H), 7.23 (d, J = 7.3 Hz, 2H), 7.03 - 6.93 (m, 2H), 4.58 (d, J = 3.1 Hz, 2H), 4.16 (tq, J = 7.0, 3.3, 2.8 Hz, 2H), 4.07 - 3.99 (m, 3H), 3.73 (s, 2H), 2.89 (s, 2H), 1.27 (tt, J = 7.0, 2.7 Hz, 3H).

LCMS (m/z) : 410.0 (M+H).

### Example 96. Preparation of ethyl3-((5-chloro-2-methoxyphenyl)sulfonamido)-7,8-dihydro-1,6-naphthyridine-6(5H)-carboxylate

A target compound (21 mg, yield: 39%) was obtained by reacting 5-chloro-2-methoxybenzenesulfonyl chloride and ethyl-3-amino-7,8-dihydro-1,6-naphthylpyridine-6(5H)-carboxylate according to the manufacturing method of Example 1.

¹H-NMR (400 MHz, Chloroform-d) δ 7.96 (s, 1H), 7.73 (t, J = 2.8 Hz, 1H), 7.45 (dt, J = 8.9, 2.4 Hz, 1H), 7.35 (s, 1H), 7.23 (s, 1H), 7.01 - 6.89 (m, 2H), 4.63 - 4.55 (m, 2H), 4.20 - 4.12 (m, 2H), 4.04 (d, J = 2.1 Hz, 3H), 3.73 (s, 2H), 2.89 (s, 2H), 1.26 (dt, J = 7.1, 3.8 Hz, 3H).

LCMS (m/z) : 426.0 (M+H).

### Example 97. Preparation of ethyl3-((5-bromo-2-methoxyphenyl)sulfonamido)-7,8-dihydro-1,6-naphthyridine-6(5H)-carboxylate

A target compound (27 mg, yield: 54%) was obtained by reacting 5-bromo-2-methoxybenzenesulfonyl chloride and ethyl-3-amino-7,8-dihydro-1,6-naphthylpyridine-6(5H)-carboxylate according to the manufacturing method of Example 1.

¹H-NMR (400 MHz, Chloroform-d) δ 7.96 (s, 1H), 7.90 - 7.83 (m, 1H), 7.60 (dd, J = 8.8, 2.6 Hz, 1H), 7.34 (s, 1H), 7.24 (s, 4H), 6.92 (d, J = 9.0 Hz, 2H), 4.59 (s, 2H), 4.17 (q, J = 7.2 Hz, 2H), 4.06 - 4.02 (m, 3H), 3.74 (s, 2H), 2.90 (t, J = 5.8 Hz, 2H), 1.28 (t, J = 7.1 Hz, 3H).

LCMS (m/z) : 470.0 (M+H).

### Example 98. Preparation of neopentyl3-((5-chloro-2-methoxyphenyl)sulfonamido)-7,8-dihydro-1,6-naphthyridine-6(5H)-carboxylate

Triethylamine (0.03 mL, 0.249 mmol, 2.0 eq) was added to a solution of 5-chloro-2-methoxybenzenesulfonyl chloride (30 mg, 0.134 mmol, 1.0 eq) and neopentyl-3-amino-7,8-dihydro-1,6-naphthylpyridine-6(5H)-carboxylate (39 mg, 0.147 mmol, 1.1 eq) mixed in 2 mL of dichloromethane at 0°C. The mixture was stirred at room temperature for 12 hours. The progress of the reaction was confirmed using TLC. Upon completion of the reaction, the mixture was concentrated. The concentrated mixture was purified by silica gel column chromatography using methanol/dichloromethane as a developing solvent to give a target compound as a solid (42 mg, yield: 72%).

¹H-NMR (400 MHz, DMSO-d6) δ 10.28 (s, 1H), 8.05 (d, J = 2.7 Hz, 1H), 7.66 (t, J = 2.1 Hz, 1H), 7.61 (dt, J = 8.7, 2.0 Hz, 1H), 7.29 (s, 1H), 7.20 (dt, J = 8.9, 1.7 Hz, 1H), 4.48 (s, 2H), 3.85 - 3.81 (m, 3H), 3.68 (t, J = 1.7 Hz, 2H), 3.62 (s, 2H), 2.74 (s, 2H), 0.89 - 0.83 (m, 9H).

LCMS (m/z) : 468.1 (M+H).

### Example 99. Preparation of neopentyl3-((5-bromo-2-methoxyphenyl)sulfonamido)-7,8-dihydro-1,6-naphthyridine-6(5H)-carboxylate

A target compound (26 mg, yield: 58%) was obtained by reacting 5-bromo-2-methoxybenzenesulfonyl chloride and neopentyl-3-amino-7,8-dihydro-1,6-naphthylpyridine-6(5H)-carboxylate according to the manufacturing method of Example 1.

¹H-NMR (400 MHz, DMSO-d6) δ 10.27 (s, 1H), 8.04 (t, J = 3.7 Hz, 1H), 7.78 - 7.69 (m, 2H), 7.28 (s, 1H), 7.13 (dd, J = 8.9, 5.5 Hz, 1H), 4.47 (s, 2H), 3.84 - 3.79 (m, 3H), 3.70 - 3.66 (m, 2H), 3.62 (s, 2H), 2.73 (d, J = 6.3 Hz, 2H), 0.86 (d, J = 5.2 Hz, 9H).

LCMS (m/z) : 512.0 (M+H).

### Example 100. Preparation of neopentyl3-((5-methoxy-2-methoxyphenyl)sulfonamido)-7,8-dihydro-1,6-naphthyridine-6(5H)-carboxylate

A target compound (43 mg, yield: 87%) was obtained by reacting 5-methoxy-2-methoxybenzenesulfonyl chloride and neopentyl-3-amino-7,8-dihydro-1,6-naphthylpyridine-6(5H)-carboxylate according to the manufacturing method of Example 1.

¹H-NMR (400 MHz, DMSO-d6) δ 10.14 (s, 1H), 8.05 (s, 1H), 7.27 (s, 1H), 7.22 (d, J = 2.9 Hz, 1H), 7.10 (d, J = 5.3 Hz, 2H), 4.45 (s, 2H), 3.79 - 3.72 (m, 3H), 3.70 - 3.55 (m, 7H), 2.71 (s, 2H), 0.85 (d, J = 9.2 Hz, 9H).

LCMS (m/z) : 464.1 (M+H).

### Example 101. Preparation of 2-fluoroethyl-3-((5-chloro-2-methoxyphenyl)sulfonamido)-7,8-dihydro-1,6-naphthyridine-6(5H)-carboxylate

A target compound (37 mg, yield: 67%) was obtained by reacting 5-chloro-2-methoxybenzenesulfonyl chloride and 2-fluoroethyl-3-amino-7,8-dihydro-1,6-naphthylpyridine-6(5H)-carboxylate according to the manufacturing method of Example 1.

¹H-NMR (400 MHz, DMSO-d6) δ 10.29 (s, 1H), 8.06 (t, J = 2.8 Hz, 1H), 7.66 (t, J = 2.9 Hz, 1H), 7.61 (dt, J = 9.0, 2.9 Hz, 1H), 7.28 (t, J = 2.8 Hz, 1H), 7.22 - 7.17 (m, 1H), 4.68 - 4.61 (m, 1H), 4.51 (t, J = 4.0 Hz, 3H), 4.30 - 4.25 (m, 1H), 4.22 - 4.17 (m, 1H), 3.83 (d, J = 2.7 Hz, 3H), 3.62 (s, 2H), 2.74 (d, J = 6.3 Hz, 2H).

LCMS (m/z) : 444.0 (M+H).

### Example 102. Preparation of 2-fluoroethyl3-((5-bromo-2-methoxyphenyl)sulfonamido)-7,8-dihydro-1,6-naphthyridine-6(5H)-carboxylate

A target compound (36 mg, yield: 71%) was obtained by reacting 5-bromo-2-methoxybenzenesulfonyl chloride and 2-fluoro-3-amino-7,8-dihydro-1,6-naphthylpyridine-6(5H)-carboxylate according to the manufacturing method of Example 1.

¹H-NMR (400 MHz, DMSO-d6) δ 10.28 (s, 1H), 8.08 - 8.01 (m, 1H), 7.78 - 7.69 (m, 2H), 7.27 (d, J = 7.7 Hz, 1H), 7.13 (t, J = 8.7 Hz, 1H), 4.62 (d, J = 9.5 Hz, 1H), 4.50 (d, J = 9.3 Hz, 3H), 4.26 (d, J = 9.3 Hz, 1H), 4.19 (q, J = 5.6, 3.8 Hz, 1H), 3.81 (dd, J = 8.9, 3.9 Hz, 3H), 3.61 (s, 2H), 2.73 (s, 2H).

LCMS (m/z) : 487.9 (M+H).

### Example 103. Preparation of 2-fluoroethyl-3-((5-methoxy-2-methoxyphenyl)sulfonamido)-7,8-dihydro-1,6-naphthyridine-6(5H)-carboxylate

A target compound (22 mg, yield: 44%) was obtained by reacting 5-methoxy-2-methoxybenzenesulfonyl chloride and 2-fluoroethyl-3-amino-7,8-dihydro-1,6-naphthylpyridine-6(5H)-carboxylate according to the manufacturing method of Example 1.

¹H-NMR (400 MHz, DMSO-d6) δ 10.15 (s, 1H), 8.09 - 8.01 (m, 1H), 7.26 (d, J = 6.0 Hz, 1H), 7.23 - 7.18 (m, 1H), 7.14 - 7.05 (m, 2H), 4.62 (d, J = 7.8 Hz, 1H), 4.48 (d, J = 17.6 Hz, 3H), 4.26 (t, J = 7.9 Hz, 1H), 4.19 (dt, J = 7.8, 4.3 Hz, 1H), 3.79 - 3.72 (m, 3H), 3.71 - 3.65 (m, 3H), 3.61 (s, 2H), 2.72 (d, J = 6.5 Hz, 2H) .

LCMS (m/z) : 440.0 (M+H).

### Example 104. Preparation of 3-((5-chloro-2-methoxyphenyl)sulfonamido)-N-isopropyl-7,8-dihydro-1,6-naphthyridine-6(5H)-carboxamide

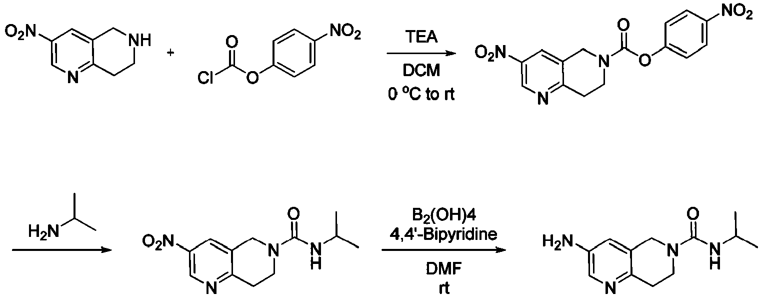

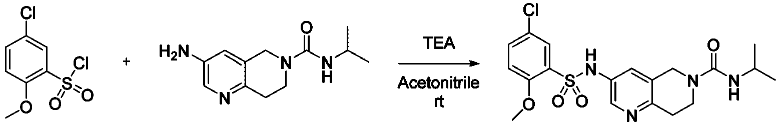

### Step 1. Preparation of N-isopropyl-3-nitro-7,8-dihydro-1,6-naphthyridine-6(5H)-carboxamide

4-Nitrophenyl carbonochloridate (216 mg, 1.07 mmol, 1.5 eq) was slowly added to a solution of 3-nitro-5,6,7,8-tetrahydro-1,6-naphthyridine (130 mg, 0.716 mmol, 1.0 eq) and triethylamine (0.20 mL, 1.43 mmol, 2.0 eq) mixed in 2 mL of dichloromethane solution at 0°C while stirring. The mixture was stirred at room temperature for 1 hour. The progress of the reaction was monitored using TLC to determine whether the starting material, 3-nitro-5,6,7,8-tetrahydro-1,6-naphthyridine, was consumed. Isopropylamine (0.19 mL, 2.15 mmol, 3.0 eq) was slowly added to the mixture at 0°C. The mixture was stirred at room temperature for 2 hours. The progress of the reaction was confirmed using TLC. Upon completion of the reaction, the mixture was extracted using water and ethyl acetate. The organic layer was washed with brine, treated with sodium sulfate, and the filtrate was concentrated. The concentrated mixture was purified by silica gel column chromatography using 70% ethyl acetate/hexane as a developing solvent to give a target compound as a solid (166 mg, yield: 87%).

LCMS (m/z) : 265.1 (M+H).

### Step 2. Preparation of 3-amino-N-isopropyl-3-nitro-7,8-dihydro-1,6-naphthyridine-6(5H)-carboxamide

A target compound (89 mg, yield: 61%) was obtained by reacting N-isopropyl-3-nitro-7,8-dihydro-1,6-naphthyridine-6(5H)-carboxamide according to the manufacturing method of step 2 of Intermediate 4.

LCMS (m/z) : 235.1 (M+H).

### Step 3. Preparation of 3-((5-chloro-2-methoxyphenyl)sulfonamido)-N-isopropyl-7,8-dihydro-1,6-naphthyridine-6(5H)-carboxamide

A target compound (22 mg, yield: 40%) was obtained by reacting 5-chloro-2-methoxybenzenesulfonyl chloride and 3-amino-N-isopropyl-3-nitro-7,8-dihydro-1,6-naphthyridine-6(5H)-carboxamide according to the manufacturing method of Example 1.

¹H-NMR (400 MHz, DMSO-d6) δ 10.29 (s, 1H), 8.04 (d, J = 2.7 Hz, 1H), 7.69 - 7.59 (m, 2H), 7.19 (d, J = 8.8 Hz, 2H), 6.25 (d, J = 7.4 Hz, 1H), 4.37 (s, 2H), 3.83 (d, J = 1.5 Hz, 3H), 3.71 (q, J = 6.5 Hz, 1H), 3.54 (d, J = 6.0 Hz, 2H), 3.13 (dd, J = 5.4, 1.9 Hz, 1H), 2.67 (d, J = 5.9 Hz, 2H), 1.01 (dd, J = 6.6, 1.7 Hz, 6H).

LCMS (m/z) : 439.0 (M+H).

### Example 105. Preparation of 3-((5-fluoro-2-methoxyphenyl)sulfonamido)-N-isopropyl-7,8-dihydro-1,6-naphthyridine-6(5H)-carboxamide

A target compound (38 mg, yield: 66%) was obtained by reacting 5-fluoro-2-methoxybenzenesulfonyl chloride and 3-amino-N-isopropyl-3-nitro-7,8-dihydro-1,6-naphthyridine-6(5H)-carboxamide according to the manufacturing method of Example 1.

¹H-NMR (400 MHz, DMSO-d6) δ 10.27 (s, 1H), 8.04 (q, J = 2.6 Hz, 1H), 7.50 (tt, J = 5.1, 3.0 Hz, 1H), 7.44 (tt, J = 8.4, 2.9 Hz, 1H), 7.22 - 7.16 (m, 2H), 6.24 (d, J = 7.4 Hz, 1H), 4.36 (d, J = 3.7 Hz, 2H), 3.84 - 3.78 (m, 3H), 3.70 (dt, J = 12.2, 6.0 Hz, 1H), 3.53 (dd, J = 6.1, 4.1 Hz, 2H), 2.66 (t, J = 5.5 Hz, 2H), 1.04 - 0.97 (m, 6H).

LCMS (m/z) : 423.1 (M+H).

### Example 106. Preparation of 3-((5-chloro-2-methoxyphenyl)sulfonamido)-N-pentyl-7,8-dihydro-1,6-naphthyridine-6(5H)-carboxamide

A target compound (27 mg, yield: 47%) was obtained by reacting 5-chloro-2-methoxybenzenesulfonyl chloride and 3-amino-N-pentyl-7,8-dihydro-1,6-naphthyridine-6(5H)-carboxamide according to the manufacturing method of Example 1.

¹H-NMR (400 MHz, DMSO-d6) δ 10.29 (s, 1H), 8.03 (d, J = 2.5 Hz, 1H), 7.67 - 7.58 (m, 2H), 7.23 - 7.16 (m, 2H), 6.54 (d, J = 5.7 Hz, 1H), 4.37 (s, 2H), 3.82 (s, 3H), 3.53 (t, J = 5.6 Hz, 2H), 2.96 (q, J = 6.5 Hz, 2H), 2.67 (t, J = 5.9 Hz, 2H), 1.35 (q, J = 7.3 Hz, 2H), 1.19 (dt, J = 13.6, 8.5 Hz, 5H), 0.80 (t, J = 7.0 Hz, 3H)

LCMS (m/z) : 467.1 (M+H).

### Example 107. Preparation of 3-((5-chloro-2-methoxyphenyl)sulfonamido)-N-(2,2-difluoroethyl)-7,8-dihydro-1,6-naphthyridine-6(5H)-carboxamide

A target compound (9 mg, yield: 21%) was obtained by reacting 5-fluoro-2-methoxybenzenesulfonyl chloride and 3-amino-N-(2,2-difluoroethyl)-3-nitro-7,8-dihydro-1,6-naphthyridine-6(5H)-carboxamide according to the manufacturing method of Example 1.

¹H-NMR (400 MHz, DMSO-d6) δ 10.29 (s, 1H), 8.05 (s, 1H), 7.65 (dd, J = 2.7, 1.5 Hz, 1H), 7.61 (dt, J = 8.7, 2.0 Hz, 1H), 7.22 - 7.17 (m, 2H), 7.02 (s, 1H), 6.08 - 5.75 (m, 1H), 4.42 (s, 2H), 3.85 - 3.81 (m, 3H), 3.57 (t, J = 5.6 Hz, 2H), 3.42 - 3.31 (m, 3H), 2.69 (t, J = 5.8 Hz, 2H).

LCMS (m/z) : 461.0 (M+H).

### Example 108. Preparation of 3-((5-bromo-2-methoxyphenyl)sulfonamido)-N-(2,2-difluoroethyl)-7,8-dihydro-1,6-naphthyridine-6(5H)-carboxamide

A target compound (30 mg, yield: 68%) was obtained by reacting 5-fluoro-2-methoxybenzenesulfonyl chloride and 3-amino-N-(2,2-difluoroethyl)-3-nitro-7,8-dihydro-1,6-naphthyridine-6(5H)-carboxamide according to the manufacturing method of Example 1.

¹H-NMR (400 MHz, DMSO-d6) δ 10.28 (s, 1H), 8.05 (t, J = 2.9 Hz, 1H), 7.78 - 7.70 (m, 2H), 7.21 (t, J = 2.9 Hz, 1H), 7.14 (dd, J = 8.8, 3.0 Hz, 1H), 7.02 (d, J = 5.1 Hz, 1H), 5.91 (td, J = 56.3, 4.0 Hz, 1H), 4.42 (s, 2H), 3.82 (d, J = 2.9 Hz, 3H), 3.57 (t, J = 6.0 Hz, 2H), 3.43 - 3.30 (m, 3H), 2.69 (t, J = 5.7 Hz, 2H).

LCMS (m/z) : 505.0 (M+H).

### Example 109. Preparation of 5-chloro-2-methoxy-N-(6-(morpholine-4-carbonyl)-5,6,7,8-tetrahydro-1,6-naphthyridine-3-yl)benzenesulfonamide

A target compound (14 mg, yield: 33%) was obtained by reacting 5-chloro-2-methoxybenzenesulfonyl chloride and (3-amino-7,8-dihydro-1,6-naphthyridine-6(5H)-yl)(morpholino)methanone according to the manufacturing method of Example 1.

¹H-NMR (400 MHz, DMSO-d6) δ 10.27 (s, 1H), 8.04 (t, J = 3.8 Hz, 1H), 7.70 - 7.64 (m, 1H), 7.61 (dd, J = 8.8, 2.9 Hz, 1H), 7.26 (d, J = 2.6 Hz, 1H), 7.20 (t, J = 7.1 Hz, 1H), 4.26 (d, J = 5.1 Hz, 2H), 3.88 - 3.81 (m, 3H), 3.53 (q, J = 4.8 Hz, 4H), 3.39 (t, J = 6.0 Hz, 2H), 3.12 (q, J = 4.9 Hz, 4H), 2.79 - 2.72 (m, 2H).

LCMS (m/z) : 467.0 (M+H).

### Example 110. Preparation of 5-bromo-2-methoxy-N-(6-(morpholine-4-carbonyl)-5,6,7,8-tetrahydro-1,6-naphthyridine-3-yl)benzenesulfonamide

A target compound (25 mg, yield: 53%) was obtained by reacting 5-bromo-2-methoxybenzenesulfonyl chloride and (3-amino-7,8-dihydro-1,6-naphthyridine-6(5H)-yl)(morpholino)methanone according to the manufacturing method of Example 1.

¹H-NMR (400 MHz, DMSO-d6) δ 10.26 (s, 1H), 8.04 (t, J = 2.8 Hz, 1H), 7.74 (ddt, J = 14.3, 5.6, 3.0 Hz, 2H), 7.26 (d, J = 2.9 Hz, 1H), 7.17 - 7.12 (m, 1H), 4.26 (d, J = 5.6 Hz, 2H), 3.83 (d, J = 2.7 Hz, 3H), 3.53 (q, J = 4.9, 3.8 Hz, 4H), 3.40 (d, J = 6.0 Hz, 2H), 3.14 - 3.10 (m, 4H), 2.76 (d, J = 6.2 Hz, 2H).

LCMS (m/z) : 511.0 (M+H).

### Example 111. Preparation of 3-((5-bromo-2-methoxyphenyl)sulfonamido)-N-methyl-7,8-dihydro-1,6-naphthyridine-6(5H)-carboxamide

### Step 1. Preparation of 3-N-methyl-3-nitro-7,8-dihydro-1,6-naphthyridine-6(5H)-carboxamide

Using methamine as an amine substituent, the reaction was performed according to the manufacturing method of step 2 of Example 104, and the mixture was used in the next step without purification.

### Step 2. Preparation of 3-N-methyl-3-amino-7,8-dihydro-1,6-naphthyridine-6(5H)-carboxamide

A target compound (10 mg, yield: 12%) was obtained by reacting 3-N-methyl-3-nitro-7,8-dihydro-1,6-naphthyridine-6(5H)-carboxamide according to the manufacturing method of step 2 of Intermediate 4.

### Step 3. Preparation of 3-((5-bromo-2-methoxyphenyl)sulfonamido)-N-methyl-7,8-dihydro-1,6-naphthyridine-6(5H)-carboxamide

A target compound (3 mg, yield: 13%) was obtained by reacting 5-bromo-2-methoxybenzenesulfonyl chloride and -N-methyl-3-amino-7,8-dihydro-1,6-naphthyridine-6(5H)-carboxamide according to the manufacturing method of Example 1.

¹H-NMR (400 MHz, DMSO-d6) δ 10.31 (s, 1H), 8.05 (d, J = 2.5 Hz, 1H), 7.68 - 7.54 (m, 2H), 7.20 - 7.16 (m, 2H), 4.35 (s, 2H), 3.82 (s, 3H), 3.53 (t, J = 5.6 Hz, 2H), 2.96 (q, J = 6.5 Hz, 2H), 2.67 (s, 3H).

LCMS (m/z) : 456.0 (M+H).

### Example 112. Preparation of 5-chloro-N-(6-isopropyl-5,6,7,8-tetrahydro-1,6-naphthyridine-3-yl)-2-methoxybenzenesulfonamide

### Step 1. Preparation of 6-isopropyl-3-nitro-5,6,7,8-tetrahydro-1,6-naphthyridine

3-Nitro-5,6,7,8-tetrahydro-1,6-naphthyridine (300 mg, 1.0 eq) was dissolved in 10 ml of tetrahydrofuran. Acetone (0.17 ml, 2.0 eq), DIPEA (1.0 ml, 5 eq) and triacetoxysodiumborohydride (504 mg, 2.0 eq) were added to the above mixture and stirred at room temperature for 3 hours. Upon completion of the reaction, the mixture was extracted with sodium bicarbonate aqueous solution and ethyl acetate. The obtained organic solvent layer was dried over sodium sulfate, filtered, and concentrated. The concentrate was purified by column chromatography to give a target compound (50 mg, yield: 20%).

### Step 2. Preparation of 6-isopropyl-3-amino-5,6,7,8-tetrahydro-1,6-naphthyridine

A target compound (40 mg, yield: 92%) was obtained by reacting 6-isopropyl-3-nitro-5,6,7,8-tetrahydro-1,6-naphthyridine according to the manufacturing method of step 2 of Intermediate 4.

### Step 3. Preparation of 5-chloro-N-(6-isopropyl-5,6,7,8-tetrahydro-1,6-naphthyridine-3-yl)-2-methoxybenzenesulfonamide

A target compound (5 mg, yield: 12%) was obtained by reacting 5-chloro-2-methoxybenzenesulfonyl chloride and 6-isopropyl-3-amino-5,6,7,8-tetrahydro-1,6-naphthyridine according to the manufacturing method of Example 1.

¹H-NMR (400 MHz, DMSO-d6) δ 10.31 (s, 1H), 8.0 (t, J = 3.8 Hz, 1H), 7.70 - 7.64 (m, 1H), 7.61 (dd, J = 8.8, 2.9 Hz, 1H), 7.26 (d, J = 2.6 Hz, 1H), 7.20 (t, J = 7.1 Hz, 1H), 3.91 (s, 3H), 3.79 (s, 2H), 3.09 (t, J = 7.2 Hz, 2H), 3.02 - 2.85 (m, 3H), 1.08 (d, J = 6.8 Hz, 6H).

LCMS (m/z) : 396.0 (M+H).

### Example 113. Preparation of 5-bromo-N-(6-isopropyl-5,6,7,8-tetrahydro-1,6-naphthyridine-3-yl)-2-methoxybenzenesulfonamide

A target compound (3 mg, yield: 6%) was obtained by reacting 5-bromo-2-methoxybenzenesulfonyl chloride and 6-isopropyl-3-amino-5,6,7,8-tetrahydro-1,6-naphthyridine according to the manufacturing method of Example 1.

¹H-NMR (400 MHz, DMSO-d6) δ 10.43 (s, 1H), 9.24 (s, 2H), 8.18 (d, J = 2.5 Hz, 1H), 7.80 - 7.64 (m, 2H), 7.29 (d, J = 2.5 Hz, 1H), 7.18 (d, J = 8.9 Hz, 1H), 3.91 (s, 3H), 3.65 (s, 2H), 3.15 (t, J = 7.2 Hz, 2H), 3.18 - 2.75 (m, 3H), 1.12 (d, J = 6.2 Hz, 6H).

LCMS (m/z) : 441.0 (M+H).

### Example 114. Preparation of 3-5-chloro-2-methoxy-N-(6-(pyrimidine-2-yl)-5,6,7,8-tetrahydro-1,6-naphthyridine-3-yl)benzenesulfonamide

### Step 1. Preparation of 3-nitro-6-(pyrimidine-2-yl)-5,6,7,8-tetrahydro-1,6-naphthyridine

3-Nitro-5,6,7,8-tetrahydro-1,6-naphthyridine (200 mg, 1.0 eq), 2-bromopyrimidine (138 mg. 1.1 eq), sodium tert-butoxide (152 mg, 2.0 eq), BINAP (24 mg, 0.05 eq), and Pd₂(dba)₃ (15 mg, 0.02 eq) were added to 3 ml of toluene. After replacement with nitrogen, the mixture was stirred at 100°C for 12 hours. After confirming with TLC, filtering was performed with a celite pad. The obtained solution was concentrated and purified by column chromatography to give a target compound (110 mg, yield: 53%).

### Step 2. Preparation of 3-amino-6-(pyrimidine-2-yl)-5,6,7,8-tetrahydro-1,6-naphthyridine

A target compound (40 mg, yield: 85%) was obtained by reacting 3-nitro-6-(pyrimidine-2-yl)-5,6,7,8-tetrahydro-1,6-naphthyridine according to the manufacturing method of step 2 of Intermediate 4.

### Step 3. Preparation of 3-5-chloro-2-methoxy-N-(6-(pyrimidine-2-yl)-5,6,7,8-tetrahydro-1,6-naphthyridine-3-yl)benzenesulfonamide

A target compound (5 mg, yield: 6%) was obtained by reacting 5-chloro-2-methoxybenzenesulfonyl chloride and 6-(pyrimidine-2-yl)-5,6,7,8-tetrahydro-1,6-naphthyridine-3-amine according to the manufacturing method of Example 1.

¹H-NMR (400 MHz, Chloroform-d) δ 8.33 (s, 1H), 8.20 (d, J = 1.4 Hz, 1H), 7.93 (d, J = 1.4 Hz, 1H), 7.75 (d, J = 1.4 Hz, 1H), 7.35 (dd, J = 7.5, 1.5 Hz, 1H), 7.14 (d, J = 7.5 Hz, 1H), 6.50 (t, J = 7.5 Hz, 1H), 4.47 (s, 2H), 3.93 (s, 3H), 3.71 (t, J = 7.1 Hz, 2H), 2.96 (t, J = 7.1 Hz, 2H).

LCMS (m/z) : 432.0 (M+H).

### Example 115. Preparation of 3-((5-chloro-2-methoxyphenyl)sulfonamido)-N-phenyl-7,8-dihydro-1,6-naphthyridine-6(5H)-carboxamide

### Step 1. Preparation of 3-nitro-N-phenyl-7,8-dihydro-1,6-naphthyridine-6(5H)-carboxamide

A mixture synthesized by reacting with aniline as an amine substituent according to the manufacturing method of step 1 of Example 104 was extracted using water and ethyl acetate after the reaction was completed. The organic layer was washed with brine, treated with sodium sulfate, and the filtrate was concentrated. The concentrated mixture was purified by silica gel column chromatography using ethyl acetate/hexane as a developing solvent to give a target compound as a solid (61 mg, yield: 27%).

LCMS (m/z) : 299.3 (M+H).

### Step 2. Preparation of 3-amino-N-phenyl-7,8-dihydro-1,6-naphthyridine-6(5H)-carboxamide

A target compound (42 mg, yield: 76%) was obtained by reacting 3-nitro-N-phenyl-7,8-dihydro-1,6-naphthyridine-6(5H)-carboxamide according to the manufacturing method of step 2 of Intermediate 4.

LCMS (m/z) : 257.1 (M+H).

### Step 3. Preparation of 3-((5-chloro-2-methoxyphenyl)sulfonamido)-N-phenyl-7,8-dihydro-1,6-naphthyridine-6(5H)-carboxamide

A target compound (36 mg, yield: 60%) was obtained by reacting 5-fluoro-2-methoxybenzenesulfonyl chloride and 3-amino-N-phenyl-3-nitro-7,8-dihydro-1,6-naphthyridine-6(5H)-carboxamide according to the manufacturing method of Example 1.

¹H-NMR (400 MHz, DMSO-d6) δ 10.31 (d, J = 7.5 Hz, 1H), 8.56 (d, J = 7.5 Hz, 1H), 8.15 - 7.99 (m, 1H), 7.73 - 7.56 (m, 2H), 7.47 - 7.36 (m, 2H), 7.30 - 7.14 (m, 4H), 6.97 - 6.85 (m, 1H), 4.54 (d, J = 6.7 Hz, 2H), 3.92 - 3.80 (m, 3H), 3.69 (d, J = 6.3 Hz, 2H), 2.76 (d, J = 6.1 Hz, 2H).

LCMS (m/z) : 473.0 (M+H).

### Example 116. Preparation of 3-((5-chloro-2-methoxyphenyl)sulfonamido)-N-(cyclopropylmethyl)-7,8-dihydro-1,6-naphthyridine-6(5H)-carboxamide

### Step 1. Preparation of N-(cyclopropylmethyl)-3-nitro-7,8-dihydro-1,6-naphthyridine-6(5H)-carboxamide

A mixture synthesized by reacting with cyclopropylmethanamine hydrochloride as an amine substituent according to the manufacturing method of step 1 of Example 104 was extracted using water and ethyl acetate after the reaction was completed. The organic layer was washed with brine, treated with sodium sulfate, and the filtrate was concentrated. The concentrated mixture was purified by silica gel column chromatography using ethyl acetate/hexane as a developing solvent to give a target compound as a solid (72 mg, yield: 23%).

LCMS (m/z) : 277.1 (M+H).

### Step 2. Preparation of 3-amino-N-(cyclopropylmethyl)-7,8-dihydro-1,6-naphthyridine-6(5H)-carboxamide

A target compound (39 mg, yield: 61%) was obtained by reacting N-(cyclopropylmethyl)-3-nitro-7,8-dihydro-1,6-naphthyridine-6(5H)-carboxamide according to the manufacturing method of step 2 of Intermediate 4.

LCMS (m/z) : 247.1 (M+H).

### Step 3. Preparation of 3-((5-chloro-2-methoxyphenyl)sulfonamido)-N-(cyclopropylmethyl)-7,8-dihydro-1,6-naphthyridine-6(5H)-carboxamide

A target compound (36 mg, yield: 46%) was obtained by reacting 5-chloro-2-methoxybenzenesulfonyl chloride and 3-amino-N-(cyclopropylmethyl)-3-nitro-7,8-dihydro-1,6-naphthyridine-6(5H)-carboxamide according to the manufacturing method of Example 1.

¹H-NMR (400 MHz, DMSO-d6) δ 10.33 - 10.26 (m, 1H), 8.05 (dt, J = 5.9, 2.9 Hz, 1H), 7.63 (ddt, J = 14.1, 5.3, 3.2 Hz, 2H), 7.20 (t, J = 7.8 Hz, 2H), 6.65 (d, J = 5.6 Hz, 1H), 4.39 (t, J = 3.4 Hz, 2H), 3.83 (dd, J = 7.2, 3.3 Hz, 3H), 3.54 (dt, J = 8.3, 4.8 Hz, 2H), 2.85 (q, J = 6.8, 5.3 Hz, 2H), 2.67 (q, J = 5.9, 5.3 Hz, 2H), 0.86 (s, 1H), 0.30 (dtt, J = 8.2, 5.8, 3.5 Hz, 2H), 0.08 (qd, J = 6.5, 4.1 Hz, 2H).

LCMS (m/z) : 451.0 (M+H).

### Example 117. Preparation of 3-((5-chloro-2-methoxyphenyl)sulfonamido)-N-(2-fluoroethyl)-7,8-dihydro-1,6-naphthyridine-6(5H)-carboxamide

### Step 1. Preparation of N-(2-fluoroethyl)-3-nitro-7,8-dihydro-1,6-naphthyridine-6(5H)-carboxamide

A mixture synthesized by reacting with 2-fluoroethane-1-amine hydrochloride as an amine substituent according to the manufacturing method of step 1 of Example 104 was extracted using water and ethyl acetate after the reaction was completed. The organic layer was washed with brine, treated with sodium sulfate, and the filtrate was concentrated. The concentrated mixture was purified by silica gel column chromatography using ethyl acetate/hexane as a developing solvent to give a target compound as a solid (55 mg, yield: 18%).

LCMS (m/z) : 269.1 (M+H).

### Step 2. Preparation of 3-amino-N-(2-fluoroethyl)-7,8-dihydro-1,6-naphthyridine-6(5H)-carboxamide

A target compound (46 mg, yield: 94%) was obtained by reacting N-(2-fluoroethyl)-3-nitro-7,8-dihydro-1,6-naphthyridine-6(5H)-carboxamide according to the manufacturing method of step 2 of Intermediate 4.

LCMS (m/z) : 239.1 (M+H).

### Step 3. Preparation of 3-((5-chloro-2-methoxyphenyl)sulfonamido)-N-(2-fluoroethyl)-7,8-dihydro-1,6-naphthyridine-6(5H)-carboxamide

A target compound (23 mg, yield: 35%) was obtained by reacting 5-fluoro-2-methoxybenzenesulfonyl chloride and 3-amino-N-(2-fluoroethyl)-3-nitro-7,8-dihydro-1,6-naphthyridine-6(5H)-carboxamide according to the manufacturing method of Example 1.

¹H-NMR (400 MHz, DMSO-d6) δ 10.29 (s, 1H), 8.05 (d, J = 2.7 Hz, 1H), 7.66 - 7.60 (m, 2H), 7.20 (dd, J = 8.4, 2.3 Hz, 2H), 6.84 (d, J = 6.9 Hz, 1H), 4.44 - 4.37 (m, 3H), 4.32 - 4.27 (m, 1H), 3.85 - 3.81 (m, 3H), 3.56 (t, J = 5.0 Hz, 2H), 3.31 (d, J = 5.4 Hz, 1H), 3.27 - 3.22 (m, 1H), 2.69 (d, J = 7.9 Hz, 2H).

LCMS (m/z) : 443.0 (M+H).

### Example 118. Preparation of 5-chloro-N-(6-(3,3-dimethylbutanoyl)-5,6,7,8-tetrahydro-1,6-naphthyridine-3-yl)-2-methoxybenzenesulfonamide

### Step 1. Preparation of 3,3-dimethyl-1-(3-nitro-7,8-dihydro-1,6-naphthyridine-6(5H)-yl)butane-1-one

3-Nitro-5,6,7,8-tetrahydro-1,6-naphthyridine (100 mg, 1.0 eq) was dissolved in 2 ml of methylene chloride, to which 0.13 ml of triethylamine was added at 0°C. 3,3-Dimethylbutanoylchloride (65 mg, 1.05 eq) was slowly added to the mixed solution at the same temperature. The mixture was warmed to room temperature and stirred for 1 hour. The reaction was confirmed to be terminated by TLC, and the reactant was concentrated and used for the next reaction without further purification (140 mg).

LCMS (m/z) : 278.3 (M+H).

### Step 2. Preparation of 1-(3-amino-7,8-dihydro-1,6-naphthyridine-6(5H)-yl)-3,3-dimethylbutane-1-one

A target compound (61 mg, yield: 48%) was obtained by reacting 3,3-dimethyl-1-(3-nitro-7,8-dihydro-1,6-naphthyridine-6(5H)-yl)butane-1-one according to the manufacturing method of step 2 of Intermediate 4.

LCMS (m/z) : 248.3 (M+H).

### Step 3. Preparation of 5-chloro-N-(6-(3,3-dimethylbutanoyl)-5,6,7,8-tetrahydro-1,6-naphthyridine-3-yl)-2-methoxybenzenesulfonamide

A target compound (10 mg, yield: 18%) was obtained by reacting 5-chloro-2-methoxybenzenesulfonyl chloride and 1-(3-amino-7,8-dihydro-1,6-naphthyridine-6(5H)-yl)-3,3-dimethylbutane-1-one according to the manufacturing method of Example 1.

¹H-NMR (400 MHz, Methanol-d4) δ 8.07 (d, J = 17.3 Hz, 1H), 7.72 (s, 1H), 7.39 (s, 2H), 7.13 (d, J = 8.5 Hz, 1H), 4.74 - 4.63 (m, 2H), 3.97 - 3.79 (m, 5H), 2.85 (d, J = 33.0 Hz, 3H), 2.38 (s, 2H), 0.99 (dd, J = 43.6, 8.9 Hz, 9H).

LCMS (m/z) : 452.9 (M+H).

### Example 119. Preparation of 5-bromo-N-(6-(3,3-dimethylbutanoyl)-5,6,7,8-tetrahydro-1,6-naphthyridine-3-yl)-2-methoxybenzenesulfonamide

A target compound (15 mg, yield: 24%) was obtained by reacting 5-bromo-2-methoxybenzenesulfonyl chloride and 1-(3-amino-7,8-dihydro-1,6-naphthyridine-6(5H)-yl)-3,3-dimethylbutane-1-one according to the manufacturing method of Example 1.

¹H-NMR (400 MHz, Methanol-d4) δ 8.14 - 8.03 (m, 1H), 7.85 (s, 1H), 7.64 (d, J = 7.1 Hz, 1H), 7.44 (d, J = 34.8 Hz, 1H), 7.08 (t, J = 7.7 Hz, 1H), 4.68 (d, J = 22.5 Hz, 2H), 4.00 - 3.78 (m, 5H), 2.86 (dd, J = 33.2, 6.1 Hz, 3H), 2.39 (d, J = 5.1 Hz, 2H), 0.99 (dd, J = 42.0, 4.6 Hz, 9H).

LCMS (m/z) : 497.4 (M+H).

### Example 120. Preparation of 2-methoxy-N-(5-oxo-5,6,7,8-tetrahydro-1,6-naphthyridine-3-yl)-5-(1-(pyridine-4-yl)-1H-pyrazole-4-yl)benzenesulfonamide

A target compound (16 mg, yield: 46%) was obtained by reacting 4-(4-(4,4,5,5-tetramethyl-1,3,2-dioxaborolane-2-yl)-1H-pyrazole-1-yl)pyridine with the compound of Example 9 according to the manufacturing method of Example 27.

¹H-NMR (400 MHz, DMSO-d6) δ 10.39 (s, 1H), 9.21 (d, J = 3.8 Hz, 1H), 8.69 - 8.54 (m, 2H), 8.34 (dd, J = 4.0, 2.6 Hz, 1H), 8.30 (d, J = 3.9 Hz, 1H), 8.11 - 8.00 (m, 2H), 7.96 - 7.82 (m, 4H), 7.24 (dd, J = 8.8, 3.9 Hz, 1H), 3.87 (s, 3H), 3.37 - 3.30 (m, 2H), 2.90 - 2.79 (m, 2H).

LCMS (m/z) : 477.5 (M+H).

### Example 121. Preparation of 2-methoxy-N-(5-oxo-5,6,7,8-tetrahydro-1,6-naphthyridine-3-yl)-5-(1,3,5-trimethyl-1H-pyrazole-4-yl)benzenesulfonamide

A target compound (18 mg, yield: 52%) was obtained by reacting 1',3',5'-trimethyl-4-(4,4,5,5-tetramethyl-1,3,2-dioxaborolane-2-yl)-1'H-1,4'-bipyrazole with the compound of Example 9 according to the manufacturing method of Example 27.

¹H-NMR (400 MHz, DMSO-d6) δ 10.43 (s, 1H), 8.30 (d, J = 2.7 Hz, 1H), 8.07 (s, 1H), 7.87 (d, J = 2.7 Hz, 1H), 7.52 - 7.46 (m, 1H), 7.45 - 7.37 (m, 1H), 7.18 (d, J = 8.5 Hz, 1H), 3.84 (s, 3H), 3.65 (s, 3H), 3.38 - 3.31 (m, 2H), 2.86 (t, J = 6.5 Hz, 2H), 2.06 (s, 3H), 1.97 (s, 3H).

LCMS (m/z) : 442.5(M+H).

### Example 122. Preparation of 5-(1-(tert-butyl)-1H-pyrazole-4-yl)-2-methoxy-N-(5-oxo-5,6,7,8-tetrahydro-1,6-naphthyridine-3-yl)benzenesulfonamide

A target compound (12 mg, yield: 36%) was obtained by reacting 1-(tert-butyl)-4-(4,4,5,5-tetramethyl-1,3,2-dioxaborolane-2-yl)-1H-pyrazole with the compound of Example 9 according to the manufacturing method of Example 27.

¹H-NMR (400 MHz, DMSO-d6) δ 10.33 (s, 1H), 8.31 (s, 1H), 8.28-8.19 (m, 1H), 8.04 (s, 1H), 7.92-7.83 (m, 2H), 7.82-7.71 (m, 2H), 7.13 (d, J = 8.7 Hz, 1H), 3.83 (s, 3H), 3.36-3.30 (m, 2H), 2.93 - 2.78 (m, 2H), 1.50 (s, 9H).

LCMS (m/z) : 456.5(M+H).

### Example 123. Preparation of 5-(1-butyl-1H-pyrazole-4-yl)-2-methoxy-N-(5-oxo-5,6,7,8-tetrahydro-1,6-naphthyridine-3-yl)benzenesulfonamide

A target compound (18 mg, yield: 54%) was obtained by reacting 1-butyl-4-(4,4,5,5-tetramethyl-1,3,2-dioxaborolane-2-yl)-1H-pyrazole with the compound of Example 9 according to the manufacturing method of Example 27.

¹H-NMR (400 MHz, DMSO-d6) δ 10.33 (s, 1H), 8.30 (s, 1H), 8.14 (d, J = 5.5 Hz, 1H), 8.04 (s, 1H), 7.90 - 7.79 (m, 2H), 7.79 - 7.66 (m, 2H), 7.14 (dd, J = 8.8, 5.5 Hz, 1H), 4.04 (q, J = 6.6 Hz, 2H), 3.81 (s, 3H), 3.35-3.31 (m, 2H), 2.90 - 2.79 (m, 2H), 1.79 - 1.64 (m, 2H), 1.24 - 1.15 (m, 2H), 0.90 - 0.78 (m, 3H).

LCMS (m/z) : 456.5(M+H).

### Example 124. Preparation of 2-methoxy-N-(5-oxo-5,6,7,8-tetrahydro-1,6-naphthyridine-3-yl)-5-(1-phenyl-1H-pyrazole-4-yl)benzenesulfonamide

A target compound (21 mg, yield: 60%) was obtained by reacting 1-phenyl-4-(4,4,5,5-tetramethyl-1,3,2-dioxaborolane-2-yl)-1H-pyrazole with the compound of Example 9 according to the manufacturing method of Example 27.

¹H NMR (400 MHz, DMSO-d6) δ 10.37 (s, 1H), 9.00 (d, J = 4.2 Hz, 1H), 8.39 - 8.30 (m, 1H), 8.15 (d, J = 4.2 Hz, 1H), 8.09 - 7.99 (m, 2H), 7.93 - 7.80 (m, 4H), 7.54 - 7.41 (m, 2H), 7.34 - 7.18 (m, 2H), 3.86 (s, 3H), 3.37 - 3.30 (m, 2H), 2.91 - 2.79 (m, 2H).

LCMS (m/z) : 476.5(M+H).

The structural formulas of the compounds manufactured in Examples 1 to 124 are shown in Table 1 below.

**[Table 1]**

| | | | |
|---|---|---|---|
| 1 | 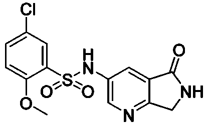 | 2 | 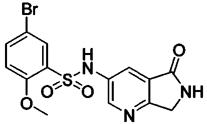 |
| 3 | 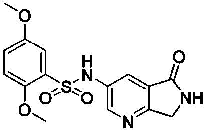 | 4 | 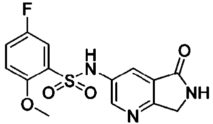 |
| 5 | 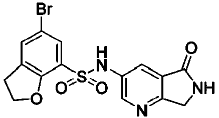 | 6 | 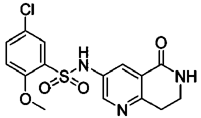 |
| 7 | 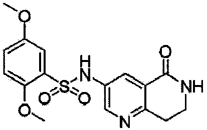 | 8 | 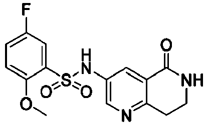 |
| 9 | 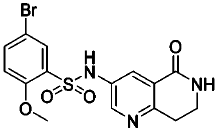 | 10 | 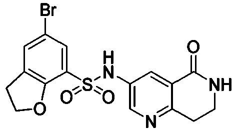 |
| 11 | 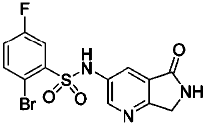 | 12 | 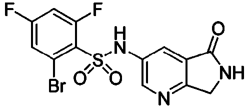 |
| 13 | 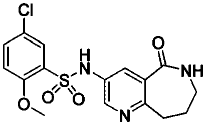 | 14 | 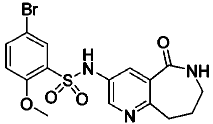 |
| 15 | 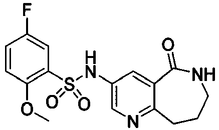 | 16 | 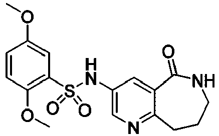 |
| 17 | 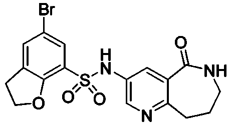 | 18 | 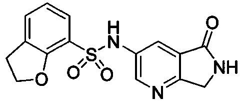 |
| 19 | 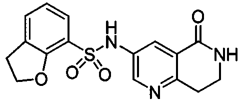 | 20 | 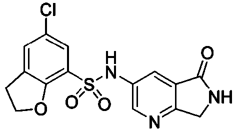 |
| 21 | 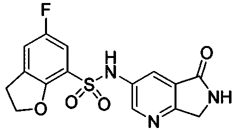 | 22 | 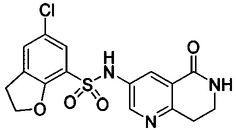 |
| 23 | 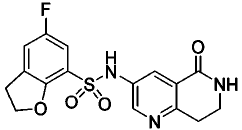 | 24 | 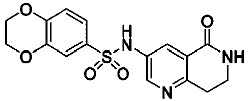 |
| 25 | 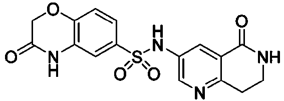 | 26 | 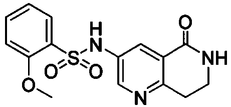 |
| 27 | 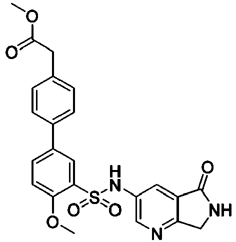 | 28 | 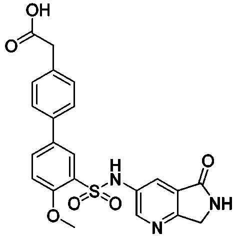 |
| 29 | 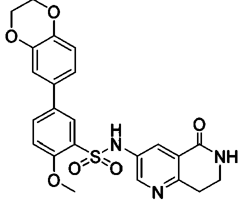 | 30 | 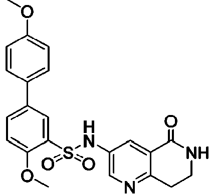 |
| 31 | 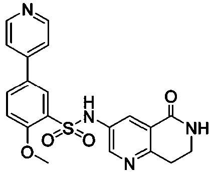 | 32 | 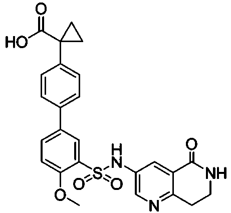 |
| 33 | 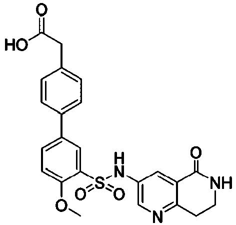 | 34 | 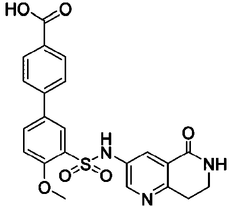 |
| 35 | 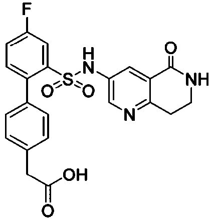 | 36 | 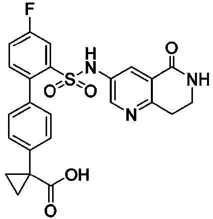 |
| 37 | 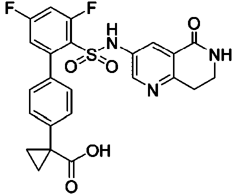 | 38 | 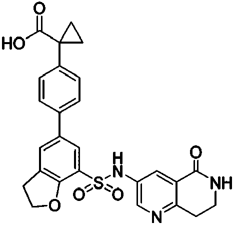 |
| 39 | 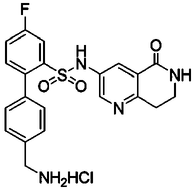 | 40 | 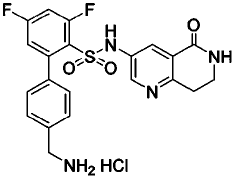 |
| 41 | 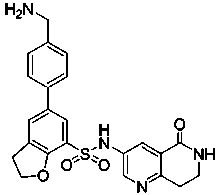 | 42 | 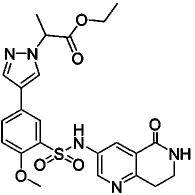 |
| 43 | 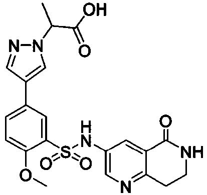 | 44 | 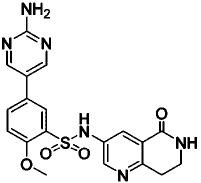 |
| 45 | 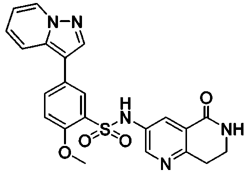 | 46 | 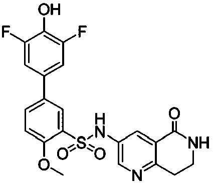 |
| 47 | 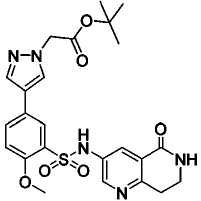 | 48 | 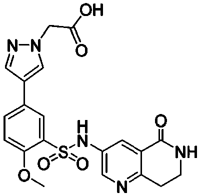 |
| 49 | 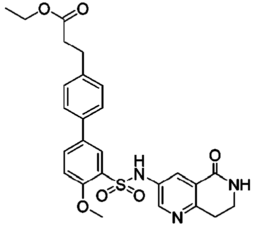 | 50 | 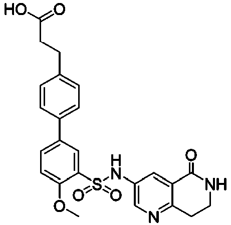 |
| 51 | 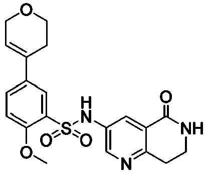 | 52 | 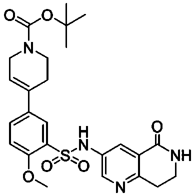 |
| 53 | 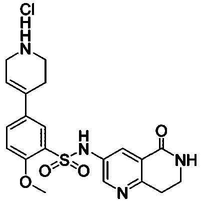 | 54 | 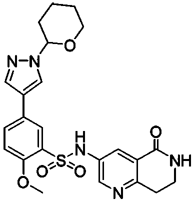 |
| 55 | 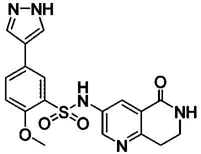 | 56 | 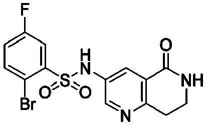 |
| 57 | 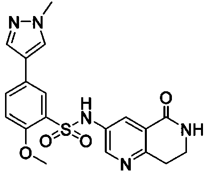 | 58 | 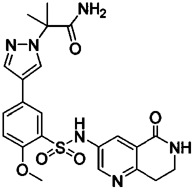 |
| 59 | 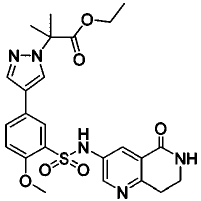 | 60 | 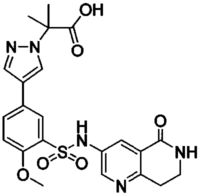 |
| 61 | 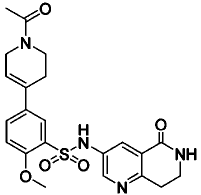 | 62 | 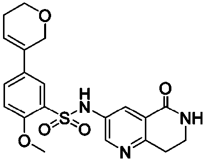 |
| 63 | 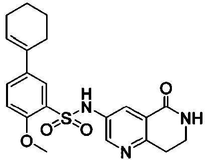 | 64 | 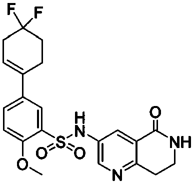 |
| 65 | 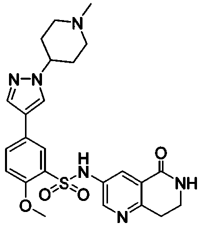 | 66 | 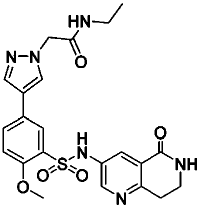 |
| 67 | 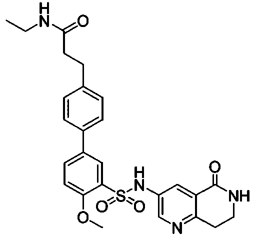 | 68 | 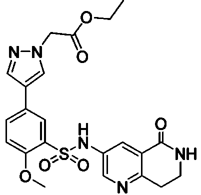 |
| 69 | 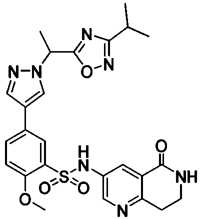 | 70 | 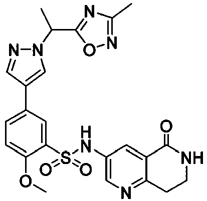 |
| 71 | 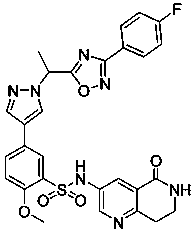 | 72 | 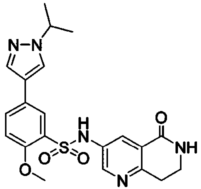 |
| 73 | 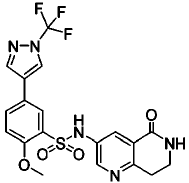 | 74 | 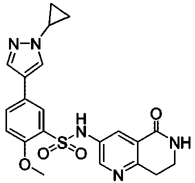 |
| 75 | 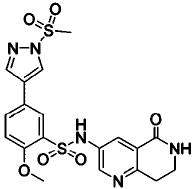 | 76 | 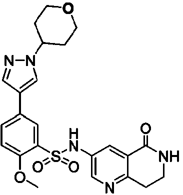 |
| 77 | 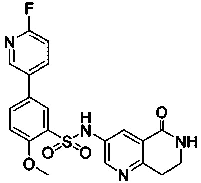 | 78 | 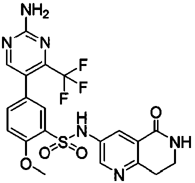 |
| 79 | 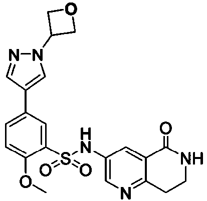 | 80 | 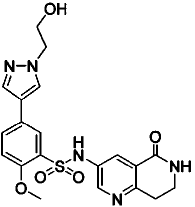 |
| 81 | 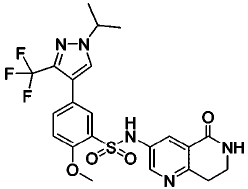 | 82 | 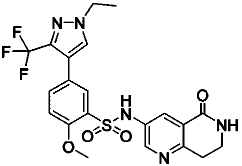 |
| 83 | 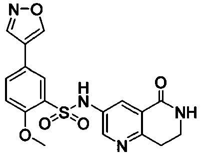 | 84 | 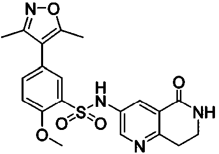 |
| 85 | 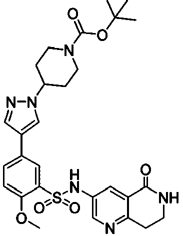 | 86 | 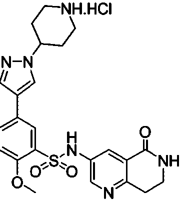 |
| 87 | 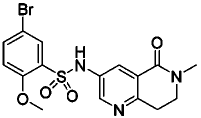 | 88 | 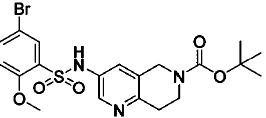 |
| 89 | 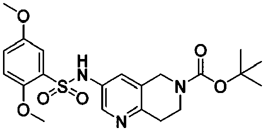 | 90 | 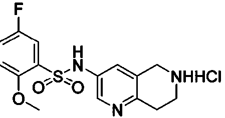 |
| 91 | 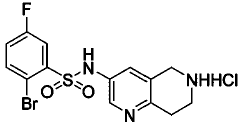 | 92 | I 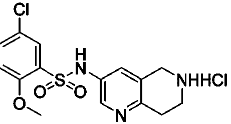 |
| 93 | 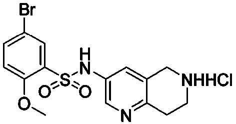 | 94 | 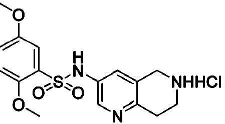 |
| 95 | 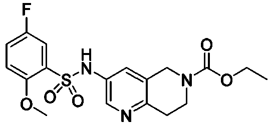 | 96 | I 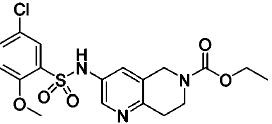 |
| 97 | 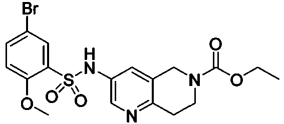 | 98 | 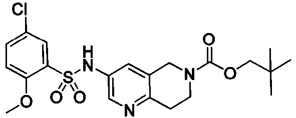 |
| 99 | 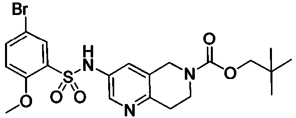 | 100 | 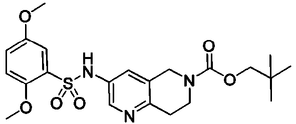 |
| 101 | 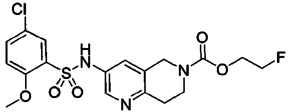 | 102 | 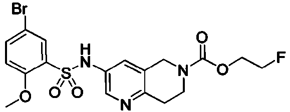 |
| 103 | 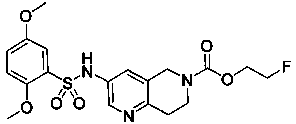 | 104 | 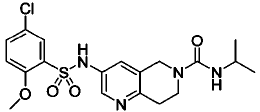 |
| 105 | 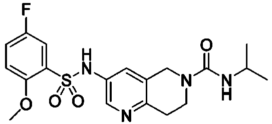 | 106 | 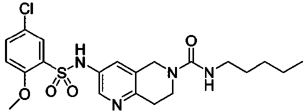 |
| 107 | 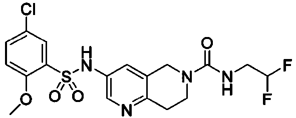 | 108 | 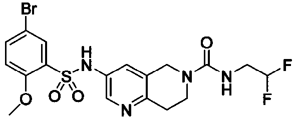 |
| 109 | 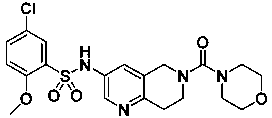 | 110 | 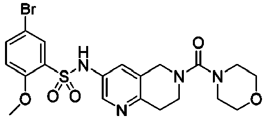 |
| 111 | 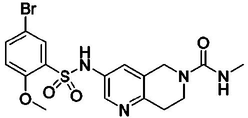 | 112 | 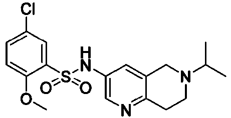 |
| 113 | 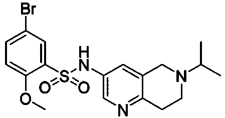 | 114 | 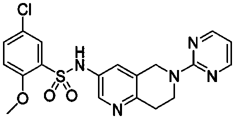 |
| 115 | 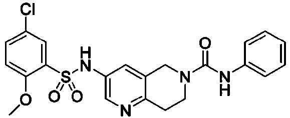 | 116 | 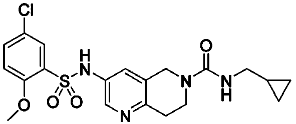 |
| 117 | 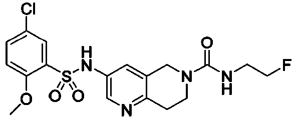 | 118 | 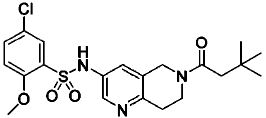 |
| 119 | 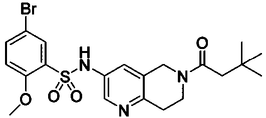 | 120 | 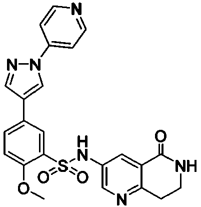 |
| 121 | 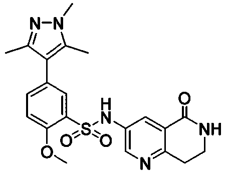 | 122 | 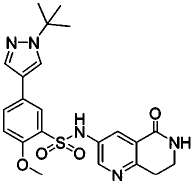 |
| 123 | 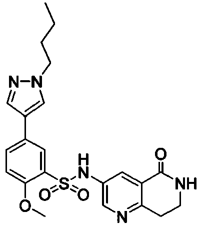 | 124 | 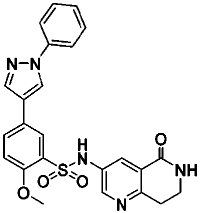 |

### Experimental Example 1: TNAP inhibitory activity

The TNAP inhibitory activity of the compounds according to the present invention was analyzed by colorimetric assay.

The experiments were performed according to the protocol of the alkaline phosphatase assay kit ab83369.

Enzyme (TNAP, Tissue None specific Alkaline Phosphatase), substrate (pNPP, p-nitrophenyl phosphate), and compounds were all prepared as stocks using the ALP assay buffer included in ab83369.
- TNAP enzyme: TNAP stock at a concentration of 0.2 µg/µl was diluted to 0.2 ng/µl using ALP assay buffer.
- Substrate: pNPP was dissolved in ALP assay buffer to make 5 mM and then diluted to 1 mM.
- Both test compounds and positive control compounds (MLS-0038949, SBI-425, and DS-1211) dissolved in DMSO were diluted 10-fold using ALP assay buffer.

For IC₅₀ measurement, the concentration of the test substance was divided into 11 sections (0, 1, 5, 10, 25, 50, 100, 250, 500, 1000, and 10000 nM) between 0 and 10 µM, including zero.

TNAP enzyme at a concentration of 0.2 ng/µl was dispensed into a 96-well plate (Costar) (10 µl/well), to which the 10-fold diluted test substance was added (15 µl/well). The plate was incubated at room temperature, shielded from light, for 10 minutes to allow the enzyme and compound to act. After incubation, 125 µl of 1 mM substrate (pNPP) was added to each well of the plate, and the plate was incubated for 2 hours at room temperature in a darkened state to allow the enzyme, substrate, and test compound to act. Absorbance was measured at 405 nm using a microplate reader (Tecan spark), and IC₅₀ was calculated using GraphPad Prism 9. The amount of p-nitrophenol produced was calculated through the absorbance, and the degree of TNAP activity inhibition was evaluated through the degree of inhibition of p-nitrophenol production, and the concentration of the compound that inhibited 50% was calculated as the IC₅₀ value. The IC₅₀ value of the test compound was determined based on the IC₅₀ of the positive control (190 nM).

The results are shown in Table 2 below.

When indicating IC₅₀ values, "A" is 10 nM or less, "B" is 10 nM or more but less than 100 nM, "C" is 100 nM or more but less than 1000 nM (1 µM), and "D" is 1000 nM (1 µM) or more. In the IC₅₀ values of the positive controls, DS-1211 exhibited B, while MLS-0038949 and SBD-425 exhibited C.

**[Table 2]**

| **Example** | **IC₅₀** | **Example** | **IC₅₀** | **Example** | **IC₅₀** | **Example** | **IC₅₀** |
|---|---|---|---|---|---|---|---|
| 1 | B | 32 | B | 63 | C | 94 | B |
| 2 | B | 33 | B | 64 | D | 95 | C |
| 3 | B | 34 | C | 65 | D | 96 | B |
| 4 | B | 35 | A | 66 | D | 97 | C |
| 5 | C | 36 | B | 67 | C | 98 | C |
| 6 | A | 37 | D | 68 | B | 99 | C |
| 7 | A | 38 | D | 69 | C | 100 | C |
| 8 | B | 39 | B | 70 | C | 101 | B |
| 9 | A | 40 | D | 71 | D | 102 | B |
| 10 | C | 41 | D | 72 | B | 103 | B |
| 11 | D | 42 | A | 73 | C | 104 | B |
| 12 | D | 43 | B | 74 | C | 105 | B |
| 13 | C | 44 | C | 75 | C | 106 | C |
| 14 | C | 45 | C | 76 | C | 107 | B |
| 15 | C | 46 | C | 77 | C | 108 | B |
| 16 | C | 47 | C | 78 | D | 109 | B |
| 17 | D | 48 | B | 79 | C | 110 | B |
| 18 | D | 49 | B | 80 | C | 111 | B |
| 19 | D | 50 | B | 81 | B | 112 | C |
| 20 | D | 51 | C | 82 | A | 113 | C |
| 21 | D | 52 | C | 83 | B | 114 | B |
| 22 | C | 53 | C | 84 | B | 115 | B |
| 23 | D | 54 | C | 85 | C | 116 | B |
| 24 | D | 55 | C | 86 | C | 117 | B |
| 25 | D | 56 | C | 87 | C | 118 | B |
| 26 | B | 57 | C | 88 | C | 119 | B |
| 27 | C | 58 | D | 89 | B | 120 | C |
| 28 | B | 59 | C | 90 | C | 121 | C |
| 29 | C | 60 | C | 91 | D | 122 | B |
| 30 | D | 61 | C | 92 | B | 123 | B |
| 31 | C | 62 | B | 93 | B | 124 | C |

### Experimental Example 2: Evaluation of anticalcification efficacy in saos-2 cells

### 1. Preparation of saos-2 cells

To confirm the inhibitory effect of calcification in saos-2 cells in which calcification is induced by overexpression of TNAP, saos-2 cells were cultured in a culture medium in an incubator at 37°C, 95% humidity, and 5% CO₂. RPMI-1640 (Corning) supplemented with 10% FBS (Gibco) and 1% Pen/Strep (Gibco) was used as the culture medium.

Saos-2 cells were seeded in a 48-well plate at the density of 1.5 x 10⁴ cells/well and used in the experiment when they reached 80-90% confluence after stabilization for 2 days.

### 2. Osteogenic differentiation of saos2 cells

After saos-2 cells were cultured in a 48-well plate to 80-90% confluence, the culture medium was removed and replaced with an osteogenic medium (OM). DMEM (Gibco) containing 10% FBS, 1% Pen/Strep, 100 nM dexamethasone (Sigma), 10 mM β-glycerophosphate (Sigma), and 0.05 mM L-ascorbic acid (Sigma) was used as the OM. Osteogenic differentiation of saos-2 cells by OM was induced for 7 days, and OM was replaced every 2 to 3 days.

### 3. Calcification inhibition by osteogenic differentiation and test compounds

When inducing calcification through OM in saos-2 cells, the cells were treated with concentrations of 0.5, 1, 5, and 10 µl of the TNAP inhibitor test compounds with the OM replacement cycle, except for the control group (OM). At this time, MLS-0038949, SBI-425, and DS-1211 compounds were treated together for comparison as positive controls.

### 4. Confirmation and quantification of calcification using Alizarin Red staining

After 7 days of OM induction, the medium was removed from each well and the plate was washed twice with PBS. Cells were fixed with 4% paraformaldehyde for 1 hour, then the fixative was removed and washed twice with deionized water (DW). ARS solution was added to the plate and shaken for 1 hour to stain the cells. When the cells were sufficiently stained red, the dye was removed, the cells were washed carefully with DW, dried, and then photographs were taken of the stained cells. The red-stained area represents calcification induced by OM, and the degree of calcification was determined based on the degree of redness. To quantify this, 10% cetylpyridinium chloride was dispensed into the wells and the dye was dissolved. The dye-dissolved solution was transferred to each well of a 96 well plate and absorbance was measured at 540 nm.

The degree of decrease in the measured absorbance by the test compounds based on the absorbance for OM was evaluated as the degree of inhibition of calcification.

### 5. Results

The inhibition efficacy of the test compound in saos-2 cells on calcification was expressed as follows: based on a test compound treatment concentration of 1 µM, inhibition of 50% or more was expressed as "A", inhibition of 50% or less was expressed as "B", and no efficacy was expressed as "C". The positive controls all exhibited calcification inhibition efficacy A in saos-2 cells.

**[Table 3]**

| Exa mpl e | calcifi cation inhibit ion | Exa mpl e | calcifi cation inhibit ion | Exa mpl e | calcifi cation inhibit ion | Exa mpl e | calcifi cation inhibit ion |
|---|---|---|---|---|---|---|---|
| 1 | A | 32 | A | 63 | C | 94 | A |
| 2 | A | 33 | A | 64 | C | 95 | A |
| 3 | A | 34 | A | 65 | C | 96 | A |
| 4 | A | 35 | A | 66 | C | 97 | A |
| 5 | B | 36 | A | 67 | B | 98 | A |
| 6 | A | 37 | C | 68 | A | 99 | C |
| 7 | A | 38 | C | 69 | C | 100 | A |
| 8 | A | 39 | A | 70 | A | 101 | C |
| 9 | A | 40 | C | 71 | C | 102 | A |
| 10 | A | 41 | C | 72 | A | 103 | A |
| 11 | A | 42 | A | 73 | A | 104 | A |
| 12 | C | 43 | A | 74 | A | 105 | A |
| 13 | A | 44 | A | 75 | A | 106 | A |
| 14 | A | 45 | A | 76 | A | 107 | A |
| 15 | A | 46 | A | 77 | A | 108 | A |
| 16 | A | 47 | A | 78 | C | 109 | A |
| 17 | C | 48 | A | 79 | A | 110 | A |
| 18 | C | 49 | A | 80 | A | 111 | A |
| 19 | C | 50 | A | 81 | A | 112 | C |
| 20 | C | 51 | A | 82 | A | 113 | C |
| 21 | C | 52 | A | 83 | A | 114 | A |
| 22 | A | 53 | B | 84 | A | 115 | A |
| 23 | C | 54 | A | 85 | C | 116 | A |
| 24 | C | 55 | A | 86 | C | 117 | A |
| 25 | C | 56 | A | 87 | C | 118 | A |
| 26 | A | 57 | A | 88 | A | 119 | A |
| 27 | A | 58 | B | 89 | A | 120 | C |
| 28 | A | 59 | A | 90 | B | 121 | C |
| 29 | A | 60 | A | 91 | C | 122 | C |
| 30 | B | 61 | A | 92 | A | 123 | A |
| 31 | A | 62 | A | 93 | A | 124 | C |

### Experimental Example 3: Warfarin-induced calcification mouse animal model experiment

1. Animal: C57BL/6 mouse (OrientBio, Sungnam-city, Korea)
   Gender: male
   Age on arrival: C57BL/6 (6 weeks)
   Raising: Animals were raised in filter cap polycarbonate cages, 5 animals each, in a temperature (25°C) and humidity (45-55%) controlled sterile clean room. The light:dark cycle was 12 hours:12 hours, and sterilized food and water were provided with free access.
   Guidelines: All animals were treated in accordance with the Daegu Haany University Institutional Animal Care and Use Committee (Gyeongsan, Korea).
2. General experimental methods: *In vivo* drug administration

After acclimating the mice for one week, they were divided into each group and tested.

After acclimation for one week, the groups were randomized.

The mice were divided into four groups: a group that received no treatment (A), a group that was treated with warfarin and vitamin K1 to induce calcification (B), a group that was administered orally with 0.5% CMC (carboxymethyl Cellulose) (C), and a group that was administered with the test compound. The compounds of Example 1 (K) and Example 6 (L) were selected as the test compound.

All groups, except the untreated control group (Normal), were administered both warfarin and vitamin K1 at 8:00 AM and warfarin alone at 8:00 PM for 4 weeks.

Heart tissue samples were obtained from each group and used for analysis.

Individual heart samples were cut crosswise, one section at a time, around the origin/root region of the ascending aorta. Histological sections were then prepared from all the samples received. All cross-sectioned cardiac-aortic valve sections were refixed in 10% neutral buffered formalin for 24 hours to prepare histological samples. After the samples were embedded in paraffin, two serial sections of 3 to 4 µm thickness were prepared on each paraffin block and stained with hematoxylin & eosin (HE) for general histopathology or von Kossa (VK) for calcium deposition, respectively. To observe more detailed changes, a computer-assisted image analysis program and a histological camera system were used to calculate the mean aortic valve thickness (µm) and the number of inflammatory cells infiltrating the aortic valve area (cells/mm²) in HE staining, and the mean calcified aortic valve area (%/mm²) in VK staining in this histopathological analysis. As shown in Table 4, the number of inflammatory cells was decreased and the calcified area was significantly reduced in the group treated with the compound of the present invention compared to the calcification-induced group.

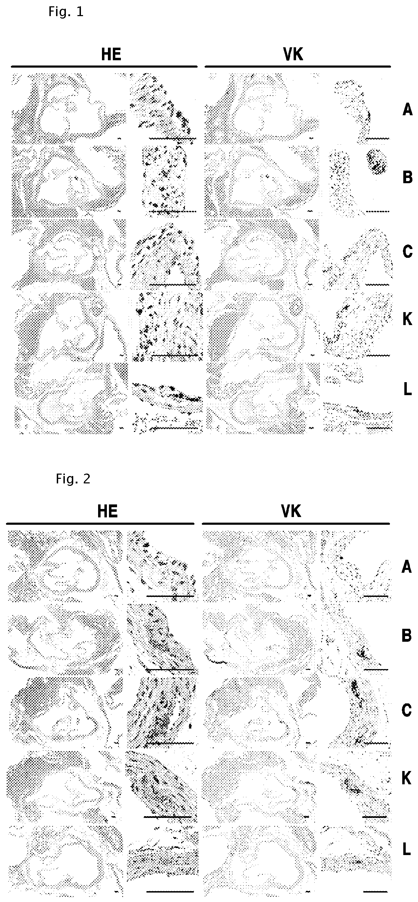

Meanwhile, Figure 1 shows the results of staining. According to this, in the warfarin and vitamin K1-induced calcification group (B), it was possible to confirm through HE staining that the tissue thickness was increased and inflammatory cells gathered due to increased inflammation. The area with increased calcification is stained black by von Kossa staining, and in the groups (K, L) treated with the compounds of Example 1 and 6, the blackened area was significantly reduced.

**[Table 4]**

| Group | Mean thickness (µm) | Number of inflammatory cells (cells/mm²) | Calcified area (%/mm²) |
|---|---|---|---|
| Normal group (A) | 72.06±8.31 | 25.20±13.31 | 1.00±0.33 |
| Calcification group (B) | 155.74±14.12 | 385.20±70.34 | 5.75±0.95 |
| Vehicle treated group (C) | 152.30±8.78 | 386.80±77.92 | 5.77±0.58 |
| Example 1 treated group (K) | 112.42±10.81 | 144.40±43.12 | 2.45±0.64 |
| Example 6 treated group (L) | 91.69±15.70 | 80.40±14.90 | 1.16±0.30 |

### Experimental Example 4: Vitamin D-induced calcification mouse animal model experiment

Experimental animals were prepared similarly to Experimental Example 3. Excluding the normal group, four groups were administered with high-dose (6.5x10⁵ IU/Kg) vitamin D3 subcutaneously once a day for a total of 3 days to induce calcification. The compounds of Examples 1 and 6 were administered orally together from the first day of vitamin D3 administration, and then administered once a day for 5 more days without vitamin D3 administration, for a total of 8 days.

Samples were obtained and analyzed in the same manner as in Experimental Example 3. As shown in Table 5, the number of inflammatory cells was decreased and the calcified area was significantly reduced in the group treated with the compound of the present invention compared to the calcification-induced group.

Meanwhile, Figure 2 shows the results of staining. According to this, in the high-dose vitamin D3-induced calcification group (B), it was possible to confirm through HE staining that the tissue thickness was increased and inflammatory cells gathered due to increased inflammation. The area with increased calcification is stained black by von Kossa staining, and in the groups (K, L) treated with the compounds of Example 1 and 6, the blackened area was significantly reduced.

**[Table 5]**

| Group | Mean thickness (µm) | Number of inflammatory cells (cells/mm²) | Calcified area (%/mm²) |
|---|---|---|---|
| Normal group (A) | 43.05±10.30 | 22.80±10.06 | 1.30±0.56 |
| Calcification group (B) | 113.80±23.85 | 184.89±19.37 | 12.89±2.18 |
| Vehicle treated group (C) | 112.55±12.34 | 182.22±23.14 | 13.19±2.54 |
| Example 1 treated group (K) | 64.26±12.34 | 110.89±21.64 | 7.95±1.16 |
| Example 6 treated group (L) | 51.76±10.39 | 58.89±17.32 | 2.44±0.92 |

As confirmed through Experimental Examples 1 to 4, the compounds according to the present invention were not only effective in inhibiting TNAP, but also highly effective in inhibiting ectopic calcification and slowing its progression. Thus, the compounds of the present invention are expected to be very useful in the prevention or treatment of diseases associated with overexpression or overactivation of TNAP or diseases associated with ectopic calcification.

## Claims

1. A compound of formula 1 below or a pharmaceutically acceptable salt thereof: 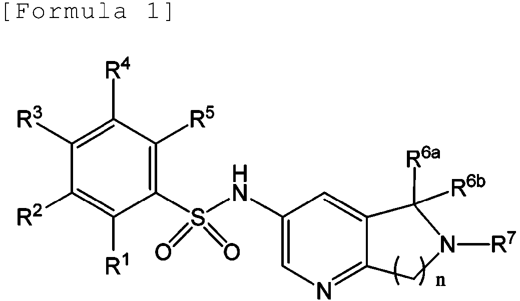 wherein,
R¹ is hydrogen, halogen, nonsubstituted or substituted phenyl, or C₁₋₁₀ alkoxy;
R² is hydrogen;
R³ is hydrogen or halogen;
R⁴ is hydrogen, halogen, nonsubstituted or substituted phenyl, nonsubstituted or substituted 5-9 membered heteroaryl, 6 membered cycloalkenyl nonsubstituted or substituted with one or more halogens, nonsubstituted or substituted 6 membered heterocycloalkenyl, or C₁₋₁₀ alkoxy;
R⁵ is hydrogen or halogen;
or
two adjacent substituents of R¹ to R⁵ together form -(CH₂)m-O-, -O-(CH₂)m-O-, or -O-(CH₂)m-CO-NH-, while remaining three substituents are as described above;
the substituted phenyl is substituted with 1 to 3 substituents selected from the group consisting of C₁₋₁₀ alkoxy, carboxyl, nonsubstituted or substituted C₁₋₁₀ alkyl, 3-6 membered cycloalkyl substituted with carboxyl, halogen and hydroxyl, or is substituted at two adjacent positions to form -O-(CH₂)m-O-, wherein the substituted C₁₋₁₀ alkyl is substituted with carboxyl, C₁₋₁₀ alkoxycarbonyl, amino or C₁₋₁₀ alkylaminocarbonyl;
the substituted 5-9 membered heteroaryl is substituted with 1 to 3 substituents selected from the group consisting of halogen, amino, nonsubstituted C₁₋₁₀ alkyl, substituted C₁₋₁₀ alkyl, NH₂CO-, nonsubstituted or substituted 4-6 membered heterocycloalkyl, C₁₋₁₀ alkylsulfonyl, 3-6 membered cycloalkyl, 5-6 membered heteroaryl and phenyl, wherein the substituted C₁₋₁₀ alkyl is substituted with carboxyl, C₁₋₁₀ alkoxycarbonyl, C₁₋₁₀ alkylaminocarbonyl, 5-6 membered heteroaryl substituted with C₁₋₁₀ alkyl or phenyl substituted with one or more halogens, one or more halogens, or hydroxyl, and the substituted 4-6 membered heterocycloalkyl is substituted with C₁₋₁₀ alkyl or C₁₋₁₀ alkoxycarbonyl;
the substituted 6 membered heterocycloalkenyl is substituted with C₁₋₁₀ alkoxycarbonyl or C₁₋₁₀ alkylcarbonyl;
R^{6a} and R^{6b} are both hydrogens or together form oxo;
R⁷ is hydrogen, 5-6 membered heteroaryl, -COₓ-R^{8a}, -CONR^{8b}R^{8c}, or C₁₋₁₀alkyl, wherein x is 1 or 2,
R^{8a} and R^{8b} are independently nonsubstituted or substituted C₁₋₁₀ alkyl, or C₆₋₁₀ aryl, wherein the substituted C₁₋₁₀ alkyl is substituted with one or more halogens, or 3-6 membered cycloalkyl,
R^{8c} is hydrogen or forms 5-6 membered heterocycloalkyl together with R^{8b} and the nitrogen atom substituted therewith;
m is an integer of 1 to 4; and
n is an integer of 1 to 3.

2. The compound or the pharmaceutically acceptable salt thereof according to claim 1, wherein:
R¹ is hydrogen, halogen, nonsubstituted or substituted phenyl, or C₁₋₅ alkoxy, and the substituted phenyl is cyclopropyl substituted with carboxyl, or nonsubstituted or substituted C₁₋₅ alkyl, wherein the substituted C₁₋₅ alkyl is substituted with carboxyl or amino.

3. The compound or the pharmaceutically acceptable salt thereof according to claim 1, wherein:
R⁴ is hydrogen, halogen, nonsubstituted or substituted phenyl, 9 membered bicyclic heteroaryl, nonsubstituted or substituted 5-6 membered heteroaryl, 6 membered cycloalkenyl nonsubstituted or substituted with one or more halogens, nonsubstituted or substituted 6 membered heterocycloalkenyl containing one heteroatom among O and N, or C₁₋₅ alkoxy;
the substituted phenyl is substituted with 1 to 3 substituents selected from the group consisting of C₁₋₅ alkoxy, carboxyl, nonsubstituted or substituted C₁₋₅ alkyl, cyclopropyl substituted with carboxyl, halogen and hydroxyl, or is substituted at two adjacent positions to form -O-(CH₂)m-O-, wherein the substituted C₁₋₅ alkyl is substituted with carboxyl, C₁₋₅ alkoxycarbonyl, amino or C₁₋₅ alkylaminocarbonyl;
the substituted 5-6 membered heteroaryl is substituted with 1 to 3 substituents selected from the group consisting of halogen, amino, nonsubstituted C₁₋₅ alkyl, substituted C₁₋₅ alkyl, NH₂CO-, nonsubstituted or substituted 4-6 membered heterocycloalkyl, methylsulfonyl, cyclopropyl, 6 membered heteroaryl, and phenyl, wherein the substituted C₁₋₅ alkyl is substituted with carboxyl, C₁₋₅ alkoxycarbonyl, C₁₋₅ alkylaminocarbonyl, 5 membered heteroaryl substituted with C₁₋₅ alkyl or phenyl substituted with one or more halogens, one or more halogens, or hydroxyl, and the substituted 4-6 membered heterocycloalkyl is substituted with C₁₋₅ alkyl or C₁₋₅ alkoxycarbonyl;
the substituted 6 membered heterocycloalkenyl is substituted with C₁₋₅ alkoxycarbonyl or C₁₋₅ alkylcarbonyl; and
m is an integer of 1 to 3.

4. The compound or the pharmaceutically acceptable salt thereof according to claim 3, wherein:
when the two adjacent substituents of R¹ to R⁵ together form -(CH₂)m-O-, -O-(CH₂)m-O-, or -O-(CH₂)ₘ-CO-NH-, each of remaining three substituents is independently hydrogen, halogen, or substituted phenyl,
in which the substituted phenyl is substituted with 3-6 membered cycloalkyl substituted with carboxyl, or C₁₋₁₀ alkyl substituted with amino, and
m is an integer of 1 to 3.

5. The compound or the pharmaceutically acceptable salt thereof according to claim 1, wherein:
when the two adjacent substituents of R¹ to R⁵ is R¹ and R², and R¹ and R² together form -(CH₂)m-O-, -O-(CH₂)m-O-, or -O-(CH₂)ₘ-CO-NH-,
each of remaining three substituents is independently hydrogen, halogen, or substituted phenyl,
in which the substituted phenyl is substituted with cyclopropyl substituted with carboxyl, or C₁₋₅ alkyl substituted with amino;
or
when the two adjacent substituents of R¹ to R⁵ is R² and R³, and R² and R³ together form -(CH₂)m-O- or -O-(CH₂)ₘ-CO-NH-,
remaining three substituents are all hydrogens; and
m is an integer of 1 to 2.

6. The compound or the pharmaceutically acceptable salt thereof according to claim 1, wherein:
R⁷ is hydrogen, 6 membered heteroaryl, -COₓ-R^{8a},-CONR^{8b}R^{8c}, or C₁₋₅ alkyl, wherein x is 1 or 2,
R^{8a} is C₁₋₆ alkyl nonsubstituted or substituted with 1 halogen,
R^{8b} is nonsubstituted or substituted C₁₋₆ alkyl, or phenyl,
in which the substituted C₁₋₆ alkyl is substituted with 1 or 2 halogens, or cyclopropyl,
R^{8c} is hydrogen or forms 6 membered heterocycloalkyl together with R^{8b} and the nitrogen atom substituted therewith.

7. The compound or the pharmaceutically acceptable salt thereof according to claim 1, wherein:
R¹ is C₁₋₅ alkoxy, halogen, hydrogen, or nonsubstituted or substituted phenyl;
R² is hydrogen;
R³ is hydrogen or halogen;
R⁴ is hydrogen, halogen, nonsubstituted or substituted phenyl, pyrazolopyridinyl, nonsubstituted or substituted 5-6 membered heteroaryl, 6 membered cycloalkenyl nonsubstituted or substituted with one or more halogens, nonsubstituted or substituted heterocycloalkenyl containing one heteroatom among O and N, or C₁₋₅ alkoxy;
R⁵ is hydrogen or halogen;
or
two adjacent substituents of R¹ to R⁵ together form -(CH₂)m-O-, -O-(CH₂)m-O-, or -O-(CH₂)m-CO-NH-, and each of remaining three substituents is independently hydrogen, halogen, or substituted phenyl, wherein the substituted phenyl is substituted with cyclopropyl substituted with carboxyl, or C₁₋₃ alkyl substituted with amino;
the substituted phenyl is substituted with C₁₋₅ alkoxy, carboxyl, nonsubstituted or substituted C₁₋₅ alkyl, or cyclopropyl substituted with carboxyl, or is substituted with 1 to 3 substituents selected from the group consisting of halogen and hydroxyl, or is substituted at two adjacent positions to form -O-(CH₂)m-O-, wherein the substituted C₁₋₅ alkyl is substituted with carboxyl, C₁₋₅ alkoxycarbonyl, amino or C₁₋₅ alkylaminocarbonyl;
the substituted 5-6 membered heteroaryl is substituted with 1 to 3 substituents selected from the group consisting of halogen, amino, nonsubstituted C₁₋₅ alkyl, substituted C₁₋₅ alkyl, NH₂CO-, nonsubstituted or substituted 4-6 membered heterocycloalkyl, methylsulfonyl, cyclopropyl, 6 membered heteroaryl and phenyl, wherein the substituted C₁₋₅ alkyl is substituted with carboxyl, C₁₋₅ alkoxycarbonyl, C₁₋₅ alkylaminocarbonyl, 5 membered heteroaryl substituted with C₁₋₅ alkyl or phenyl substituted with one halogen, one or more halogens, or hydroxyl, and the substituted 4-6 membered heterocycloalkyl is substituted with C₁₋₅ alkyl or C₁₋₅ alkoxycarbonyl;
the substituted 6 membered heterocycloalkenyl is substituted with C₁₋₅ alkoxycarbonyl or C₁₋₅ alkylcarbonyl;
R^{6a} and R^{6b} are both hydrogen or together form oxo;
R⁷ is hydrogen, pyrimidinyl, -COₓ-R^{8a}, -CONR^{8b}R^{8c}, or C₁₋₅ alkyl, wherein x is 1 or 2,
R^{8a} is C₁₋₆ alkyl nonsubstituted or substituted with 1 halogen,
R^{8b} is nonsubstituted or substituted C₁₋₆ alkyl, or phenyl,
wherein the substituted C₁₋₆ alkyl is substituted with 1 or 2 halogens, or cyclopropyl,
R^{8c} is hydrogen or forms morpholinyl together with R^{8b} and the nitrogen atoms substituted therewith;
m is an integer of 1 or 2; and
n is an integer of 1 to 3.

8. The compound or the pharmaceutically acceptable salt thereof according to claim 1, wherein:
R¹ is hydrogen, halogen, 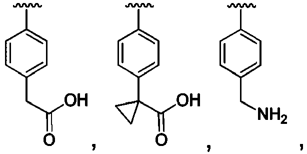 or methoxy;
R² is hydrogen;
R³ is hydrogen or halogen;
R⁴ is hydrogen, halogen, 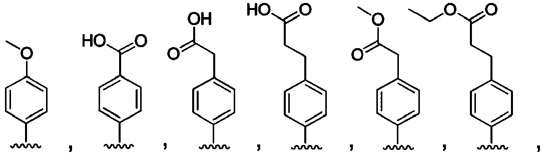 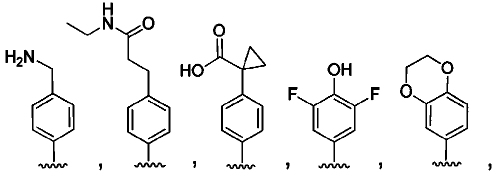 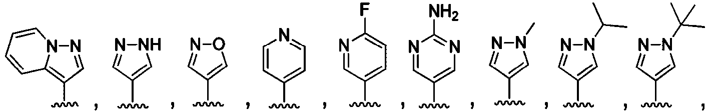 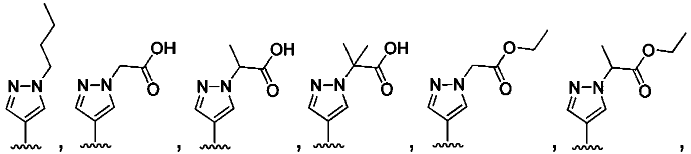 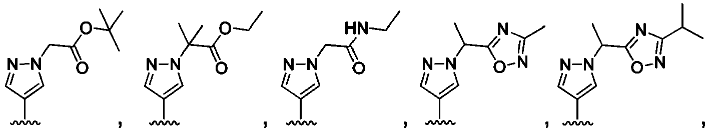 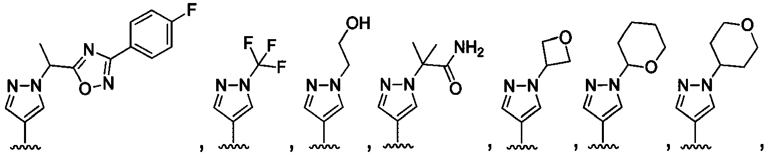 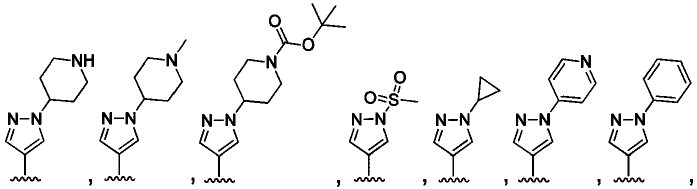 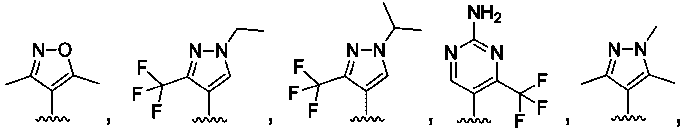 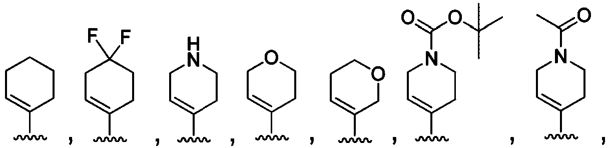 or methoxy;
R⁵ is hydrogen or halogen;
or
two adjacent substituents of R¹ to R⁵ together form 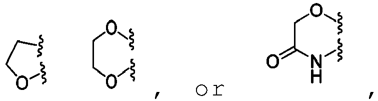 and each of remaining three substituents is independently hydrogen, halogen, or substituted phenyl, wherein the substituted phenyl is substituted with cyclopropyl substituted with carboxyl or C₁₋₃ alkyl substituted with amino;
R^{6a} and R^{6b} are both hydrogen or together forms oxo;
R⁷ is hydrogen, 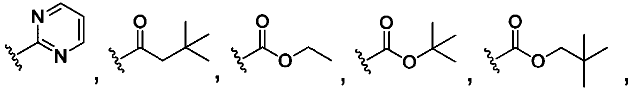 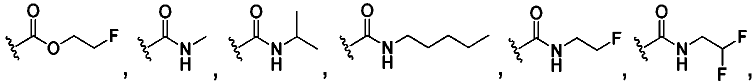 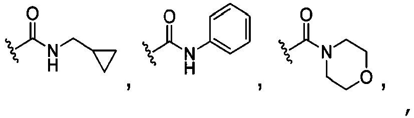 or C₁₋₃ alkyl; and
n is an integer of 1 to 3.

9. The compound or the pharmaceutically acceptable salt thereof according to claim 1, wherein the compound or the pharmaceutically acceptable salt thereof is selected from the group consisting of the following compounds:
<1> 5-chloro-2-methoxy-N-(5-oxo-6,7-dihydro-5H-pyrrolo[3,4-b]pyridine-3-yl)benzenesulfonamide;
<2> 5-bromo-2-methoxy-N-(5-oxo-6,7-dihydro-5H-pyrrolo[3,4-b]pyridine-3-yl)benzenesulfonamide;
<3> 2,5-dimethoxy-N-(5-oxo-6,7-dihydro-5H-pyrrolo[3,4-b]pyridine-3-yl)benzenesulfonamide;
<4> 5-fluoro-2-methoxy-N-(5-oxo-6,7-dihydro-5H-pyrrolo[3,4-b]pyridine-3-yl)benzenesulfonamide;
<5> 5-bromo-N-(5-oxo-6,7-dihydro-5H-pyrrolo[3,4-b]pyridine-3-yl)-2,3-dihydrobenzofuran-7-sulfonamide;
<6> 5-chloro-2-methoxy-N-(5-oxo-5,6,7,8-tetrahydro-1,6-naphthyridine-3-yl)benzenesulfonamide;
<7> 2,5-dimethoxy-N-(5-oxo-5,6,7,8-tetrahydro-1,6-naphthyridine-3-yl)benzenesulfonamide;
<8> 5-fluoro-2-methoxy-N-(5-oxo-5,6,7,8-tetrahydro-1,6-naphthyridine-3-yl)benzenesulfonamide;
<9> 5-bromo-2-methoxy-N-(5-oxo-5,6,7,8-tetrahydro-1,6-naphthyridine-3-yl)benzenesulfonamide;
<10> 5-bromo-N-(5-oxo-5,6,7,8-tetrahydro-1,6-naphthyridine-3-yl)-2,3-dihydrobenzofuran-7-sulfonamide;
<11> 2-bromo-5-fluoro-N-(5-oxo-6,7-dihydro-5H-pyrrolo[3,4-b]pyridine-3-yl)benzenesulfonamide;
<12> 2-bromo-4,6-difluoro-N-(5-oxo-6,7-dihydro-5H-pyrrolo[3,4-b]pyridine-3-yl)benzenesulfonamide;
<13> 5-chloro-2-methoxy-N-(5-oxo-6,7,8,9-tetrahydro-5H-pyrido[3,2-c]azepine-3-yl)benzenesulfonamide;
<14> 5-bromo-2-methoxy-N-(5-oxo-6,7,8,9-tetrahydro-5H-pyrido[3,2-c]azepine-3-yl)benzenesulfonamide;
<15> 5-fluoro-2-methoxy-N-(5-oxo-6,7,8,9-tetrahydro-5H-pyrido[3,2-c]azepine-3-yl)benzenesulfonamide;
<16> 2,5-dimethoxy-N-(5-oxo-6,7,8,9-tetrahydro-5H-pyrido[3,2-c]azepine-3-yl)benzenesulfonamide;
<17> 5-bromo-N-(5-oxo-6,7,8,9-tetrahydro-5H-pyrido[3,2-c]azepine-3-yl)-2,3-dihydrobenzofuran-7-sulfonamide;
<18> N-(5-oxo-6,7-dihydro-5H-pyrrolo[3,4-b]pyridine-3-yl)-2,3-dihydrobenzofuran-7-sulfonamide;
<19> N-(5-oxo-5,6,7,8-tetrahydro-1,6-naphthyridine-3-yl)-2,3-dihydrobenzofuran-7-sulfonamide;
<20> 5-chloro-N-(5-oxo-6,7-dihydro-5H-pyrrolo[3,4-b]pyridine-3-yl)-2,3-dihydrobenzofuran-7-sulfonamide;
<21> 5-fluoro-N-(5-oxo-6,7-dihydro-5H-pyrrolo[3,4-b]pyridine-3-yl)-2,3-dihydrobenzofuran-7-sulfonamide;
<22> 5-chloro-N-(5-oxo-5,6,7,8-tetrahydro-1,6-naphthyridine-3-yl)-2,3-dihydrobenzofuran-7-sulfonamide;
<23> 5-fluoro-N-(5-oxo-5,6,7,8-tetrahydro-1,6-naphthyridine-3-yl)-2,3-dihydrobenzofuran-7-sulfonamide;
<24> N-(5-oxo-5,6,7,8-tetrahydro-1,6-naphthyridine-3-yl)-2,3-dihydrobenzo[b][1,4]dioxine-6-sulfonamide;
<25> 3-oxo-N-(5-oxo-5,6,7,8-tetrahydro-1,6-naphthyridine-3-yl)-3,4-dihydro-2H-benzo[b][1,4]oxazine-6-sulfonamide;
<26> 2-methoxy-N-(5-oxo-5,6,7,8-tetrahydro-1,6-naphthyridine-3-yl)benzenesulfonamide;
<27> methyl 2-(4'-methoxy-3'-(N-(5-oxo-6,7-dihydro-5H-pyrrolo[3,4-b]pyridine-3-yl)sulfamoyl)-[1,1'-biphenyl]-4-yl)acetate;
<28> 2-(4'-methoxy-3'-(N-(5-oxo-6,7-dihydro-5H-pyrrolo[3,4-b]pyridine-3-yl)sulfamoyl)-[1,1'-biphenyl]-4-yl)acetic acid;
<29> 5-(2,3-dihydrobenzo[b][1,4]dioxine-6-yl)-2-methoxy-N-(5-oxo-5,6,7,8-tetrahydro-1,6-naphthyridine-3-yl)benzenesulfonamide;
<30> 4,4'-dimethoxy-N-(5-oxo-5,6,7,8-tetrahydro-1,6-naphthyridine-3-yl)-[1,1'-biphenyl]-3-sulfonamide;
<31> 2-methoxy-N-(5-oxo-5,6,7,8-tetrahydro-1,6-naphthyridine-3-yl)-5-(pyridine-4-yl)benzenesulfonamide;
<32> 1-(4'-methoxy-3'-(N-(5-oxo-5,6,7,8-tetrahydro-1,6-naphthyridine-3-yl)sulfamoyl)-[1,1'-biphenyl]-4-yl)cyclopropane-1-carboxylic acid;
<33> 2-(4'-methoxy-3'-(N-(5-oxo-5,6,7,8-tetrahydro-1,6-naphthyridine-3-yl)sulfamoyl)-[1,1'-biphenyl]-4-yl)acetic acid;
<34> 4'-methoxy-3'-(N-(5-oxo-5,6,7,8-tetrahydro-1,6-naphthyridine-3-yl)sulfamoyl)-[1,1'-biphenyl]-4-carboxylic acid;
<35> 2-(4'-fluoro-2'-(N-(5-oxo-5,6,7,8-tetrahydro-1,6-naphthyridine-3-yl)sulfamoyl)-[1,1'-biphenyl]-4-yl)acetic acid;
<36> 1-(4'-fluoro-2'-(N-(5-oxo-5,6,7,8-tetrahydro-1,6-naphthyridine-3-yl)sulfamoyl)-[1,1'-biphenyl]-4-yl)cyclopropane-1-carboxylic acid;
<37> 1-(3',5'-difluoro-2'-(N-(5-oxo-5,6,7,8-tetrahydro-1,6-naphthyridine-3-yl)sulfamoyl)-[1,1'-biphenyl]-4-yl)cyclopropane-1-carboxylic acid;
<38> 1-(4-(7-(N-(5-oxo-5,6,7,8-tetrahydro-1,6-naphthyridine-3-yl)sulfamoyl)-2,3-dihydrobenzofuran-5-yl)phenyl)cyclopropane-1-carboxylic acid;
<39> 4'-(aminomethyl)-4-fluoro-N-(5-oxo-5,6,7,8-tetrahydro-1,6-naphthyridine-3-yl)-[1,1'-biphenyl]-2-sulfonamide hydrochloride;
<40> 4'-(aminomethyl)-3,5-difluoro-N-(5-oxo-5,6,7,8-tetrahydro-1,6-naphthyridine-3-yl)-[1,1'-biphenyl]-2-sulfonamide hydrochloride;
<41> 5-(4-(aminomethyl)phenyl)-N-(5-oxo-5,6,7,8-tetrahydro-1,6-naphthyridine-3-yl)-2,3-dihydrobenzofuran-7-sulfonamide;
<42> ethyl 2-(4-(4-methoxy-3-(N-(5-oxo-5,6,7,8-tetrahydro-1,6-naphthyridine-3-yl)sulfamoyl)phenyl)-1H-pyrazole-1-yl)propanoate;
<43> 2-(4-(4-methoxy-3-(N-(5-oxo-5,6,7,8-tetrahydro-1,6-naphthyridine-3-yl)sulfamoyl)phenyl)-1H-pyrazole-1-yl)propanoic acid;
<44> 5-(2-aminopyrimidine-5-yl)-2-methoxy-N-(5-oxo-5,6,7,8-tetrahydro-1,6-naphthyridine-3-yl)benzenesulfonamide;
<45> 2-methoxy-N-(5-oxo-5,6,7,8-tetrahydro-1,6-naphthyridine-3-yl)-5-(pyrazolo[1,5-a]pyridine-3-yl)benzenesulfonamide;
<46> 3',5'-difluoro-4'-hydroxy-4-methoxy-N-(5-oxo-5,6,7,8-tetrahydro-1,6-naphthyridine-3-yl)-[1,1'-biphenyl]-3-sulfonamide;
<47> tert-butyl 2-(4-(4-methoxy-3-(N-(5-oxo-5,6,7,8-tetrahydro-1,6-naphthyridine-3-yl)sulfamoyl)phenyl)-1H-pyrazole-1-yl)acetate;
<48> 2-(4-(4-methoxy-3-(N-(5-oxo-5,6,7,8-tetrahydro-1,6-naphthyridine-3-yl)sulfamoyl)phenyl)-1H-pyrazole-1-yl)acetic acid;
<49> ethyl 3-(4'-methoxy-3'-(N-(5-oxo-5,6,7,8-tetrahydro-1,6-naphthyridine-3-yl)sulfamoyl)-[1,1'-biphenyl]-4-yl)propanoate;
<50> 3-(4'-methoxy-3'-(N-(5-oxo-5,6,7,8-tetrahydro-1,6-naphthyridine-3-yl)sulfamoyl)-[1,1'-biphenyl]-4-yl)propanoic acid;
<51> 5-(3,6-dihydro-2H-pyran-4-yl)-2-methoxy-N-(5-oxo-5,6,7,8-tetrahydro-1,6-naphthyridine-3-yl)benzenesulfonamide;
<52> tert-butyl 4-(4-methoxy-3-(N-(5-oxo-5,6,7,8-tetrahydro-1,6-naphthyridine-3-yl)sulfamoyl)phenyl)-3,6-dihydropyridine-1(2H)-carboxylate;
<53> 2-methoxy-N-(5-oxo-5,6,7,8-tetrahydro-1,6-naphthyridine-3-yl)-5-(1,2,3,6-tetrahydropyridine-4-yl)benzenesulfonamide hydrochloride;
<54> 2-methoxy-N-(5-oxo-5,6,7,8-tetrahydro-1,6-naphthyridine-3-yl)-5-(1-(tetrahydro-2H-pyran-2-yl)-1H-pyrazole-4-yl)benzenesulfonamide;
<55> 2-methoxy-N-(5-oxo-5,6,7,8-tetrahydro-1,6-naphthyridine-3-yl)-5-(1H-pyrazole-4-yl)benzenesulfonamide;
<56> 2-bromo-5-fluoro-N-(5-oxo-5,6,7,8-tetrahydro-1,6-naphthyridine-3-yl)benzenesulfonamide;
<57> 2-methoxy-5-(1-methyl-1H-pyrazole-4-yl)-N-(5-oxo-5,6,7,8-tetrahydro-1,6-naphthyridine-3-yl)benzenesulfonamide;
<58> 2-(4-(4-methoxy-3-(N-(5-oxo-5,6,7,8-tetrahydro-1,6-naphthyridine-3-yl)sulfamoyl)phenyl)-1H-pyrazole-1-yl)-2-methylpropanamide;
<59> ethyl 2-(4-(4-methoxy-3-(N-(5-oxo-5,6,7,8-tetrahydro-1,6-naphthyridine-3-yl)sulfamoyl)phenyl)-1H-pyrazole-1-yl)-2-methylpropanoate;
<60> 2-(4-(4-methoxy-3-(N-(5-oxo-5,6,7,8-tetrahydro-1,6-naphthyridine-3-yl)sulfamoyl)phenyl)-1H-pyrazole-1-yl)-2-methylpropanoic acid;
<61> 5-(1-acetyl-1,2,3,6-tetrahydropyridine-4-yl)-2-methoxy-N-(5-oxo-5,6,7,8-tetrahydro-1,6-naphthyridine-3-yl)benzenesulfonamide;
<62> 5-(5,6-dihydro-2H-pyran-3-yl)-2-methoxy-N-(5-oxo-5,6,7,8-tetrahydro-1,6-naphthyridine-3-yl)benzenesulfonamide;
<63> 4-methoxy-N-(5-oxo-5,6,7,8-tetrahydro-1,6-naphthyridine-3-yl)-2',3',4',5'-tetrahydro-[1,1'-biphenyl]-3-sulfonamide;
<64> 4',4'-difluoro-4-methoxy-N-(5-oxo-5,6,7,8-tetrahydro-1,6-naphthyridine-3-yl)-2',3',4',5'-tetrahydro-[1,1'-biphenyl]-3-sulfonamide;
<65> 2-methoxy-5-(1-(1-methylpiperidine-4-yl)-1H-pyrazole-4-yl)-N-(5-oxo-5,6,7,8-tetrahydro-1,6-naphthyridine-3-yl)benzenesulfonamide;
<66> N-ethyl-2-(4-(4-methoxy-3-(N-(5-oxo-5,6,7,8-tetrahydro-1,6-naphthyridine-3-yl)sulfamoyl)phenyl)-1H-pyrazole-1-yl)acetamide;
<67> N-ethyl-3-(4'-methoxy-3'-(N-(5-oxo-5,6,7,8-tetrahydro-1,6-naphthyridine-3-yl)sulfamoyl)-[1,1'-biphenyl]-4-yl)propanamide;
<68> ethyl 2-(4-(4-methoxy-3-(N-(5-oxo-5,6,7,8-tetrahydro-1,6-naphthyridine-3-yl)sulfamoyl)phenyl)-1H-pyrazole-1-yl)acetate;
<69> 5-(1-(1-(3-isopropyl-1,2,4-oxadiazole-5-yl)ethyl)-1H-pyrazole-4-yl)-2-methoxy-N-(5-oxo-5,6,7,8-tetrahydro-1,6-naphthyridine-3-yl)benzenesulfonamide;
<70> 2-methoxy-5-(1-(1-(3-methyl-1,2,4-oxadiazole-5-yl)ethyl)-1H-pyrazole-4-yl)-N-(5-oxo-5,6,7,8-tetrahydro-1,6-naphthyridine-3-yl)benzenesulfonamide;
<71> 5-(1-(1-(3-(4-fluorophenyl)-1,2,4-oxadiazole-5-yl)ethyl)-1H-pyrazole-4-yl)-2-methoxy-N-(5-oxo-5,6,7,8-tetrahydro-1,6-naphthyridine-3-yl)benzenesulfonamide;
<72> 5-(1-isopropyl-1H-pyrazole-4-yl)-2-methoxy-N-(5-oxo-5,6,7,8-tetrahydro-1,6-naphthyridine-3-yl)benzenesulfonamide;
<73> 2-methoxy-N-(5-oxo-5,6,7,8-tetrahydro-1,6-naphthyridine-3-yl)-5-(1-(trifluoromethyl)-1H-pyrazole-4-yl)benzenesulfonamide;
<74> 5-(1-cyclopropyl-1H-pyrazole-4-yl)-2-methoxy-N-(5-oxo-5,6,7,8-tetrahydro-1,6-naphthyridine-3-yl)benzenesulfonamide;
<75> 2-methoxy-5-(1-(methylsulfonyl)-1H-pyrazole-4-yl)-N-(5-oxo-5,6,7,8-tetrahydro-1,6-naphthyridine-3-yl)benzenesulfonamide;
<76> 2-methoxy-N-(5-oxo-5,6,7,8-tetrahydro-1,6-naphthyridine-3-yl)-5-(1-(tetrahydro-2H-pyran-4-yl)-1H-pyrazole-4-yl)benzenesulfonamide;
<77> 5-(6-fluoropyridine-3-yl)-2-methoxy-N-(5-oxo-5,6,7,8-tetrahydro-1,6-naphthyridine-3-yl)benzenesulfonamide;
<78> 5-(2-amino-4-(trifluoromethyl)pyrimidine-5-yl)-2-methoxy-N-(5-oxo-5,6,7,8-tetrahydro-1,6-naphthyridine-3-yl)benzenesulfonamide;
<79> 2-methoxy-5-(1-(oxetane-3-yl)-1H-pyrazole-4-yl)-N-(5-oxo-5,6,7,8-tetrahydro-1,6-naphthyridine-3-yl)benzenesulfonamide;
<80> 5-(1-(2-hydroxyethyl)-1H-pyrazole-4-yl)-2-methoxy-N-(5-oxo-5,6,7,8-tetrahydro-1,6-naphthyridine-3-yl)benzenesulfonamide;
<81> 5-(1-isopropyl-3-(trifluoromethyl)-1H-pyrazole-4-yl)-2-methoxy-N-(5-oxo-5,6,7,8-tetrahydro-1,6-naphthyridine-3-yl)benzenesulfonamide;
<82> 5-(1-ethyl-3-(trifluoromethyl)-1H-pyrazole-4-yl)-2-methoxy-N-(5-oxo-5,6,7,8-tetrahydro-1,6-naphthyridine-3-yl)benzenesulfonamide;
<83> 5-(isoxazole-4-yl)-2-methoxy-N-(5-oxo-5,6,7,8-tetrahydro-1,6-naphthyridine-3-yl)benzenesulfonamide;
<84> 5-(3,5-dimethylisoxazole-4-yl)-2-methoxy-N-(5-oxo-5,6,7,8-tetrahydro-1,6-naphthyridine-3-yl)benzenesulfonamide;
<85> tert-butyl 4-(4-(4-methoxy-3-(N-(5-oxo-5,6,7,8-tetrahydro-1,6-naphthyridine-3-yl)sulfamoyl)phenyl)-1H-pyrazole-1-yl)piperidine-1-carboxylate;
<86> 2-methoxy-N-(5-oxo-5,6,7,8-tetrahydro-1,6-naphthyridine-3-yl)-5-(1-(piperidine-4-yl)-1H-pyrazole-4-yl)benzenesulfonamide hydrochloride;
<87> 5-bromo-2-methoxy-N-(6-methyl-5-oxo-5,6,7,8-tetrahydro-1,6-naphthyridine-3-yl)benzenesulfonamide;
<88> tert-butyl 3-((5-bromo-2-methoxyphenyl)sulfonamido)-7,8-dihydro-1,6-naphthyridine-6(5H)-carboxylate;
<89> tert-butyl 3-((2,5-dimethoxyphenyl)sulfonamido)-7,8-dihydro-1,6-naphthyridine-6(5H)-carboxylate;
<90> 5-fluoro-2-methoxy-N-(5,6,7,8-tetrahydro-1,6-naphthyridine-3-yl)benzenesulfonamide hydrochloride;
<91> 5-fluoro-2-bromo-N-(5,6,7,8-tetrahydro-1,6-naphthyridine-3-yl)benzenesulfonamide hydrochloride;
<92> 5-chloro-2-methoxy-N-(5,6,7,8-tetrahydro-1,6-naphthyridine-3-yl)benzenesulfonamide hydrochloride;
<93> 5-bromo-2-methoxy-N-(5,6,7,8-tetrahydro-1,6-naphthyridine-3-yl)benzenesulfonamide hydrochloride;
<94> 2,5-dimethoxy-N-(5,6,7,8-tetrahydro-1,6-naphthyridine-3-yl)benzenesulfonamide hydrochloride;
<95> ethyl 3-((5-fluoro-2-methoxyphenyl)sulfonamido)-7,8-dihydro-1,6-naphthyridine-6(5H)-carboxylate;
<96> ethyl 3-((5-chloro-2-methoxyphenyl)sulfonamido)-7,8-dihydro-1,6-naphthyridine-6(5H)-carboxylate;
<97> ethyl 3-((5-bromo-2-methoxyphenyl)sulfonamido)-7,8-dihydro-1,6-naphthyridine-6(5H)-carboxylate;
<98> neopentyl 3-((5-chloro-2-methoxyphenyl)sulfonamido)-7,8-dihydro-1,6-naphthyridine-6(5H)-carboxylate;
<99> neopentyl 3-((5-bromo-2-methoxyphenyl)sulfonamido)-7,8-dihydro-1,6-naphthyridine-6(5H)-carboxylate;
<100> neopentyl 3-((5-methoxy-2-methoxyphenyl)sulfonamido)-7,8-dihydro-1,6-naphthyridine-6(5H)-carboxylate;
<101> 2-fluoroethyl-3-((5-chloro-2-methoxyphenyl)sulfonamido)-7,8-dihydro-1,6-naphthyridine-6(5H)-carboxylate;
<102> 2-fluoroethyl3-((5-bromo-2-methoxyphenyl)sulfonamido)-7,8-dihydro-1,6-naphthyridine-6(5H)-carboxylate;
<103> 2-fluoroethyl-3-((5-methoxy-2-methoxyphenyl)sulfonamido)-7,8-dihydro-1,6-naphthyridine-6(5H)-carboxylate;
<104> 3-((5-chloro-2-methoxyphenyl)sulfonamido)-N-isopropyl-7,8-dihydro-1,6-naphthyridine-6(5H)-carboxamide;
<105> 3-((5-fluoro-2-methoxyphenyl)sulfonamido)-N-isopropyl-7,8-dihydro-1,6-naphthyridine-6(5H)-carboxamide;
<106> 3-((5-chloro-2-methoxyphenyl)sulfonamido)-N-pentyl-7,8-dihydro-1,6-naphthyridine-6(5H)-carboxamide;
<107> 3-((5-chloro-2-methoxyphenyl)sulfonamido)-N-(2,2-difluoroethyl)-7,8-dihydro-1,6-naphthyridine-6(5H)-carboxamide;
<108> 3-((5-bromo-2-methoxyphenyl)sulfonamido)-N-(2,2-difluoroethyl)-7,8-dihydro-1,6-naphthyridine-6(5H)-carboxamide;
<109> 5-chloro-2-methoxy-N-(6-(morpholine-4-carbonyl)-5,6,7,8-tetrahydro-1,6-naphthyridine-3-yl)benzenesulfonamide;
<110> 5-bromo-2-methoxy-N-(6-(morpholine-4-carbonyl)-5,6,7,8-tetrahydro-1,6-naphthyridine-3-yl)benzenesulfonamide;
<111> 3-((5-bromo-2-methoxyphenyl)sulfonamido)-N-methyl-7,8-dihydro-1,6-naphthyridine-6(5H)-carboxamide;
<112> 5-chloro-N-(6-isopropyl-5,6,7,8-tetrahydro-1,6-naphthyridine-3-yl)-2-methoxybenzenesulfonamide;
<113> 5-bromo-N-(6-isopropyl-5,6,7,8-tetrahydro-1,6-naphthyridine-3-yl)-2-methoxybenzenesulfonamide;
<114> 3-5-chloro-2-methoxy-N-(6-(pyrimidine-2-yl)-5,6,7,8-tetrahydro-1,6-naphthyridine-3-yl)benzenesulfonamide;
<115> 3-((5-chloro-2-methoxyphenyl)sulfonamido)-N-phenyl-7,8-dihydro-1,6-naphthyridine-6(5H)-carboxamide;
<116> 3-((5-chloro-2-methoxyphenyl)sulfonamido)-N-(cyclopropylmethyl)-7,8-dihydro-1,6-naphthyridine-6(5H)-carboxamide;
<117> 3-((5-chloro-2-methoxyphenyl)sulfonamido)-N-(2-fluoroethyl)-7,8-dihydro-1,6-naphthyridine-6(5H)-carboxamide;
<118> 5-chloro-N-(6-(3,3-dimethylbutanoyl)-5,6,7,8-tetrahydro-1,6-naphthyridine-3-yl)-2-methoxybenzenesulfonamide;
<119> 5-bromo-N-(6-(3,3-dimethylbutanoyl)-5,6,7,8-tetrahydro-1,6-naphthyridine-3-yl)-2-methoxybenzenesulfonamide;
<120> 2-methoxy-N-(5-oxo-5,6,7,8-tetrahydro-1,6-naphthyridine-3-yl)-5-(1-(pyridine-4-yl)-1H-pyrazole-4-yl)benzenesulfonamide;
<121> 2-methoxy-N-(5-oxo-5,6,7,8-tetrahydro-1,6-naphthyridine-3-yl)-5-(1,3,5-trimethyl-1H-pyrazole-4-yl)benzenesulfonamide;
<122> 5-(1-(tert-butyl)-1H-pyrazole-4-yl)-2-methoxy-N-(5-oxo-5,6,7,8-tetrahydro-1,6-naphthyridine-3-yl)benzenesulfonamide;
<123> 5-(1-butyl-1H-pyrazole-4-yl)-2-methoxy-N-(5-oxo-5,6,7,8-tetrahydro-1,6-naphthyridine-3-yl)benzenesulfonamide; and
<124> 2-methoxy-N-(5-oxo-5,6,7,8-tetrahydro-1,6-naphthyridine-3-yl)-5-(1-phenyl-1H-pyrazole-4-yl)benzenesulfonamide.

10. The compound or the pharmaceutically acceptable salt thereof according to any one of claims 1 to 9, wherein the compound inhibits tissue-nonspecific alkaline phosphatase (TNAP).

11. A pharmaceutical composition for the prevention or treatment of a disease associated with overexpression or overactivation of tissue-nonspecific alkaline phosphatase (TNAP), comprising the compound or the pharmaceutically acceptable salt thereof according to any one of claims 1 to 9 as an active ingredient, wherein the disease is selected from the group consisting of pseudoxanthoma elasticum (PXE), generalized arterial calcification of infancy (GACI), craniometaphyseal dysplasia (CMD), ossification of the yellow ligament (OYL), arterial calcification, calcification of joints, arthrosis deformans, osteoarthritis, ankylosis of the joint, idiopathic infantile arterial calcification (IIAC), ankylosing spondylitis (AS), tumoral calcinosis (TC), progressive osseous heteroplasia (POH), Keutel syndrome, vascular calcification associated with chronic renal failure (Chronic renal failure includes glomerulonephritis, IgA nephropathy, hypertensive nephropathy, and diabetic nephropathy), secondary parathyroid hyperplasia, metastatic calcification, calciphylaxis, calcific tendinitis of the longus colli muscle, fibrodysplasia ossificans progressive (FOP), calcific aortic stenosis, pericarditis calculosa, atherosclerotic vascular calcification, calcific uremic arteriopathy (CUA), Kawasaki disease, calcification due to obesity and aging, tibial arterial calcification, bone metastasis, prosthetic calcification, Paget 's disease, idiopathic basal ganglia calcification (IBGC), heterotopic ossification (HO), calcific aortic valve disease (CAVD), aortic valve stenosis (AVS), calcific tendinitis, ossification of the posterior longitudinal ligament (OPLL), ossification of the anterior longitudinal ligament (OALL), diffuse idiopathic skeletal hyperostosis (DISH), meniscal calcification, and peritoneal calcification.

12. The pharmaceutical composition according to claim 11, wherein the compound inhibits tissue-nonspecific alkaline phosphatase (TNAP).

13. A method for treating a disease associated with overexpression or overactivation of tissue-nonspecific alkaline phosphatase (TNAP) comprising administering the compound or the pharmaceutically acceptable salt thereof according to any one of claims 1 to 8 in a pharmaceutically effective amount to a subject in need thereof.

14. A use of the compound or the pharmaceutically acceptable salt thereof according to any one of claims 1 to 8 for the preparation of a medicament for preventing or treating a disease associated with overexpression or overactivation of tissue-nonspecific alkaline phosphatase (TNAP).
